# EUROPEAN PATENT APPLICATION

(11) **EP 4 306 535 A1**
(43) Date of publication of application: **17.01.2024**
(21) Application number: 22767237.5
(22) Date of filing: 10.03.2022
(51) Int. Cl.: C07K 7/08, A61K 39/395, A61K 47/55, A61K 47/68, A61K 49/00, A61P 35/00, C07C 247/12, C07K 14/00, C07K 16/00

(54) **COMPOUND OR SALT THEREOF, AND ANTIBODY OBTAINED USING SAME**

(30) Priority: 11.03.2021 JP 2021039706
(71) Applicant: AJINOMOTO CO., INC., Chuo-ku Tokyo 104-8315 (JP)
(72) Inventor: FUJII, Tomohiro, Kawasaki-shi, Kanagawa 210-8681 (JP); YAMADA, Kei, Kawasaki-shi, Kanagawa 210-8681 (JP); MATSUDA, Yutaka, Kawasaki-shi, Kanagawa 210-8681 (JP); HIRAMA, Ryusuke, Kawasaki-shi, Kanagawa 210-8681 (JP); HATADA, Noriko, Kawasaki-shi, Kanagawa 210-8681 (JP); ARASHIDA, Naoko, Kawasaki-shi, Kanagawa 210-8681 (JP)
(74) Representative: Mewburn Ellis LLP
(86) International application number: PCT/JP2022/010574
(87) International publication number: WO 2022/191283

(57) **Abstract**

The present invention provides a technique that controls a bonding ratio of an antibody and a modifying group within a desired range. More specifically, the present invention provides a compound or salt thereof represented by the following formula (I): 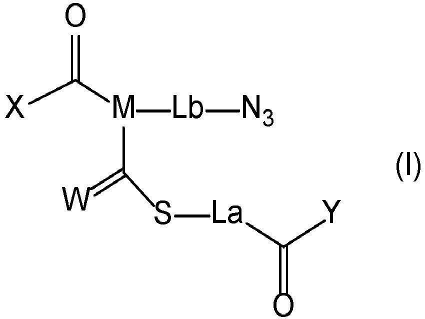 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group; and antibodies or salts thereof which can be prepared using such a compound or salt thereof.

## Description

### TECHNICAL FIELD

The present invention relates to a compound or salt thereof, and antibody obtained by using the same, and the like.

### BACKGROUND ART

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) which is jointly developed by Immunogen and Roche.

ADCs including T-DM1 have had the problem of their nonuniformity from the beginning of their development. That is, a small compound drug is randomly reacted with about 70 to 80 lysine residues in an antibody, and thus a drug antibody ratio (DAR) and a conjugation position are not constant. It is known that such a random conjugation method normally provides a DAR within a range of 0 to 8, producing a plurality of antibody medicines having different numbers of bonds of a drug. In recent years, it has been reported that when the number of bonds and the bond positions of a drug of an ADC are changed, pharmacokinetics, and a releasing rate and effects of the drug change. Given these circumstances, next-generation ADCs are required to control the number and positions of a drug to be conjugated. It is believed that when the number and positions are fixed, the problems of expected efficacy, variations in conjugation medicines, and lot difference, or what is called regulation, will be solved.

Although methods for regioselectively modifying antibodies are being investigated worldwide, most of them are methods of modification using genetic engineering techniques or enzymes. For the genetic engineering methods of modification, problems have been pointed out such as reductions in the expression efficiency of antibodies themselves (reductions in total yield when ADCs are prepared), although regioselectivity and number selectivity can be controlled. In addition, there is a problem in that it takes long years to construct an antibody expression system and the like.

In recent years, a chemical conjugation by affinity peptide (CCAP) method which can regioselectively modify an antibody by chemical synthetic technique has recently been developed (Patent Literature 1). This method has succeeded in regioselective modification of antibodies by a method that reacts a peptide reagent in which an NHS-activated ester and a drug are coupled with an affinity peptide with an antibody. However, in the ADC produced by this method, the antibody and the drug are bonded via a linker containing a peptide moiety. The peptide moiety has potential immunogenicity and is susceptible to hydrolysis in the blood. Therefore, the ADC produced by this method has room for improvement in that it contains a peptide moiety in the linker.

As an improved method of the above C-CAP method, techniques which can prepare an antibody which does not contain a peptide moiety as a linker and has a functional substance (e.g., a drug) regioselectively by a chemical synthesis method using a certain compound containing an affinity peptide have been reported (Patent Literature 2 to 6). Avoiding the use of linkers containing peptide moieties is desirable in clinical applications. In these techniques, plural positions corresponding to various amino acid residues in CH2 and CH3 domains (e.g., lysine residues, tyrosine residues, serine residues, and threonine residue) have been proposed as the positions of amino acid residues in antibodies that can be regioselectively modified with drugs. However, it is not necessarily easy to regioselectively modify an antibody with a functional substance and control the bonding ratio of the antibody to the functional substance within a desired range.

### PRIOR ART REFERENCES

### Patent Literature

Patent Literature 1: WO2016/186206
Patent Literature 2: WO2018/199337
Patent Literature 3: WO2019/240287
Patent Literature 4: WO2019/240288
Patent Literature 5: WO2020/009165
Patent Literature 6: WO2020/090979

### DISCLOSURE OF INVENTION

### PROBLEM TO BE SOLVED BY THE INVENTION

An object of the present invention is to control a bonding ratio of an antibody and a modifying group within a desired range.

### Solution to PROBLEM

As a result of diligent studies, the present inventors have found that by selecting a lysine residue of a heavy chain in an immunoglobulin unit as a modification position of an antibody, and by using a compound represented by formula (I) as a compound which can regioselectively modify the lysine residue, it is easy to highly control an average bonding ratio of an immunogloblin unit and an azide group-containing modifying group within a desired range (1.0 to 3.0).

The inventors have also found that the azide group-containing compound is excellent in the versatility of the regioselective modification of the immunoglobulin unit since the regioselective modification of different lysine residues in the heavy chain in the immunoglobulin unit is facilitated. For example, by using an azide group-containing compound having an affinity peptide having a certain kind of amino acid sequences, the average bonding ratio of the immunoglobulin unit and an azide group-containing modifying group can be highly controlled within the above desired range while regioselectively modifying the lysine residue at the position 246/248 in a human IgG heavy chain. In addition, by using an azide group-containing compound having an affinity peptide having a another kind of amino acid sequences, the average bonding ratio of the immunoglobulin unit and an azide group-containing modifying group can be highly controlled within the above desired range while regioselectively modifying the lysine residue at the position 288/290 in a human IgG heavy chain.

The inventors have further found that an antibody modified with the azide group-containing modifying group at the lysine residue in heavy chains in the immunoglobulin unit, and showing the average bonding ratio of the immunoglobulin unit and azide group-containing modifying group (the number of azide group-containing modifying groups/immunoglobulin unit) within a desired range (1.0-3.0) can present excellent stability and the like, and have completed the present invention.

Accordingly, the present invention provides a compound or salt thereof shown as below, or a reagent for derivatizing an antibody which comprises the same.
[1] A compound or salt thereof represented by formula (I).
[2] The compound or salt thereof accordng to [1], wherein the leaving group is selected from:
   (a) R-S wherein R indicates a hydrogen atom, a monovalent hydrocarbon group which may have a substituent, or a monovalent heterocyclic group which may have a substituent, and S indicates a sulfur atom;
   (b) R-O wherein R indicates a hydrogen atom, a monovalent hydrocarbon group which may have a substituent, or a monovalent heterocyclic group which may have a substituent, and O indicates an oxygen atom; or
   (c) R_{A}-(R_{B})-N wherein R_{A} and R_{B} each independently represent a hydrogen atom, a monovalent hydrocarbon group which may have a substituent, or a monovalent heterocyclic group which may have a substituent, and N indicates a nitrogen atom; or
   (d) a halogen atom.
[3] The compound or salt according to [1] or [2], wherein the immunoglobulin unit is a human immunoglobulin unit.
[4] The compound or salt thereof according to any of [1] to [3], wherein the immunoglobulin unit is a human IgG.
[5] The compound or salt thereof according to any of [1] to [4], wherein the main chain portion consisting of 3 to 5 carbon atoms in M is a portion comprising a linear alkylene, or a ring-constituting carbon atom, or a combination thereof.
[6] The compound or salt thereof according to any of [1] to [5], wherein the number of atoms in a main chain linking M and Y is 6 to 20.
[7] The compound or salt thereof according to any of [1] to [6], wherein the carbonyl group adjacent to La forms an amide bond with an amino group in a side chain of a lysine residue in the affinity peptide.
[8] The compound or salt thereof according to any of [1] to [7], wherein the affinity peptide comprises the amino acid sequence of the following (A):
   (A) (X0-3)a-C-Xaa1-Xaa2-Xaa3-Xaa4-Xaa5-Xaa6-I-I-W-C-(X0-3)b (SEQ ID NO: 1).
   wherein
   (X₀₋₃)ₐ is none, or one to three consecutive arbitrary amino acid residues (other than lysine residue and cysteine residue) which are the same or different,
   (X₀₋₃)_{b} is none, or one to three consecutive arbitrary amino acid residues (other than lysine residue and cysteine residue) which are the same or different,
   Xaa1 is an alanine residue, a glycine residue, a leucine residue, a proline residue, an arginine residue, a valine residue, an asparagine residue, a glutamic acid residue, or a phenylalanine residue,
   Xaa2 is a tyrosine residue, a tryptophan residue, a histidine residue, or a phenylalanine residue,
   Xaa3 is a histidine residue, a phenylalanine residue, a tyrosine residue, a tryptophan residue, an arginine residue, or a glycine residue,
   Xaa 4 is a lysine residue,
   Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue,
   Xaa6 is a glutamine residue, a glutamic acid residue, an asparagine residue, an aspartic acid residue, a proline residue, a glycine residue, an arginine residue, a phenylalanine residue, or a histidine residue.
[9] The compound or salt thereof according to [8], wherein the affinity peptide comprising the amino acid sequence of (A) comprises the amino acid sequence of the following (1):
   (1) RGNCAYHKGQIIWCTYH (SEQ ID NO: 2).
[10] The compound or salt thereof according to any of [1] to [7], wherein the affinity peptide comprises the amino acid sequence of the following (B):
   (B) PNLNEEQRNARIRSI (SEQ ID NO: 3).
[11] The compound or salt according to [10], wherein the affinity peptide comprising the amino acid sequence of (B) comprises an amino acid sequence selected from the group consisting of the following (1)-(3):
   (1) FNMQCQRRFYEALHDPNLNEEQRNARIRSIKDDC (SEQ ID NO: 4);
   (2) FNMQCQRRFYEALHDPNLNEEQRNARIRSIKEEC (SEQ ID NO: 5); or
   (3) MQCQRRFYEALHDPNLNEEQRNARIRSI(Orn)EEC (SEQ ID NO: 6).
[12] The compound or salt thereof according to any of [8] to [11], wherein the N-terminal and C-terminal amino acid residues in the affinity peptide may be protected, and
   the two thiol groups in the side chain of the two cysteine residues (C) in the affinity peptide may be linked by a disulfide bond or via a linker.
[13] A reagent for derivatizing an antibody, comprising a compound or salt thereof represented by formula (I).

The present invention provides an antibody intermediate or salt thereof.
[1] The antibody intermediate or salt thereof comprising a structural unit represented by formula (II).
[2] The antibody intermediate or salt thereof according to [1], wherein the antibody is a human antibody.
[3] The antibody intermediate or salt thereof according to [1] or [2], wherein the antibody is human IgG.
[4] The antibody intermediate or salt thereof according to any of [1] to [3], wherein the lysine residue is present at one or more positions selected from the group consisting of the position 246/248, the position 288/290, and the position 317 in a heavy chain of a human IgG according to EU numbering.
[5] The antibody intermediate or salt thereof according to any of [1] to [4], wherein the lysine residue has regioselectivity for lysine residues at the 246/248 position of the human IgG heavy chain according to EU numbering.
[6] The antibody intermediate or salt thereof according to any of [1] to [4], wherein the lysine residue has regioselectivity for the lysine residue at the position 288/290 in a heavy chain of a human IgG according to EU numbering.
[7] The antibody intermediate or salt thereof according to any of [1] to [6], wherein the average ratio r is 1.5 to 2.5.
[8] The antibody intermediate or salt thereof according to any of [1] to [7], wherein the main chain portion consisting of 3 to 5 carbon atoms in M is a portion comprising a linear alkylene, or a ring-constituting carbon atom, or a combination thereof.
[9] The antibody intermediate or salt thereof according to any of [4] to [8], wherein the lysine residue is present at two positions selected from the group consisting of the position 246/248, the position 288/290, and the position 317 in a heavy chain of a human IgG according to EU numbering.
[10] The antibody intermediate or salt thereof according to [9], wherein the two positions are the position 246/248 and the position 288/290.
[11] The antibody intermediate or salt thereof according to any of [1] to [10], wherein the number of atoms in a main chain linking M and Y is 6 to 20.
[12] The antibody intermediate or salt thereof according to any of [1] to [11], wherein the carbonyl group adjacent to La forms an amide bond with an amino group in a side chain of a lysine residue in the affinity peptide.
[13] The antibody intermediate or salt thereof according to any of [1] to [12], wherein the affinity peptide comprises the amino acid sequence of the following (A):
   (A) (X0-3)a-C-Xaa1-Xaa2-Xaa3-Xaa4-Xaa5-Xaa6-I-I-W-C-(X0-3)b (SEQ ID NO: 1).
   wherein
   (X₀₋₃)ₐ is none, or one to three consecutive arbitrary amino acid residues (other than lysine residue and cysteine residue) which are the same or different,
   (X₀₋₃)_{b} is none, or one to three consecutive arbitrary amino acid residues (other than lysine residue and cysteine residue) which are the same or different,
   Xaa1 is an alanine residue, a glycine residue, a leucine residue, a proline residue, an arginine residue, a valine residue, an asparagine residue, a glutamic acid residue, or a phenylalanine residue,
   Xaa2 is a tyrosine residue, a tryptophan residue, a histidine residue, or a phenylalanine residue,
   Xaa3 is a histidine residue, a phenylalanine residue, a tyrosine residue, a tryptophan residue, an arginine residue, or a glycine residue,
   Xaa 4 is a lysine residue,
   Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue,
   Xaa6 is a glutamine residue, a glutamic acid residue, an asparagine residue, an aspartic acid residue, a proline residue, a glycine residue, an arginine residue, a phenylalanine residue, or a histidine residue.
[14] The antibody intermediate or salt thereof according to [13], wherein the affinity peptide comprising the amino acid sequence of (A) comprises the amino acid sequence of the following (1):
   (1) RGNCAYHKGQIIWCTYH (SEQ ID NO: 2).
[15] The antibody intermediate or salt thereof according to any of [1] to [12], wherein the affinity peptide comprises the amino acid sequence of the following (B):
   (B) PNLNEEQRNARIRSI (SEQ ID NO: 3).
[16] The antibody intermediate or salt according to [15], wherein the affinity peptide comprising the amino acid sequence of (B) comprises an amino acid sequence selected from the group consisting of the following (1)-(3):
   (1) FNMQCQRRFYEALHDPNLNEEQRNARIRSIKDDC (SEQ ID NO: 4);
   (2) FNMQCQRRFYEALHDPNLNEEQRNARIRSIKEEC (SEQ ID NO: 5); or
   (3) MQCQRRFYEALHDPNLNEEQRNARIRSI(Orn)EEC (SEQ ID NO: 6).
[17] The antibody intermediate or salt thereof according to any of [1] to [16], wherein the N-terminal and C-terminal amino acid residues in the affinity peptide may be protected, and
   the two thiol groups in the side chain of the two cysteine residues (C) in the affinity peptide may be linked by a disulfide bond or via a linker.

The present invention also provides an azide group-introduced antibody derivative or salt thereof.
[1] An azide group-introduced antibody derivative or salt thereof comprising a structural unit represented by formula (III) .
[2] The azide group-introduced antibody derivative or salt according to [1], wherein the monovalent group indicated by T is a hydroxyamino group which may be substituted.
[3] The azide group-introduced antibody derivative or salt according to [1] or [2], wherein the antibody is a human antibody.
[4] The azide group-introduced antibody derivative or salt thereof according to any of [1] to [3], wherein the antibody is human IgG.
[5] The azide group-introduced antibody derivative or salt thereof according to any of [1] to [4], wherein the main chain portion consisting of 3 to 5 carbon atoms in M is a portion comprising a linear alkylene, or a ring-constituting carbon atom, or a combination thereof.
[6] The azide group-introduced antibody derivative or salt thereof according to any of [1] to [5], wherein the number of atoms in a main chain linking M and Y is 6 to 20.
[7] The azide group-introduced antibody derivative or salt thereof according to any of [1] to [6], wherein the lysine residue is present at one or more positions selected from the group consisting of the position 246/248, the position 288/290, and the position 317 in a heavy chain of a human IgG according to EU numbering.
[8] The azide group-introduced antibody derivative or salt thereof according to any of [1] to [7], wherein the lysine residue has regioselectivity for lysine residues at the 246/248 position of the human IgG heavy chain according to EU numbering.
[9] The azide group-introduced antibody derivative or salt thereof according to any of [1] to [7], wherein the lysine residue has regioselectivity for the lysine residue at the position 288/290 in a heavy chain of a human IgG according to EU numbering.
[10] The azide group-introduced antibody derivative or salt thereof according to any of [1] to [9], wherein the average ratio r is 1.5-2.5.
[11] The azide group-introduced antibody derivative or salt thereof according to any of [7] to [10], wherein the lysine residue is present at two positions selected from the group consisting of the position 246/248, the position 288/290, and the position 317 in a heavy chain of a human IgG according to EU numbering.
[12] The azide group-introduced antibody derivative or salt thereof according to [11], wherein the two positions are the position 246/248 and the position 288/290.

The present invention also provides a conjugate of an antibody and a functional substance or a salt thereof.
[1] A conjugate of an antibody and a functional substance or a salt thereof comprising a structural unit represented by formula (IV).
[2] The conjugate or salt thereof according to [1], wherein the antibody is a human antibody.
[3] The conjugate or salt thereof according to [1] or [2], wherein the antibody is a human IgG.
[4] The conjugate or salt thereof according to any of [1] to [3], wherein the main chain portion consisting of 3 to 5 carbon atoms in M is a portion comprising a linear alkylene, or a ring-constituting carbon atom, or a combination thereof.
[5] The conjugate or salt thereof according to any of [1] to [4], wherein the number of atoms in a main chain linking M and Y is 6 to 20.
[6] The conjugate or salt thereof according to any of [1] to [5], wherein the lysine residue is present at one or more positions selected from the group consisting of the position 246/248, the position 288/290, and the position 317 in a heavy chain of a human IgG according to EU numbering.
[7] The conjugate or salt thereof according to any of [1] to [6], wherein the lysine residue has regioselectivity for the lysine residue at the position 246/248 in a heavy chain of a human IgG according to EU numbering.
[8] The conjugate or salt thereof according to any of [1] to [7], wherein the lysine residue has regioselectivity for the lysine residue at the position 288/290 in a heavy chain of a human IgG according to EU numbering.
[9] The conjugate or salt thereof according to any of [1] to [8], wherein the average ratio r is 1.5 to 2.5.
[10] The conjugate or salt thereof according to any of [6] to [9], wherein the lysine residue is present at two positions selected from the group consisting of the position 246/248, the position 288/290, and the position 317 in a heavy chain of a human IgG according to EU numbering.
[11] The conjugate or salt thereof according to [10], wherein the two positions are the position 246/248 and the position 288/290.

The present invention also provides a method for producing an antibody intermediate, an azide group-introduced antibody derivative, or a conjugate of an antibody and a functional substance, or a salt thereof.
[1] A method for producing an antibody intermediate or salt thereof, the method comprising reacting a compound or salt thereof represented by formula (I) with an antibody comprising the immunoglobulin unit to give an antibody intermediate or salt thereof comprising a structural unit represented by formula (II).
[2] A method for producing an azide group-introduced antibody derivative or salt thereof, the method comprising:
   (1) reacting a compound or salt thereof represented by formula (I) with an antibody comprising the immunoglobulin unit to give an antibody intermediate or salt thereof comprising a structural unit represented by formula (II); and
   (2) subjecting the antibody intermediate or salt thereof to a thioester cleavage reaction to give an azide group-introduced antibody derivative or salt thereof comprising a structural unit represented by formula (III).
[3] A method for producing a conjugate of an antibody and a functional substance or a salt thereof, the method comprising:
   (1) reacting a compound or salt thereof represented by formula (I) with an antibody comprising the immunoglobulin unit to give an antibody intermediate or salt thereof comprising a structural unit represented by formula (II);
   (2) subjecting the antibody intermediate or salt thereof to a thioester cleavage reaction to give an azide group-introduced antibody derivative or salt thereof comprising a structural unit represented by formula (III); and
   (3) reacting the azide group-introduced antibody derivative or salt thereof with a target substance represented by formula (V) to give the conjugate of an antibody and a functional substance or the salt thereof which comprises a structural unit represented by formula (IV).
[4] A method for producing an azide group-introduced antibody derivative or salt thereof, the method comprising subjecting the antibody intermediate or salt thereof comprising a structural unit represented by formula (II) to a thioester cleavage reaction to give an azide group-introduced antibody derivative or salt thereof comprising a structural unit represented by formula (III).
[5] A method for producing a conjugate of an antibody and a functional substance or a salt thereof, comprising:
   (1) subjecting the antibody intermediate or salt thereof comprising a structural unit represented by formula (II) to a thioester cleavage reaction to give an azide group-introduced antibody derivative or salt thereof comprising a structural unit represented by formula (III); and
   (2) reacting the azide group-introduced antibody derivative or salt thereof with a target substance represented by formula (V) to give the conjugate of an antibody and a functional substance or the salt thereof comprising a structural unit represented by formula (IV).
[6] A method for producing a conjugate of an antibody and a functional substance or a salt thereof, the method comprising reacting the azide group-introduced antibody derivative or salt thereof comprises a structural unit represented by formula (III) with a target substance represented by formula (V) to give the conjugate of an antibody and a functional substance or the salt thereof comprising a structural unit represented by formula (IV).

### Effect of Invention

The compound or salt thereof represented by formula (I) can highly modify a lysine residue of a heavy chain in an immunoglobulin unit so as to be the average ratio of the bonds between the immunoglobulin unit and the azide group-containing modifying group (the number of azide group-containing modifying groups/immunoglobulin unit) in a desired range (1.0 to 3.0). The azide group-containing compound or salt thereof also has the advantage of having excellent versatility of regioselective modification of a lysine residue at different position in a heavy chain of the immunoglobulin unit. Therefore, the compound or salt thereof represented by formula (I) is useful as a reagent for derivatizing an antibody.

In addition, the compound or salt thereof represented by formula (I) can provide an antibody intermediate or salt thereof represented by formula (II) in which the lysine residue in the heavy chain of the immunoglobulin unit is specifically modified with an affinity peptide-containing group, and the average ratio of the bonds between the immunoglobulin unit and the zide group-containing modifying group (the number ofzide group-containing modifying group/immunoglobulin unit) is highly controlled within a desired range.

Furthermore, an antibody prepared by using the antibody intermediate or salt thereof represented by formula (II) as a raw material can inherit desired properties (e.g., average bonding ratio, regioselectivity) of the antibody intermediate or salt thereof. Therefore, an azide group-introduced antibody derivative or salt thereof represented by formula (III) and a conjugate of an antibody and a functional substance represented by the formula (IV) or a salt thereof, which has the above properties can be provided.

In addition, the antibody of the present invention as described above has excellent stability.

### BRIEF DESCRIPTION OF DRAWINGS

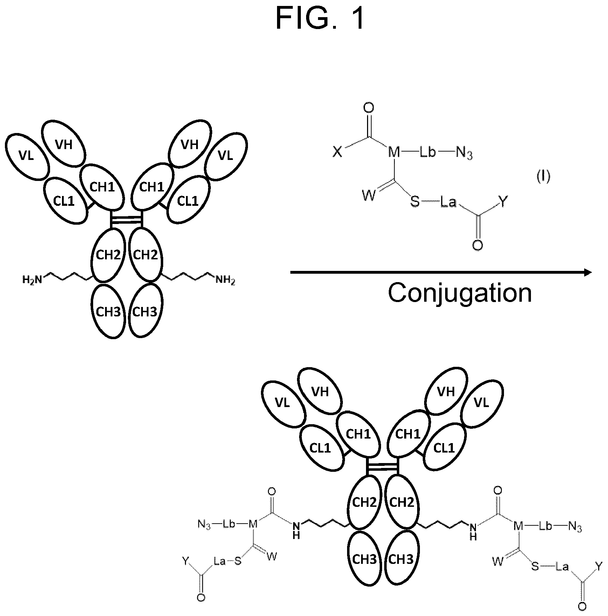

FIG. 1 is a schematic diagram (1) showing the concept of modification of an immunoglobulin unit with the compound of the present invention or salt thereof represented by the formula (I). First, the compound of the present invention or salt thereof represented by the formula (I) associates with the CH2 domain in the immunoglobulin unit via an affinity peptide (Y). Next, the compound of the present invention or salt thereof represented by the formula (I) reacts with a side chain of a specific amino acid residue in the CH2 domain (In the figure, an amino group in a side chain of a lysine residue) via an activated carbonyl group having a leaving group (X).

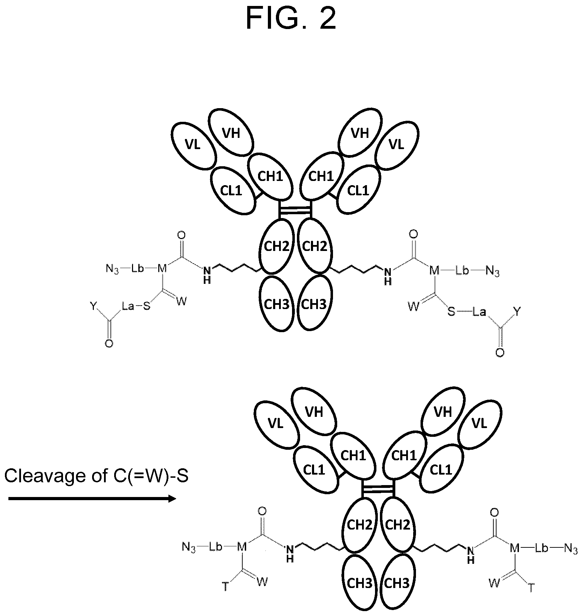

FIG. 2 is a schematic diagram (2) showing the concept of modification of an immunoglobulin unit with the compound of the present invention or salt thereof represented by the formula (I). Cleavage of the thioester group produces an azide group-introduced antibody derivative or a salt thereof.

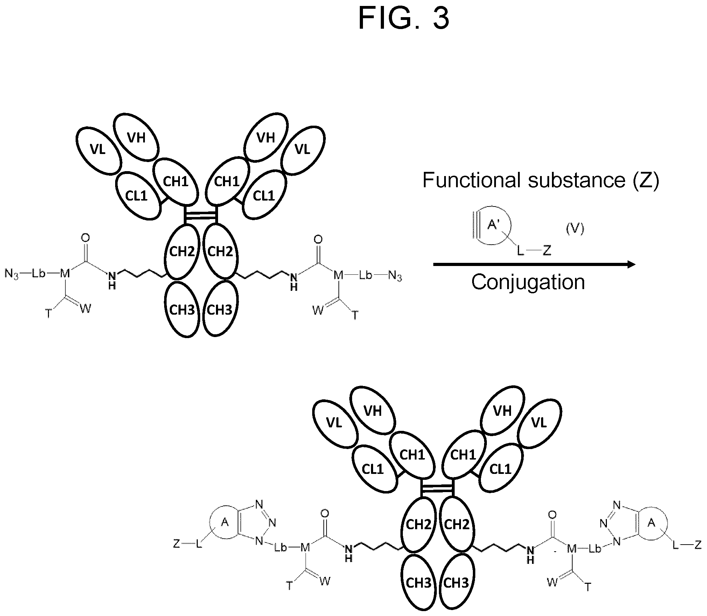

FIG. 3 is a schematic diagram (3) showing the concept of modification of an immunoglobulin unit with the compound of the present invention or salt thereof represented by the formula (I). The reaction of an azide group in an azide group-introduced antibody derivative or a salt thereof with a functional substance (Z) produces a conjugate of an antibody and a functional substance or a salt thereof. Such a reaction is known as strain-promoted azide-alkyne cyclization (SPAAC) reaction.

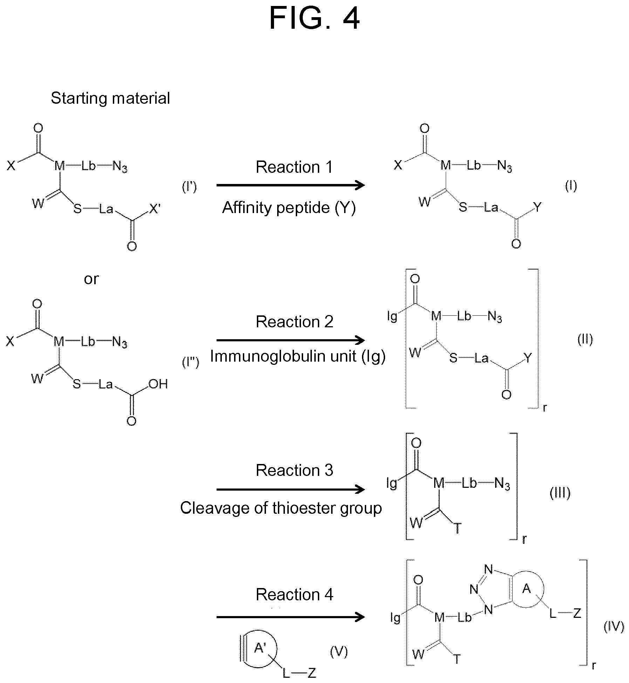

FIG. 4 is a diagram showing an outline of an embodiment of the present invention.

### EMBODIMENTS FOR CARRYING OUT THE INVENTION

### 1.Definitions of general terms

In the present invention, the term "antibody" is as follows. The term "immunoglobulin unit" corresponds to a divalent monomer unit that is a basic constituent element of such an antibody, and is a unit comprising two heavy chains and two light chains. Therefore, definitions, examples, and preferred examples of the origin, type (polyclonal or monoclonal, isotype, and full-length antibody or antibody fragment), antigen, position of a lysine residue, and regioselectivity of the immunoglobulin unit are similar to those of the antibody described below.

The origin of the antibody is not particularly limited, and for example, the antibody may be derived from an animal such as a mammal or a bird (e.g., a domestic fowl). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or a variable region, and an antibody fragment comprising a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include a protein [which comprises an oligopeptide and a polypeptide, and may be a protein modified with a biomolecule such as a sugar (e.g., a glycoprotein)], a sugar chain, a nucleic acid, and a small compound. The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.

### (1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

### (2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, and Siglec-15

### (8) Blood Diseases

vWF, Factor IXa, Factor X, IFNγ, C5, BMP-6, Ferroportin, and TFPI

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

The positions of an amino acid residue in the antibody and the position of a constant region of a heavy chain (e.g., CH2 domain) are in accordance with EU numbering (see, http://www.imgt.org/IMGTScientificChart/Numbering/Hu_IGHGnb er. html). For example, when human IgG is a target, a lysine residue at position 246 corresponds to an amino acid residue at position 16 of a human IgG CH2 region, a lysine residue at position 248 corresponds to an amino acid residue at position 18 of a human IgG CH2 region, a lysine residue at position 288 corresponds to an amino acid residue at position 58 of a human IgG CH2 region, a lysine residue at position 290 corresponds to an amino acid residue at position 60 of a human IgG CH2 region, and a lysine residue at position 317 corresponds to an amino acid residue at position 87 of a human IgG CH2 region. The notation at position 246/248 indicates that a lysine residue at position 246 or position 248 is a target. The notation at position 288/290 indicates that a lysine residue at position 288 or position 290 is a target.

According to the present invention, a specific lysine residue (e.g., lysine residue at the position 246/248, or the position 288/290) in a heave chain of an antibody can be regioselectively modified. In the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 100%. According to the present invention, the specific lysine residue in the heavy chain of the antibody can be regioselectively modified without utilizing a linker containing a peptide. The peptide moiety has potential immunogenicity and is susceptible to hydrolysis in a blood. Therefore, avoiding the use of a linker containing a peptide moiety is desirable in clinical applications.

In the present invention, as long as a specific lysine residue in the heavy chain of the antibody is regioselectively modified, a specific lysine residue at another position may be further regioselectively modified. For example, a method for regioselectively modifying a specific amino acid residue at a predetermined position in an antibody is described in WO 2018/199337 A, WO 2019/240288 A, WO 2019/240287 A, and WO 2020/090979 A. As such a specific amino acid residue, an amino acid residue (e.g., a lysine residue, an aspartic acid residue, a glutamic acid residue, an asparagine residue, a glutamine residue, a threonine residue, a serine residue, a tyrosine residue, or a cysteine residue) having a side chain that is easily modified (e.g., an amino group, a carboxy group, an amide group, a hydroxy group, or a thiol group) can be used. However, a lysine residue having a side chain comprising an amino group, a tyrosine residue having a side chain comprising a hydroxy group, a serine residue, a threonine residue, or a cysteine residue having a side chain comprising a thiol group may be preferred, a lysine residue may be more preferred (i.e., among lysine residue at the position 246/248, lysine residue at the position 288/290, and lysine residue at the position 317, two lysine residues may be double-modified regioselectively, and the three lysine residues may be triple-modified regioselectively). Even more preferably, lysine residues present at two positions selected from the group consisting of the positions 246/248, 288/290 and 317 in a human IgG heavy chain according to EU numbering may be regioselectively modified. Particularly preferably, lysine residues present at two positions of the position 246/248 and the position 288/290 may be regioselectively modified.

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 2. Compound or salt

The present invention provide 1. A compound or salt thereof, the compound being represented by the following formula (I): 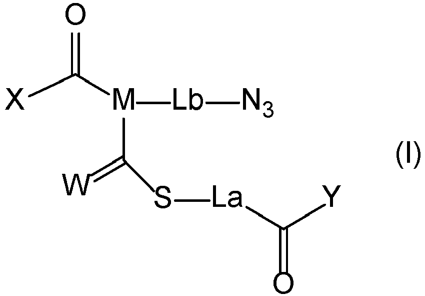 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

In formula (I) and other formulae presented in relation to the present invention, -(hyphen) indicates that two units (e.g., atoms or groups) present on both sides thereof are covalently bonded to each other. Therefore, in the formula (I), X is covalently bonded to the carbon atom constituting the carbonyl group, M is covalently bonded to the carbon atom constituting the carbonyl group, the carbon atom constituting C=W and Lb, S is covalently bonded to the carbon atom constituting C=W, and La, La is covalently bonded to S and Y, Lb is covalently bonded to M and N₃, Y is covalently bonded to La, and N₃ is covalently bonded to Lb.

The leaving group represented by X is a group that can be eliminated by a reaction between a carbon atom of a carbonyl group adjacent to X and an amino group. A person skilled in the art can appropriately design such a leaving group. Examples of such leaving groups include:
(a) R-S wherein R indicates a hydrogen atom, a monovalent hydrocarbon group which may have a substituent, or a monovalent heterocyclic group which may have a substituent, and S indicates a sulfur atom;
(b) R-O wherein R indicates a hydrogen atom, a monovalent hydrocarbon group which may have a substituent, or a monovalent heterocyclic group which may have a substituent, and O indicates an oxygen atom;
(c) R_{A}-(R_{B}-)N wherein R_{A} and R_{B} each independently indicate a hydrogen atoms, a monovalent hydrocarbon group which may have a substituent, or a monovalent heterocyclic group which may have a substituent, and N indicates a nitrogen atom; or
(d) a halogen atom.

Preferably, the leaving group represented by X may be:
(a) R-S wherein R indicates a hydrogen atom, a monovalent hydrocarbon group which may have a substituent, or a monovalent heterocyclic group which may have a substituent, and S indicates a sulfur atom;
(b) R-O wherein R indicates a hydrogen atom, a monovalent hydrocarbon group which may have a substituent, or a monovalent heterocyclic group which may have a substituent, and O indicates an oxygen atom; or
(c) R_{A}-(R_{B}-)N wherein R_{A} and R_{B} each independently indicate a hydrogen atoms, a monovalent hydrocarbon group which may have a substituent, or a monovalent heterocyclic group which may have a substituent, and N indicates a nitrogen atom.

More preferably, the leaving group represented by X may be:
(a) R-S wherein R indicates a hydrogen atom, a monovalent hydrocarbon group which may have a substituent, or a monovalent heterocyclic group which may have a substituent, and S indicates a sulfur atom; or
(b) R-O wherein R indicates a hydrogen atom, a monovalent hydrocarbon group which may have a substituent, or a monovalent heterocyclic group which may have a substituent, and O indicates an oxygen atom.

Even more preferably, the leaving group represented by X may be:
(a) R-S wherein R indicates a hydrogen atom, a monovalent hydrocarbon group which may have a substituent, or a monovalent heterocyclic group which may have a substituent, and S indicates a sulfur atom.

Particularly preferably, the leaving group represented by X may be:
(a') R-S wherein R indicates a monovalent hydrocarbon group (e.g., phenyl) which may have a substituent, and S indicates a sulfur atom.

Examples of the halogen atom include a fluorine atom, a chlorine atom, a bromine atom, and an iodine atom.

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in a main chain thereof. Note that the chain structure may be linear or branched. Examples of the monovalent chain hydrocarbon group include alkyl, alkenyl, and alkynyl. The alkyl, alkenyl, and alkynyl may be linear or branched.

The alkyl is preferably C₁₋₁₂ alkyl, more preferably C₁₋₆ alkyl, and even more preferably C₁₋₄ alkyl. When the alkyl has a substituent, the number of carbon atoms does not comprise the number of carbon atoms of the substituent. Examples of the C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C₂₋₁₂ alkenyl, more preferably C₂₋₆ alkenyl, and even more preferably C₂₋₄ alkenyl. When the alkenyl has a substituent, the number of carbon atoms does not comprise the number of carbon atoms of the substituent. Examples of the C₂₋₁₂ alkenyl include vinyl, propenyl, and n-butenyl.

The alkynyl is preferably C₂₋₁₂ alkynyl, more preferably C₂₋₆ alkynyl, and even more preferably C₂₋₄ alkynyl. When the alkynyl has a substituent, the number of carbon atoms does not comprise the number of carbon atoms of the substituent. Examples of the C₂₋₁₂ alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

The cycloalkyl is preferably C₃₋₁₂ cycloalkyl, more preferably C₃₋₆ cycloalkyl, and even more preferably C₅₋₆ cycloalkyl. When the cycloalkyl has a substituent, the number of carbon atoms does not comprise the number of carbon atoms of the substituent. Examples of the C₃₋₁₂ cycloalkyl include cyclopropyl, cyclobutyl, cyclopentyl, and cyclohexyl.

The cycloalkenyl is preferably C₃₋₁₂ cycloalkenyl, more preferably C₃₋₆ cycloalkenyl, and even more preferably C₅₋₆ cycloalkenyl. When the cycloalkenyl has a substituent, the number of carbon atoms does not comprise the number of carbon atoms of the substituent. Examples of the C₃₋₁₂ cycloalkenyl include cyclopropenyl, cyclobutenyl, cyclopentenyl, and cyclohexenyl.

The cycloalkynyl is preferably C₃₋₁₂ cycloalkynyl, more preferably C₃₋₆ cycloalkynyl, and even more preferably C₅₋₆ cycloalkynyl. When the cycloalkynyl has a substituent, the number of carbon atoms does not comprise the number of carbon atoms of the substituent. Examples of the C₃₋₁₂ cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C₆₋₁₂ aryl, more preferably C₆₋₁₀ aryl, and even more preferably C₆ aryl. When the monovalent aromatic hydrocarbon group has a substituent, the number of carbon atoms does not comprise the number of carbon atoms of the substituent. Examples of the C₆₋₁₂ aryl include phenyl and naphthyl.

The monovalent aromatic hydrocarbon group is preferably phenyl.

Among these groups, the monovalent hydrocarbon group is preferably alkyl, cycloalkyl, or aryl.

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom comprised in the heterocyclic group.

The monovalent aromatic heterocyclic group is preferably a C₁₋₁₅ aromatic heterocyclic group, more preferably a C₁₋₉ aromatic heterocyclic group, and even more preferably a C₁₋₆ aromatic heterocyclic group. When the monovalent aromatic heterocyclic group has a substituent, the number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and even more preferably a C₂₋₆ nonaromatic heterocyclic group. When the monovalent nonaromatic heterocyclic group has a substituent, the number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

The number of "substituents" in "monovalent hydrocarbon group which may have a substituent" and "monovalent heterocyclic group which may have a substituent" represented by R, R_{A}, and R_{B} may be, for example, 1 to 5, preferably 1 to 3, and more preferably 1 or 2. Examples of the substituent include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O-, (where Rₐ represents a hydrogen atom or a monovalent hydrocarbon group);
(vi) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a monovalent hydrocarbon group); and
(vii) a nitro group, a sulfate group, a sulfonate group, a cyano group, and a carboxyl group.

Definitions, examples, and preferred examples of the halogen atom, the monovalent hydrocarbon group, and the monovalent heterocyclic group in the substituent are similar to those explained for the above R, R_{A}, and R_{B}.

The aralkyl refers to arylalkyl. Definitions, examples, and preferred examples of the aryl and the alkyl in the arylalkyl are as described above. The aralkyl is preferably C₃₋₁₅ aralkyl. Examples of such an aralkyl include benzoyl, phenethyl, naphthylmethyl, and naphthylethyl.

Preferably, the substituent may be:
(i) a halogen atom;
(ii) a C₁₋₁₂ alkyl, a C₁₋₁₂ phenyl, or a C₁₋₁₂ naphthyl;
(iii) a C₃₋₁₅ aralkyl;
(iv) a 5- or 6-membered heterocycle;
(v) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(vi) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₁₂ alkyl); or
(vii) the same groups as listed in the above (vii).

More preferably, the substituent may be:
(i) a halogen atom;
(ii) a C₁₋₁₂ alkyl;
(iii) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₁₂ alkyl); or
(v) the same groups as listed in the above (vii).

Even more preferably, the substituent may be:
(i) a halogen atom;
(ii) a C₁₋₆ alkyl;
(iii) Rₐ-O-, Rₐ-C(=O)-, Rₐ-O-C(=O)-, or Rₐ-C(=O)-O-, (where Rₐ represents a hydrogen atom or a C₁₋₆ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₆ alkyl); or
(v) the same groups as listed in the above (vii).

Particularly preferably, the substituent may be:
(i) a halogen atom;
(ii) a C₁₋₄ alkyl;
(iii) Rₐ-O-, Rₐ-C(=O)-, Rₐ-O-C(=O)-, or Rₐ-C(=O)-O-, (where Rₐ represents a hydrogen atom or a C₁₋₄ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₄ alkyl); or
(v) the same groups as listed in the above (vii).

The affinity peptide indicated by Y has a binding region in a CH2 domain in an immunoglobulin unit containing two heavy chains and two light chains. As the affinity peptide, any peptide having a binding region in the CH2 domain in the immunoglobulin unit can be used. The affinity peptide may contain an amino acid residue containing a side chain containing a moiety capable of bonding with a carbonyl group (C=O) adjacent to Y (e.g., amino group, hydroxy group) and form an amide bond with a carbonyl group (C=O) adjacent to Y via an amino group in the side chain of the lysine residue. Preferably, the affinity peptide may contain a lysine residue and form an amide bond with a carbonyl group (C=O) adjacent to Y via an amino group in the side chain of the lysine residue. For such affinity peptides, various affinity peptides disclosed in WO2016/186206, WO2018/199337, WO2019/240287, WO2019/240288, and WO2020/090979 and literature cited in these international publications can be used.

In one enbodiment, the affinity peptide may comprise the amino acid sequence of the following (A) or analogous amino acid sequence thereof:
(A) (X0-3)a-C-Xaa1-Xaa2-Xaa3-Xaa4-Xaa5-Xaa6-I-I-W-C-(X0-3)b (SEQ ID NO: 1).

In addition, examples of the amino acid sequence of the following (A) or analogous amino acid sequence thereof includes the following:
(1) the amino acid sequences represented by formulae (I)-(V) described in WO2016/186206 (e.g., amino acid sequences of SEQ ID NOs: 1-17, 36 and 37 described in WO2016/186206);
(2) the amino acid sequence represented by formulae (i), (i-1), (i-1') and (i-1"), (i-2), and (ii)-(viii) described in WO2018/199337 (e.g., the amino acid sequences of SEQ ID NOs: 20-60, 73-91 and 94-105 described in WO2018/199337);
(3) the amino acid sequences represented by formulae (1-1)-(1-9) and (2-1) described in WO2019/240287 (e.g., the amino acid sequences of SEQ ID NOs: 5 and 8-92 described in WO2019/240287) ;
(4) the amino acid sequences represented by formulae (1-1)-(1-9) and (2-1) described in WO2019/240288 (e.g., the amino acid sequences of SEQ ID NOs: 15-24, 46-96, 99 and 108 described in WO2019/240288); and
(5) the amino acid sequence of (10)-(11) described in WO2020/090979 (e.g., the amino acid sequences of SEQ ID NOs: 24 and 25 described in WO2020/090979).

The affinity peptide comprising the amino acid sequence of (A) or analogous amino acid sequence thereof has a binding region at the same site in a CH2 domain of an immunoglobulin units, as can be understood from its conserved region. Thus, as is similar to a compound having an affinity peptide comprising the amino acid sequence of (A), a compound having an affinity peptide comprising the above analogous amino acid sequence binds to the same region in the CH2 domain in the immunoglobulin unit, and hence can highly control an average ratio of the bonding of the immunoglobulin unit and azide group-containing modifying groups within a desired range while selectively modifying the lysine residue at the position 246/248 in a human IgG heavy chain. The affinity peptide preferably comprises the amino acid sequence of (A) and more preferably comprises the amino acid sequence of (1):
(1) RGNCAYHKGQIIWCTYH (SEQ ID NO: 2).

In another embodiment, the affinity peptide may comprise the amino acid sequence of the following (B) or analogous amino acid sequence thereof:
(B) PNLNEEQRNARIRSI (SEQ ID NO: 3).

In addition, examples of the amino acid sequence of (B) or analogous amino acid sequences thereof include the following:
(1) the amino acid sequences of (a)-(d) described in WO2018/199337 (e.g., the amino acid sequences of SEQ ID NOs: 61-72 and 92 described in WO2018/199337);
(2) the amino acid sequences of (a)-(b) described in WO2019/240288 (e.g., the amino acid sequence of SEQ ID NOs: 5-8, 11-14, 37-45, 97, 98 and 100 described in WO2019/240288); and
(3) the amino acid sequences of (1)-(9) described in WO2020/090979 (e.g., the amino acid sequences of SEQ ID NOs: 5-10, 22, 23 and 51-53 described in WO2020/090979).

The affinity peptide comprising the amino acid sequence of (B) or analogous amino acid sequence thereof has a binding region at the same site in a CH2 domain of an immunoglobulin units, as can be understood from its conserved region. Thus, as is similar to a compound having an affinity peptide comprising the amino acid sequence of (B), a compound having an affinity peptide comprising the above analogous amino acid sequence binds to the same region in the CH2 domain in the immunoglobulin unit, and hence can highly control an average ratio of the bonding of the immunoglobulin unit and azide group-containing modifying groups within a desired range while selectively modifying the lysine residue at the position 288/290 in a human IgG heavy chain. The affinity peptide preferably comprises the amino acid sequence of (B) and more preferably comprises the amino acid sequence of (1) to (3):
(1) FNMQCQRRFYEALHDPNLNEEQRNARIRSIKDDC (SEQ ID NO: 4);
(2) FNMQCQRRFYEALHDPNLNEEQRNARIRSIKEEC (SEQ ID NO: 5); or
(3) MQCQRRFYEALHDPNLNEEQRNARIRSI(Orn)EEC (SEQ ID NO: 6).

At least two separated cysteine residues in each amino acid sequence of the affinity peptide can form a cyclic peptide by a disulfide bond. Alternatively, in the above peptide, the thiol groups in the two cysteine residues may be linked by a linker (e.g., a carbonyl group-containing linker represented by the following).

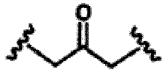

The broken line portion of the carbonyl group-containing linker represented above means the bonding portion with the thiol group. The linker is more stable to reduction reactions and the like than ordinary disulfide bonds. Such peptides can be prepared, for example, by the methods described in WO2016/186206.

The amino acids constituting the affinity peptide may be either L-form or D-form, and L-form is preferable (in the examples, all the amino acid residues constituting the peptide are L-form). The affinity peptide may be linked to the compound of formula (I) or a salt thereof by modifying a specific amino acid residue with a cross-linking agent. Examples of such a specific amino acid residue include a lysine residue, an aspartic acid residue, and a glutamic acid residue; preferred is a lysine residue. Examples of the cross-linking agent include cross-linking agents comprising preferably two or more succinimidyl groups such as disuccinimidyl glutarate (DSG) and disuccinimidyl suberate (DSS); cross-linking agents comprising preferably two or more imide acid portions such as dimethyl adipimidate·2HCl (DMA), dimethyl pimelimidate·2HCl (DMP), and dimethyl suberimidate·2HCl (DMS); and cross-linking agents having an SS bond such as dimethyl 3,3'-dithiobispropionimidate·2HCl (DTBP) and dithiobis(succinimidyl propionate) (DSP) (e.g., WO 2016/186206).

In the affinity peptide, a terminal amino group and a terminal carboxy group of the peptide may be protected. Examples of a protecting group for the N-terminal amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group). The protecting group for the N-terminal amino group is preferably an acetyl group. Examples of a protecting group for the C-terminal carboxy group include a group capable of forming an ester or an amide. Examples of the group capable of forming an ester or an amide include an alkyloxy group (e.g., methyloxy, ethyloxy, propyloxy, butyloxy, pentyloxy, and hexyloxy), an aryloxy group (e.g., phenyloxy and naphthyloxy), an aralkyloxy group (e.g., benzyloxy), and an amino group. The protecting group for the C-terminal carboxy group is preferably an amino group.

M indicates a trivalent group linking the carbon atom in C=O and the carbon atom in C=W which are adjacent to M, i.e., a first carbon atom adjacent to M (the carbon atom in C=O) and a second carbon atom adjacent to M (the carbon atom in C=W) via a main chain portion consisting of 3 to 5 carbon atoms. Thus, no heteroatoms are included in the main chain portion linking the first and second carbon atoms adjacent to M. Use of compounds having such structures can expect the following advantages.

First, the use of the above compounds makes it easier to highly control the average ratio of bonding of an antibody and an azide group-containing modifying group within a desired range (1.5 to 2.5). In the present invention, the average ratio of bonding of an antibody and a given group (e.g., an azide group-containing modifying group) can be confirmed by analyzing MS analysis data with a DAR calculator (Agilent software).

Second, the use of the above compounds allows for selective modification of different lysine residues in the heavy chain of the immunoglobulin unit. For example, by using an affinity peptide having a certain kind of amino acid sequence as Y, the lysine residue at the position 246/248 in the human IgG heavy chain can be regioselectively modified. In addition, by using an affinity peptide having another kind of amino acid sequence as Y, the lysine residue at the position 288/290 in the human IgG heavy chain can be regioselectively modified.

Third, antibodies prepared by the use of the above compounds can exhibit excellent stability.

The main chain portion consisting of 3 to 5 carbon atomes are composed of a chain structure, a cyclic structure, or a structure containing a combination thereof. When the main chain portion has a chain structure comprising no cyclic structure, the number of carbon atoms of the main chain portion can be determined by counting the number of carbon atoms in the chain structure. On the other hand, when the main chain portion has a structure comprising a cyclic structure, the number of predetermined carbon atoms constituting the cyclic structure can be determined by counting the number of carbon atoms in the main chain portion. Specifically, the number of carbon atoms of the main chain portion in the cyclic structure can be determined by counting the number of carbon atoms of the shortest route linking two bonds in the cyclic structure (see, e.g., the following thick routes (a) to (d)). When the main chain has a structure comprising a combination of a chain structure and a cyclic structure, the number of atoms of the main chain can be determined by adding the number of atoms in the chain structure not comprising the cyclic structure to the number of atoms of the shortest route linking two bonds in the cyclic structure. 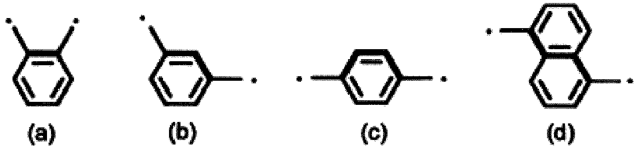
- is a bond.

In the case of (a), the shortest route is the thick route, and thus the number of atoms in the divalent cyclic structure counted as the number of carbon atoms of the main chain portion is two.

In the case of (b), the shortest route is the thick route, and thus the number of atoms in the divalent cyclic structure counted as the number of carbon atoms of the main chain portion is three.

In the case of (c), both routes are the shortest routes (equidistant), and thus the number of carbon atoms in the divalent cyclic structure counted as the number of carbon atoms in the main chain portion is four.

In the case of (d), a route of a condensation site is the shortest routes, and thus the number of carbon atoms in the divalent cyclic structure counted as the number of atoms in the main chain is four.

Since the main chain portion consisting of 3 to 5 carbon atoms links the first carbon atom (the carbon atom in C=O) adjacent to M and the second carbon atom (the carbon atom in C=W) adjacent to M, it can be considered as a divalent group in relation to such first and second carbon atoms. On the other hand, M is a trivalent group that binds not only to such first and second carbon atoms but also to Lb. Therefore, M can be expressed as a trivalent group where one hydrogen atom is removed from the main chain portion (divalent group) consisting of 3 to 5 carbon atoms. Here, the main chain portion (divalent group) consisting of 3 to 5 carbon atoms may be composed of the following:
(1) a divalent liner hydrocarbon group having 3 to 5 carbon atoms;
(2) a divalent group in which a divalent cyclic hydrocarbon group and a divalent linear hydrocarbon group having 1 to 4 carbon atoms (one or two) are linked;
(3) a divalent cyclic hydrocarbon group; and
(4) a divalent group with two divalent cyclic hydrocarbon groups linked (i.e., a divalent bicyclo structure).

The liner hydrocarbon group having 3 to 5 carbon atoms is a linear alkylene having 3 to 5 carbon atoms, a liner alkenylene having 3 to 5 carbon atoms, or a linear alkynylene having 3 to 5 carbon atoms.

The linear alkylene having 3 to 5 carbon atoms is n-propylene, n-butylene, or n-pentylene.

The linear alkenylene having 3 to 5 carbon atoms is n-propenylene, n-butenylene, or n-pentenylene.

The Linear alkynylene having 3 to 5 carbon atoms is n-propynylene, n-butynylene, or n-pentynylene.

For the divalent straight chain hydrocarbon group having 3 to 5 carbon atoms, the liner alkylene having 3 to 5 carbon atoms is preferred.

The divalent cyclic hydrocarbon group is an arylene, or a divalent non-aromatic cyclic hydrocarbon group. By appropriately setting two bonds in such a divalent cyclic hydrocarbon group, the number of atoms constituting the main chain can be set to 3 to 5.

For the arylene, an arylene having 6 to 14 carbon atoms is preferred, an arylene having 6 to 10 carbon atoms is more preferred, and an arylene having 6 carbon atoms is particularly preferred. Examples of the arylene includes phenylene, naphthylene, and anthracenylene

For the divalent non-aromatic cyclic hydrocarbon group, a divalent non-aromatic cyclic hydrocarbon group having from 3 to 12 carbon atoms which is a monocyclic or polycyclic is preferred, a divalent non-aromatic cyclic hydrocarbon group having 4 to 10 carbon atoms which is a monocyclic or polycyclic is more preferred, and a divalent non-aromatic cyclic hydrocarbon group having 5 to 8 carbon atoms which is a monocyclic is particularly preferred. Examples of the ivalent non-aromatic cyclic hydrocarbon groups include cyclopropylene, cyclobutylene, cyclopentylene, cyclohexylene, cycloheptylene, and cyclooctylene.

For the divalent cyclic hydrocarbon group, arylene is preferred.

The divalent linear hydrocarbon group having 1 to 4 carbon atoms is a linear alkylene having 1 to 4 carbon atoms, a linear alkenylene having 2 to 4 carbon atoms, or a linear alkynylene having 2 to 4 carbon atoms.

The linear alkylene having 1 to 4 carbon atoms is methylene, ethylene, n-propylene, or n-butylene.

The linear alkenylene having 1 to 4 carbon atoms is ethenylene, n-propynylene, or n-butenylene.

The linear alkynylene having 1 to 4 carbon atoms is ethynylene, n-propynylene, or n-butynylene.

For as the divalent liner hydrocarbon group having 1 to 4 carbon atoms, the linear alkylene having 1 to 4 carbon atoms is preferred.

Among these (1) to (4), the main chain portion (divalent group) consisting of 3 to 5 carbon atoms is preferably (1) to (3), and (1) and (2) are more preferred.

In a specific embodiment, the main chain portion consisting of 3 to 5 carbon atoms in M may be a portion comprising a linear alkylene, or a ring-constituting carbon atom, or a combination thereof. Thus, the main chain portion (divalent group) consisting of 3 to 5 carbon atoms may be composed of the following:
(1') a linear alkylene having 3 to 5 carbon atoms;
(2') a divalent group in which a divalent cyclic hydrocarbon group and a linear alkylene (one or two) having from 1 to 4 carbon atoms are linked;
(3') a divalent cyclic hydrocarbon group; and
(4') a divalent group with two divalent cyclic hydrocarbon groups linked (i.e., a divalent bicyclo structure).

Among these (1') to (4'), the main chain portion (divalent group) consisting of 3 to 5 carbon atoms is preferably (1')-(3') and more preferably (1') and (2'). Therefore, M may be a trivalent group where one hydrogen atom is removed from a main chain portion (divalent group) consisting of 3 to 5 carbon atoms and represented by formula (i) or (ii): 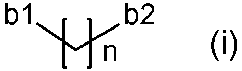 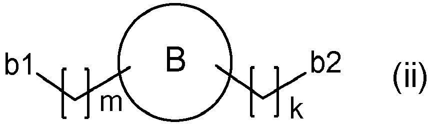 wherein
n indicates an integer from 3 to 5,
m indicates an integer from 0 to 4,
k indicates an integer from 0 to 4,
the ring B indicates a divalent cyclic hydrocarbon group,
b1 indicates the bond to the carbon atom in C=O adjacent to M,
b2 indicates the bond to the carbon atom in C=W adjacent to M.

The divalent cyclic hydrocarbon group indicated by ring B is similar to that described above. When the main chain portion (divalent group) consisting of 3 to 5 carbon atoms is represented by formula (ii), M is preferably a trivalent group obtained by removing one hydrogen atom from the ring B in the main chain portion (divalent group) consisting of 3 to 5 carbon atoms, which is represented by formula (ii).

Preferably, the main chain portion consisting of 3 to 5 carbon atoms (divalent group) represented by formula (ii) may be represented by formula (ii'). SELECTED DRAWING: Figure 5 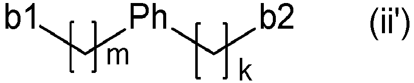 wherein
m indicates an integer from 0 to 4,
k indicates an integer from 0 to 4,
Ph indicates phenylene,
b1 indicates a bond to the carbon atom in C=O adjacent to M,
b2 indicates a bond to the carbon atom in C=W adjacent to M.

Ph can be bonded with two linear alkylene at the meta, ortho, or para position. When the main chain portion (divalent group) consisting of 3 to 5 carbon atoms is represented by formula (ii'), M is preferably a trivalent group obtained by removing one hydrogen atom from phenylene in the main chain portion (divalent group) consisting of 3 to 5 carbon atoms, which is represented by formula (ii').

More preferably, the main chain portion (divalent group) consisting of 3 to 5 carbon atoms, which is represented by formula (ii) may be represented by formula (ii"). 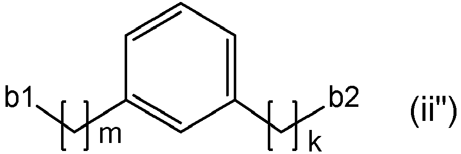 wherein
m indicates an integer from 0 to 2,
k indicates an integer from 0 to 2,
b1 indicates a bond to the carbon atom in C=O adjacent to M,
b2 indicates a bond to the carbon atom in C=W adjacent to M.

When the main chain portion (divalent group) consisting of 3 to 5 carbon atoms is represented by formula (ii''), M is preferably a trivalent group obtained by removing one hydrogen atom from phenylene (preferably carbon atoms in the ortho position to the two alkylene) in the main chain portion (divalent group) consisting of 3 to 5 carbon atoms, which is represented by formula (ii").

W indicates an oxygen atom or a sulfur atom, preferably an oxygen atom.

La and Lb each independently indicate a bond or a divalent group.

La is included in an antibody intermediate produced by a compound represented by formula (I), but is a portion not included in an azide group-introduced antibody derivative and a conjugate of an antibody and a functional substance, which are produced from the antibody intermediate. Therefore, the stability of La is less likely to be a problem for antibodies produced by compounds represented by formula (I).

Lb is portion included in an antibody intermediate, an azide group-introduced antibody derivative, and a conjugate of an antibody and a functional substance, which are produced by a compound represented by formula (I). In addition, Lb is a portion which acts as a linker linking an antibody and an azide group in an azide group-introduced antibody derivative.

The main chain in the divalent group indicated by La and Lb is preperably composed of a divalent linear hydrocarbon group, a divalent cyclic hydrocarbon group, a divalent heterocyclic group, -C(=O)-, -C(=S)- ,-NR_{d}- (R_{d} indicates a hydrogen atom or a substituent), -O-, -S-, or a group consisting of two or more thereof (e.g., 2 to 15, preferably 2 to 10, more preferably 2 to 8, even more preferably 2 to 6, particularly preferably 2, 3, 4 or 5). The number of atoms constituting the main chain in the divalent group indicated by La and Lb is preferably from 1 to 17, more preferably from 1 to 15. In addition, the lower limit of the number of atoms constituting the main chain in the divalent group indicated by La and Lb may be 2 or more, 3 or more, 4 or more, 5 or more, 6 or more, or 7 or more. The upper limit of the number of atoms constituting the main chain in the divalent group indicated by La and Lb may be 14 or less, 13 or less, 12 or less, 11 or less, or 10 or less. When the main chain is a structure comprising a cyclic structure, the number of atoms of the main chain in the cyclic structure can be determined as described above.

The divalent linear hydrocarbon group is a linear alkylene, a linear alkenylene, or a linear alkynylene.

The linear alkylene is a C₁₋₆ linear alkylene, and is preferably a C₁₋₄ linear alkylene. Examples of the linear alkylene include methylene, ethylene, n-propylene, n-butylene, n-pentylene, and n-hexylene.

The linear alkenylene is a C₂₋₆ linear alkenylene, and is preferably a C₂₋₄ linear alkenylene. Examples of the linear alkenylene include ethylenylene, n-propynylene, n-butenylene, n-pentenylene, and n-hexenylene.

The linear alkynylene is a C₂₋₆ linear alkynylene, and is preferably a C₂₋₄ linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C₆₋₁₄ arylene, more preferably a C₆₋₁₀ arylene, and particularly preferably a C₆ arylene. Examples of the arylene include phenylene, naphthylene, and anthracenylene.

The divalent nonaromatic cyclic hydrocarbon group is preferably a C₃₋₁₂ monocyclic or polycyclic divalent nonaromatic cyclic hydrocarbon group, more preferably a C₄₋₁₀ monocyclic or polycyclic divalent nonaromatic cyclic hydrocarbon group, and particularly preferably a C₅₋₈ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C₃₋₁₅ divalent aromatic heterocyclic group, more preferably a C₃₋₉ divalent aromatic heterocyclic group, and particularly preferably a C₃₋₆ divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₃₋₁₅ nonaromatic heterocyclic group, more preferably a C₃₋₉ nonaromatic heterocyclic group, and particularly preferably a C₃₋₆ nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

In a specific embodiments, the carbonyl group adjacent to La may form an amide bond with an amino group in a side chain of a lysine residue in the affinity peptide.

In a specific embodiment, the main chain in the divalent group indicated by Lb may do not comprise a cyclic structures from the viewpoint such as ease of synthesis. Thus, the main chain in the divalent group indicated by Lb represents a divalent linear hydrocarbon group, -C(=O)-, -C (=S)-, -NR_{d}- (R_{d} indicates a hydrogen atom or a substituent), -O-, -S-, or a group consistng of a combination of two or more thereof (e.g., 2, 3, 4, or 5). Preferably, from the viewpoint of ensuring stability in Lb, -C(=O)-NR_{d}-, -C(=O)-O-, -C(=O)-S-, -C(=S)-NR_{d}-, -C(=S)-O-, and -C(=S)-S- is excluded from the above combination. The number of atoms constituting the main chain in the divalent group indicated by Lb is preferably 1-12, more preferably 1-10.

The groups constituting the main chain in the divalent group indicated by La and Lb may have, for example, 1 to 5, preferably 1 to 3, and more preferably 1 or 2 substituents. Further, R_{d}, which is one of the groups constituting the main chain in the first linker and the second linker, may correspond to a substituent. Examples of such substituents include:
(i') a halogen atom;
(ii') a monovalent hydrocarbon group;
(iii') an aralkyl;
(iv') a monovalent heterocycle;
(v') Rₑ-O-, R,-C(=O)-, Rₑ-O-C(=O)-, or Rₑ-C(=O)-O-, (where Rₑ represents a hydrogen atom or a monovalent hydrocarbon group);
(vi') NR_{f}R_{g}-, NR_{f}R_{g}-C(=O)-, NR_{f}R_{g}-C(=O)-O-, or R_{f}-C(=O)-NR_{g}-, (where R_{f} and R_{g} are the same as or different from each other, and each represent a hydrogen atom or a monovalent hydrocarbon group); or
(vii') Nitro group, sulfate group, sulfonic acid group, cyano group, and carboxyl group.

Definitions, examples, and preferred examples of the halogen atoms, monovalent hydrocarbon groups, aralkyl, and monovalent heterocyclic groups in the substituents are similar to that of the halogen atom, monovalent hydrocarbon group, aralkyl, and monovalent heterocyclic group described for R, R_{A}, and R_{B} and (i) to (iv), respectively.

Preferably, the substituents may be:
(i') a halogen atom;
(ii') a C₁₋₁₂ alkyl, a phenyl, or a naphthyl;
(iii') a C₃₋₁₅ aralkyl;
(iv') a 5- or 6-membered heterocycle;
(v') Rₑ-O-, Rₑ-C(=O)-, Rₑ-O-C(=O)-, or Rₑ-C(=O)-O-, (where Rₑ represents a hydrogen atom or a C₁₋₁₂ alkyl);
(vi') NR_{f}R_{g}-, NR_{f}R_{g}-C(=O)-, NR_{f}R_{g}-C(=O)-O-, or R_{f}-C(=O)-NR_{g}- (where R_{f} and R_{g} are the same as or different from each other, and each represent a hydrogen atom or a C₁₋₁₂ alkyl); or
(vii') the same groups as listed in the above (vii').

More preferably, the substituent may be:
(i') a halogen atom;
(ii') a C₁₋₁₂ alkyl;
(iii') Rₑ-O-, Rₐ-C(=O)-, Rₐ-O-C(=O)-, or Rₐ-C(=O)-O-, (where Rₐ represents a hydrogen atom or a C₁₋₁₂ alkyl);
(iv') NR_{f}R_{g}-, NR_{f}R_{g}-C(=O)-, NR_{f}R_{g}-C(=O)-O-, or R_{f}-C(=O)-NR_{g}- (where R_{f} and R_{g} are the same as or different from each other, and each represent a hydrogen atom or a C₁₋₁₂ alkyl); or
(v') the same groups as listed in the above (vii').

Even more preferably, the substituent may be:
(i') a halogen atom;
(ii') a C₁₋₆ alkyl;
(iii') Rₑ-O-, Rₑ-C(=O)-, Rₑ-O-C(=O)-, or Rₑ-C(=O)-O-, (where Rₑ represents a hydrogen atom or a C₁₋₆ alkyl);
(iv') NR_{f}R_{g}-, NR_{f}R_{g}-C(=O)-, NR_{f}R_{g}-C(=O)-O-, or R_{f}-C(=O)-NR_{g}-, (where R_{f} and R_{g} are the same as or different from each other, and each represent a hydrogen atom or a C₁₋₆ alkyl); or
(v') the same groups as listed in the above (vii').

Particularly preferably, the substituent may be:
(i') a halogen atom;
(ii') a C₁₋₄ alkyl;
(iii') Rₑ-O-, or Rₑ-C(=O)- (where Rₑ represents a hydrogen atom or a C₁₋₄ alkyl);
(iv') NR_{f}R_{g}- (where R_{f} and R_{g} are the same as or different from each other, and each represent a hydrogen atom or a C₁₋₄ alkyl); or
(v') the same groups as listed in the above (vii').

In a specific embodiment, the compound represented by formula (I) can be defined by the number of atoms of the main chain linking M and Y. The main chain linking M and Y corresponds to the main chain "C-S-La-C" in the structure "C(=W)-S-La-C(=O)" linking M and Y. The number of atoms of the main chain linking M and Y may be 6 to 20. Compounds represented by formula (I) wherein the number of atoms of the main chain linking M and Y have such numbers are easy to synthesize. In addition, it is easy to highly control the average ratio of bonding the antibody and the azide group-containing modifying group within a desired range, and moreover, the versatility of regioselective modification of the immunoglobulin unit is excellent. The number of atoms of the main chain linking M and Y may also be 6 to 19, 6 to 18, 6 to 17, 6 to 16, 6 to 15, 6 to 14, 6 to 13, 6 to 12, 6 to 11, 6 to 10, 7 to 19, 7 to 18, 7 to 17, 7 to 16, 7 to 15, 7 to 14, 7 to 13, 7 to 12, 7 to 11, 7 to 10, 8 to 19, 8 to 18, 8 to 17, 8 to 16, 8 to 15, 8 to 14, 8 to 13, 8 to 12, 8 to 11, or 8 to 10.

The compound or salt thereof of the present invention represented by the formula (I) can be obtained by, for example, synthesizing a compound or salt thereof in which the portion Y is substituted with a leaving group (preferably, a leaving group having a higher leaving ability than X, or hydroxy) in the compound or salt thereof represented by formula (I), and then reacting the synthesized compound or salt thereof with an affinity peptide. For example, such a reaction can be carried out in a suitable reaction system (e.g., an organic solvent or aqueous solution or a mixed solvent thereof) at a suitable temperature (e.g., about 15 to 200°C). The reaction system may include a suitable catalyst. The reaction time is, for example, 1 minute to 20 hours, preferably 10 minutes to 15 hours, more preferably 20 minutes to 10 hours, and even more preferably 30 minutes to 8 hours.

Preferably, the compound or salt thereof in which the portion Y is substituted with a leaving group may be a compound or salt thereof represented by the following formula (I'): 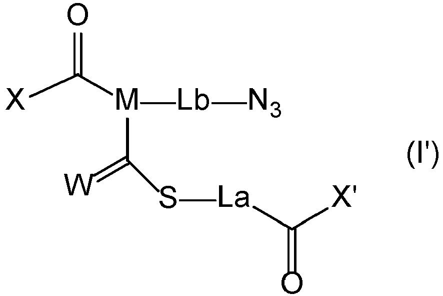 wherein
X indicates a leaving group,
X' indicates a leaving group having a leaving ability which is higher than that of the leaving group X,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

The leaving group having a leaving ability which is higher than that of the leaving group X, which is indicated by X' is not particularly limited as long as it is a leaving group that has a leaving ability which is higher than the leaving group X, and examples of such leaving group include a pentafluorophenyloxy group, a tetrafluorophenyloxy group, a para-nitrophenyloxy group, and an N-succinimidyloxy group.

The definitions, examples, and preferred examples of symbols, terms and expressions such as X (leaving group), M (trivalent group), W (oxygen atom or sulfur atom), La (bond or divalent group), Lb (bond or divalent group) in formula (I') are similar to those of the above formula.

The compound or salt thereof represented by formula (I') is useful as, for example, a synthetic intermediate for efficiently producing a compound or salt thereof represented by formula (I).

The compound or salt thereof represented by formula (I') can be produced from a compound or salt thereof represented by the following formula (I"): 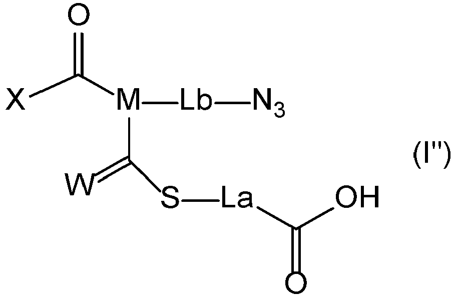 wherein
X indicates a leaving group,
OH indicates a hydroxy group,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

The definitions, examples, and preferred examples of symbols, terms and expressions such as X (leaving group), M (trivalent group), W (oxygen atom or sulfur atom), La (bond or divalent group), Lb (bond or divalent group) in formula (I") are similar to those of the above formula.

The compound or salt thereof represented by formula (I') can be obtained by reacting the compound or salt thereof represented by the formula (I") with a carboxyl group modifying reagent. Examples of the carboxyl group modifying reagent include a pentafluorophenylation reagent (e.g., pentafluorophenyl trifluoroacetate), a tetrafluorophenylation reagent (e.g., trifluoroacetate tetrafluorophenyl), and a paranitrophenylation reagent (e.g., trifluoroacetate paranitrophenyl), N-succinimidylation reagent (e.g., trifluoroacetate N-succinimidyl). For example, such a reaction may be carried out at a suitable temperature (e.g., about -10 to 30°C) in a suitable organic solvent system (e.g., an organic solvent containing an alkyl halide (e.g., methyl halide) such as CH₂Cl₂ and an amine such as triethylamine). The reaction time is, for example, 1 minute to 20 hours, preferably 10 minutes to 15 hours, more preferably 20 minutes to 10 hours, and even more preferably 30 minutes to 8 hours.

The compound or salt thereof represented by formula (I") is useful as, for example, a synthetic intermediate for efficiently producing the compound or salt thereof represented by the formula (I'). Alternatively, the compound or salt thereof represented by formula (I") is useful as, for example, a synthetic intermediate for directly and efficiently producing the compound or salt thereof represented by the formula (I).

The compound represented by formula (I") can be obtaned by various synthetic methods shown in Examples.

The determination of the formation of the above compounds or salts thereof, which depends on its specific raw materials and the molecular weight of a product, can be performed by electrophoresis, chromatography (e.g., gel permutation chromatography, ion-exchange chromatography, reversed phase column chromatography, and HPLC), or NMR or mass spectrometry. The above compounds or salts thereof can be purified as appropriate by any method such as chromatography (e.g., the chromatography described above and affinity chromatography).

### 3. Antibody Intermediate or salt thereof

The present invention provides an antibody intermediate or salt thereof comprising a structural unit represented by the following formula (II): 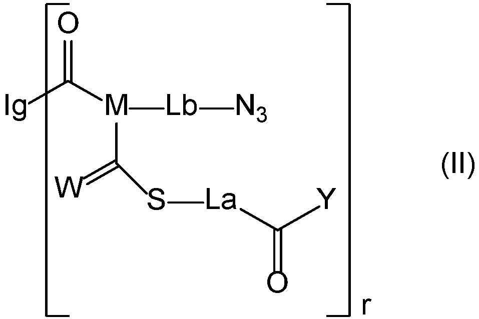 wherein
Ig indicates an immunoglobulin unit comprising two heavy chains and two light chains, and forms an amide bond with a carbonyl group adjacent to Ig via an amino group in a side chain of lysine residues in the two heavy chains,
Y indicates an affinity peptide having a binding region in a CH2 domain in the immunoglobulin unit,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group,
Lb indicates a bond or a divalent group, and
the average ratio r of the amide bond per the two heavy chains is from 1.0 to 3.0.

The immunoglobulin unit indicated by Ig is as described above. The definitions, examples, and preferred examples of symbols, terms and expressions such as Y (affinity peptide), M (trivalent group), W (oxygen atom or sulfur atom), La (bond or divalent group) and Lb (bond or divalent group) in formula (II) are the same as those of the above formula.

In the formula (II), the average ratio (r) of the amide bond per two heavy chains indicates the average ratio of the bond between the immunoglobulin unit and the azide group-containing group (number of azide group -containing group/immunoglobulin unit). Such an average ratio is 1.0 to 3.0, and preferably 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio is preferably 1.6 to 2.4, more preferably 1.7 to 2.3, even more preferably 1.8 to 2.2, and particularly preferably 1.9 to 2.1. A lysine residue having a side chain comprising an amino group which forms an amide bond with a carbonyl group adjacent to Ig may be modified so as to indicate an average ratio of such values at a desired one position in the two heavy chains (e.g., one position selected from the group consisting of the positions 246/248, 288/290, and 317 in a human IgG heavy chain according to EU numbering). In addition, where the two heavy chains are each modified at two or more positions, the lysine residue having the side chain containing the amino group which forms the amide bond with the carbonyl group adjacent to Ig may be modified so as to indicates an average ratio of such values at each position in a desired two positions in the two heavy chains (e.g., one position selected from the group consisting of the positions 246/248, 288/290, and 317 in the human IgG heavy chain according to EU numbering; preferably the positions 246/248 and 288/290).

The antibody intermediate or salt thereof of the present invention can be obtained by reacting the compound or salt thereof of the present invention with an antibody containing the above-mentioned immunoglobulin unit. In the reaction, first, the compound or salt thereof of the present invention is mixed with the antibody. This allows the compounds or salt thereof of the present invention to associate with the antibody via an affinity peptide that has an affinity for the antibody. Next, after antibody association, a carbonyl group (X-C=O) with a leaving group (X) can be regioselectively reacted with the amino group in the side chain of a lysine residue in a heavy chain of the antibody. By such a reaction, the amino group and the carbon atom of the carbonyl group are bonded, and the leaving group (X) is eliminated from the carbonyl group to obtain the antibody intermediate or salt thereof of the present invention. The molar ratio of the compound or salt thereof of the present invention to the antibody (the compound or salt thereof of the present invention/antibody) to the antibody in the reaction is not particularly limited, since it varies depending on factors such as the type of the compound or salt thereof of the present invention and antibody. It is, for example, 1 to 100, preferably 2 to 80, more preferably 4 to 60, even more preferably 5 to 50, and particularly preferably 6 to 30.

Such a reaction can be appropriately carried out under a condition that cannot cause protein denaturation/degradation (e.g., cleavage of amide bond) (mild conditions). For example, such a reaction can be carried out at room temperature (e.g., about 15-30°C) in a suitable reaction system such as buffer. The pH of the buffer is, for example, 5 to 9, preferably 5.5 to 8.5, and more preferably 6.0 to 8.0. The buffer may contain a suitable catalyst. The reaction time is, for example, 1 minute to 20 hours, preferably 10 minutes to 15 hours, more preferably 20 minutes to 10 hours, and even more preferably 30 minutes to 8 hours. For details of such a reaction, see, e.g., G.I.J.L. Bernardes et al., Chem. Rev., 115, 2174(2015); J.L. Bernardes et al., Chem. Asian. J., 4, 630 (2009); B. G. Devices et al., Nat. Commun., 5, 4740(2014); Wagner et al., Bioconjugate. Chem., 25, 825(2014).

The determination of the formation of the antibody intermediate or salt thereof, which depends on its specific raw materials and the molecular weight of a product, can be performed by electrophoresis, chromatography (e.g., gel permutation chromatography, ion-exchange chromatography, HPLC), or mass spectrometry, for example, and preferably mass spectrometry. Determination of regioselectivity can be performed by peptide mapping, for example. The peptide mapping can be performed by protease treatment and mass spectrometry, for example. For the protease, an endoprotease is preferred. Examples of such an endoprotease include trypsin, chymotrypsin, Glu-C, Lys-N, Lys-C, and Asp-N. Determination of the number of the introduced affinity peptide can be performed by electrophoresis, chromatography, or mass spectrometry, for example, and preferably mass spectrometry. The antibody intermediate or salt thereof can be purified as appropriate by any method such as chromatography (e.g., chromatography described above and affinity chromatography).

### 4. Azide group-introduced antibody derivative or salt thereof

The present invention provides a azide group-introduced antibody derivative or salt thereof, which comprises a structural unit represented by the following formula (III): 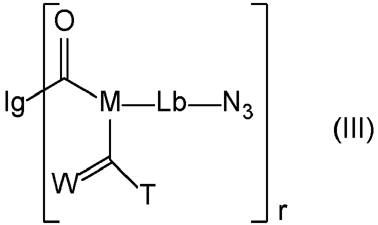 wherein
Ig indicates an immunoglobulin unit comprising two heavy chains and two light chains, and forms an amide bond with a carbonyl group adjacent to Ig via an amino group in a side chain of lysine residues in the two heavy chains,
M indicates a trivalent group linking the carbon atom in C=O adjacent to M and the carbon atom in C=W via a main chain portion consisting of 3 to 5 carbon atoms,
O indicates an oxygen atom,
T indicates a monovalent group,
W indicates an oxygen atom or a sulfur atom,
N₃ indicates an azide group,
Lb indicates a bond or a divalent group, and
the average ratio r of the amide bond per the two heavy chains is from 1.0 to 3.0.

The immunoglobulin unit indicated by Ig is as described above. The definitions, examples, and preferred examples of symbols, terms and expressions such as M (trivalent group), W (oxygen atome or sulfur atom) and Lb (bond or divalent group) in formula (III) are the same as those in the above formula.

T represents a monovalent group. T can be formed by a cleavage reaction between the carbon atom and sulfur atom in the partial structure "C(=W)-S" in the compound represented by formula (II). Thus, a suitable monovalent group can be employed as T depending on the type of cleavage reaction.

In a specific embodiment, the monovalent group indicated by T may be a hydroxyamino group which may be substituted. The hydroxyamino group which may be substituted can be represented by the following formula (α) :

NR₁-OR₂ (α)

wherein
R₁ and R₂ may be the same or different and represent a hydrogen atom or a monovalent hydrocarbon group which may be substituted.

The definitions, examples, and preferred examples of monovalent hydrocarbon groups and substituents are as described above.

In the formula (III), the average ratio (r) of the amide bond per two heavy chains indicates the average ratio of the bonds between the immunoglobulin unit and the azide group-containing group (number of azide group-containing groups/immunoglobulin unit). Such an average ratio is 1.0 to 3.0 and preferably 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio is preferably 1.6 to 2.4, more preferably 1.7 to 2.3, even more preferably 1.8 to 2.2, and particularly preferably 1.9 to 2.1. A lysine residue having a side chain comprising an amino group which forms an amide bond with a carbonyl group adjacent to Ig may be modified so as to indicate an average ratio of such values at a desired one position in the two heavy chains (e.g., one position selected from the group consisting of the positions 246/248, 288/290, and 317 in a human IgG heavy chain according to EU numbering). In addition, where the two heavy chains are each modified at two or more positions, the lysine residue having the side chain containing the amino group which forms the amide bond with the carbonyl group adjacent to Ig may be modified so as to indicates an average ratio of such values at each position in a desired two positions in the two heavy chains (e.g., one position selected from the group consisting of the positions 246/248, 288/290, and 317 in the human IgG heavy chain according to EU numbering; preferably the positions 246/248 and 288/290).

The azide group-introduced antibody derivative or salt thereof of the present invention can be obtained by subjecting the antibody intermediate or salt thereof of the present invention to a thioester cleavage reaction. The thioester cleavage reaction can be carried out under conditions that cannot cause denaturation/degradation of proteins (immunoglobulin/antibody) (e.g., cleavage of amide bonds) (mild conditions as described above). More specifically, it can be cleaved by stirring for 1 hour in a hydroxylamine hydrochloride solution in the range of pH 4.0 to pH 8.0, 10 mM to 10 M (e.g., Vance, Net al., Bioconjugate Chem. 2019, 30, 148-160.).

The determination of the formation of the azide group-introduced antibody derivative or salt thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity can be performed by peptide mapping, for example. The peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of the introduced thiol group can be performed by electrophoresis, chromatography, or mass spectrometry, for example, and preferably mass spectrometry. The azide group-introduced antibody derivative or salt thereof can be purified as appropriate by any method such as chromatography (e.g., chromatography described above and affinity chromatography).

### 5. Conjugate of antibody and functional substances or salt thereof

The present invention provides a conjugate of an antibody and a functional substances or a salt thereof, which comprises a structural unit represented by the following formula (IV): 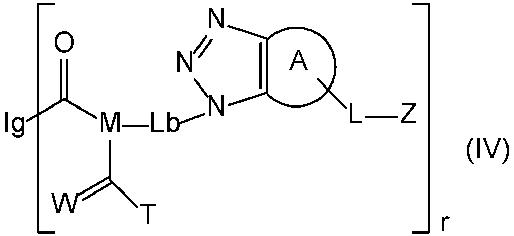 wherein
Ig indicates an immunoglobulin unit comprising two heavy chains and two light chains, and forms an amide bond with a carbonyl group adjacent to Ig via an amino group in a side chain of lysine residues in the two heavy chains,
M indicates a trivalent group linking the carbon atom in C=O adjacent to M and the carbon atom in C=W via a main chain portion consisting of 3 to 5 carbon atoms,
O indicates an oxygen atom,
T indicates a monovalent group,
W indicates an oxygen atom or a sulfur atom,
N indicates a nitrogen atom,
Lb indicates a bond or a divalent group,
Z indicates a functional substance,
L indicates a bond or a divalent group,
the ring A indicates a ring fused with the triazole ring, and
the average ratio r of the amide bond per the two heavy chains is from 1.0 to 3.0.

The immunoglobulin unit indicated by Ig is as described above. The definitions, examples, and preferred examples of symbols, terms and expressions such as M (trivalent group), W (oxygen atom or sulfur atom)and Lb (bond or divalent group) in formula (IV) are similar to those in formula above.

The ring A indicates a ring fused with the triazole ring. The component of the ring A does not include the fused triazole ring itself, but includes a portion of the double bond between carbon atoms shared with the triazole. Thus, the ring A can be a ring having a double bond between carbon atoms.

The ring A is a monocyclic ring or a fused ring of a monocyclic ring with another ring. The ring A may have a substituent. Preferably, the monocyclic ring is a homocyclic ring, or a heterocyclic ring comprising one or more selected from the group consisting of an oxygen atom, a sulfur atom, a nitrogen atom, a phosphorus atom, a boron atom, and a silicon atom. More preferably, the monocyclic ring is a homocyclic ring, or a heterocyclic ring comprising one or more selected from the group consisting of an oxygen atom, a sulfur atom, and a nitrogen atom. For the monocyclic ring, a 5-to 12-membered monocyclic ring is preferred, a 6-to 10-membered monocyclic ring is more preferred, and a 7-to 9-membered monocyclic ring is even more preferred. As a monocyclic ring, a non-aromatic monocyclic ring is preferred

When the ring A is a fused ring, other rings that are fused with the monocyclic ring include, for example, cycloalkane, arene, and heterocyclic ring.

For the cycloalkane fused with the monocyclic ring, a cycloalkane having 3 to 24 carbon atoms is preferred, a cycloalkane having 6 to 18 carbon atoms is more preferred, a cycloalkane having 3 to 14 carbon atoms is more preferred, and a cycloalkane having 3 to 10 carbon atoms is even more preferred. For the number of carbon atoms described above, the carbon atoms in a substituent is not included. Examples of the cycloalkanes include cyclopropane, cyclobutane, cyclopentane, cyclohexane, cycloheptane, and cyclooctane.

For the arene fused with the monocyclic ring, an arene having 6 to 24 carbon atoms is preferred, an arene having 6 to 18 carbon atoms is more preferred, an arene having 6 to 14 carbon atoms is more preferred, and an arene having 6 to 10 carbon atoms is even more preferred. For the number of carbon atomsdescribed above, the carbon atoms in a substituent is not included. Examples of the arenes include benzene, naphthalene, and anthracene

The heterocyclic ring fused with the monocyclic ring is an aromatic heterocyclic ring, or a non-aromatic heterocyclic ring. It is preferred that the heteroatom constituting the heterocyclic ring include one or more selected from the group consisting of oxygen atoms, sulfur atoms, nitrogen atoms, phosphorus atoms, boron atoms, and silicon atoms, and it is more preferred that the heteroatom includes one or more selected from the group consisting of oxygen atoms, sulfur atoms, and nitrogen atoms.

For the aromatic heterocyclic ring fused with the monocyclic ring, an aromatic heterocyclic ring having 3 to 21 carbon atoms is preferred, an aromatic heterocyclic ring having 3 to 15 carbon atoms is more preferred, an aromatic heterocyclic ring having 3 to 9 carbon atoms is more preferred, and an aromatic heterocyclic ring having 3 to 6 carbon atoms is even more preferred. For the number of carbon atoms, carbon atoms in a substituent is not included. More specifically, examples of the aromatic heterocyclic ring include pyrene, pyrrole, furan, thiophene, pyridine, pyridazine, pyrimidine, pyrazine, triazine, pyrroline, piperidine, triazole, purine, anthraquinone, carbazole, fluorene, quinoline, and isoquinoline.

For the non-aromatic heterocyclic ring fused with the monocyclic ring, a non-aromatic heterocyclic ring having 3 to 21 carbon atoms is preferred, a non-aromatic heterocyclic ring having 3 to 15 carbon atoms is more preferred, a non-aromatic heterocyclic ring having 3 to 9 carbon atoms is more preferred, and a non-aromatic heterocyclic ring having 3 to 6 carbon atoms is even more preferred. For the number of carbon atoms, carbon atoms in a substituent is not included. More specifically, examples of the non-aromatic heterocyclic ring include oxirane, aziridine, azetidine, oxetane, thietane, pyrrolidine, dihydrofuran, tetrahydrofuran, dioxolane, tetrahydrothiophene, imidazolidine, oxazolidine, piperidine, dihydropyran, tetrahydropyran, tetrahydrothiopyran, morpholine, thiomorpholine, piperazine, dihydrooxazine, tetrahydrooxazine, dihydropyrimidine, and tetrahydropyrimidine.

A substituent which may be possessed by the ring A are similar to those of (i) to (vii) described above, and the preferences are as well. The number of substituents is, for example, 1 to 5, preferably 1 to 3, more preferably 1 or 2.

Preferably, the ring A may be a 7 to 9 membered monocyclic ring or a fused ring of 7 to 9 membered monocyclic ring and another ring (see, e.g., Org. Biomol. Chem 2013, 11, 6439, Angew. Chem. Int. Ed. 2015, 54, 1190). For example, rings of formulae (I')-(vii") described in WO2017/191817 may be used as such ring A.

In formula (IV), the divalent group indicated by L is a linear, branched, or cyclic group, or a group consisting of a combination thereof, which links the functional substance (Z) and the ring A. The main chain in the divalent group indicated by L is, for example, a divalent linear hydrocarbon group, a divalent cyclic hydrocarbon group, a divalent heterocyclic group, -C(=O)-, -C(=S)-, -NR- (wherein R represents a hydrogen atom or a substituent), -O-, or -S-, or a group consisting of a combination thereof of two or more (e.g., 2 to 15, preferably 2 to 10, more preferably 2 to 8, even more preferably 2 to 6, particularly preferably 2, 3, 4 or 5). The divalent group indicated by L may have a substituent. Such substituents are similar to the substituents of (i) to (vii) described above, and the preference is as well. The number of substituents is, for example, 1 to 5, preferably 1 to 3, more preferably 1 or 2. L can be bonded to any ring-constituent atom different from the carbon atom shared with the triazole in the ring A.

The functional substance is not limited to a particular substance as long as it is a substance imparting any function to the antibody; examples thereof include drugs, labelling substances, and stabilizers; preferred are drugs and labelling substances. The functional substance may be a single functional substance or a substance in which two or more functional substances are linked with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., 35^{S}), radioisotopes of yttrium (e.g., ⁹⁰Y), radioisotopes of technetium (e.g., ^{99m}Tc), radioisotopes of indium (e.g., ¹¹¹In) , radioisotopes of an iodide atom (e.g., ¹²³I, ¹²⁵I, ¹²⁹I, and ¹³¹I), radioisotopes of samarium (e.g., ¹⁵³Sm) , radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubricin.

The labelling substance is a substance that makes detection of a target (e.g., a tissue, a cell, or a substance) possible. Examples of the labelling substance include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl) Duthenium, and luminol), and radioisotopes (e.g., those described above) or substances comprising them.

The stabilizer is a substance that makes stabilization of an antibody possible. Examples of the stabilizer include diols, glycerin, nonionic surfactants, anionic surfactants, natural surfactants, saccharides, and polyols.

The functional substance may also be a peptide, a protein, a nucleic acid, a low molecular weight organic compound, a sugar chain, a lipid, a high molecular polymer, a metal (e.g., gold), or a chelator. Examples of the peptide include a cell membrane permeable peptide, a blood-brain barrier permeable peptide, and a peptide medicament. Examples of the protein include enzymes, cytokines, fragment antibodies, lectins, interferons, serum albumin, and antibodies. Examples of the nucleic acid include DNA, RNA, and artificial nucleic acid. Examples of the nucleic acid also include RNA interference inducible nucleic acids (e.g., siRNA), aptamers, and antisense. Examples of the low molecular weight organic compound include proteolysis targeting chimeras, dyes, and photodegradable compounds.

When the functional sutstance does not have a desired functional group (e.g., ring A or a functional group that is likely to react with L), the functional sutstance may be derivatized to have such a functional group. The derivatization is common technical knowledge in the art (e.g., WO2004/010957, US2006/0074008, US2005/02386649). For example, the derivatization may be carried out using any cross-linking agent. Alternatively, the derivatization may be carried out using a specific linker having a desired functional group. For example, such a linker may be capable of separating a functional substance and an antibody in a suitable environment (e.g., intracellular or extracellular) by cleavage of the linker. Such linkers include, for example, peptidyl linkers that are degraded by specific proteases [e.g., intracellular proteases (e.g., proteases present in lysosomes, or endosomes), extracellular proteases (e.g., secretory proteases)] (e.g., USP6,214,345; Dubowchik et al., Pharma. Therapeutics 83: 67-123 (1999)), a linker that can be cleaved at a locally acidic site present in the living body (e.g., USP5,622,929, 5,122,368; 5,824,805). The linker may be self-immolative (e.g., WO02/083180, WO04/043493, WO05/1192919). In the present invention, the derivatized functional substance is also simply referred to as "functional substance".

In the formula (IV), the average ratio (r) of the amide bond per two heavy chains is the average ratio of the bonds between the immunoglobulin unit and the azide group-containing group (number of functional substance-containing groups/immunoglobulin unit). Such an average ratio is 1.0 to 3.0 and preferably 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio is preferably 1.6 to 2.4, more preferably 1.7 to 2.3, even more preferably 1.8 to 2.2, and particularly preferably 1.9 to 2.1. A lysine residue having a side chain comprising an amino group forming an amide bond with a carbonyl group adjacent to Ig may be modified to indicate an average ratio of such values at a desired one position in the two heavy chains (e.g., one position selected from the group consisting of the positions 246/248, 288/290, and 317 of the human IgG heavy chain according to EU numbering). In addition, where the two heavy chains are each modified at two or more positions, the lysine residue having the side chain containing the amino group forming the amide bond with the carbonyl group adjacent to Ig may be modified to indicate the average ratio of such values at desired two or more positions in the two heavy chains (e.g., at least one position selected from the group consisting of the positions 246/248, 288/290, and 317, preferably the positions 246/248 and 288/290 in the human IgG heavy chain according to EU numbering).

The conjugate or salt thereof of the present invention can be obtained by reacting an azide group-introduced antibody derivative or a salt thereof of the present invention with a functional substance. Such reaction can be performed under a ondition that may not cause denaturation or degradation (e.g., cleavage of amide bonds) (mild condition those described above). For the functional substance, that derivatized to have a cycloalkyne group capable of reacting with azide groups under a mild condition can be used. Such a functional substance can be represented by the following formula (V): 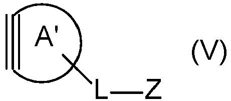 wherein
the ring A' indicates a ring having a triple bond between carbon atoms,
Z indicates a functional substance,
L indicates a bond or a divalent group.

The ring A' indicates a ring having a triple bond between carbon atoms. The ring A' is a monocyclic ring or a fused ring of a monocyclic ring with another ring. The ring A' may have a substituent. Preferably, the monocyclic ring is a homocyclic ring, or a heterocyclic ring comprising one or more selected from the group consisting of an oxygen atom, a sulfur atom, a nitrogen atom, a phosphorus atom, a boron atom, and a silicon atom. More preferably, the monocyclic ring is a homocyclic ring, or a heterocyclic ring comprising one or more selected from the group consisting of an oxygen atom, a sulfur atom, and a nitrogen atom. For the monocyclic ring, a 7- to 10-membered monocyclic ring is preferred and a 7- to 9-membered monocyclic ring is more preferred. For the monocyclic ring, a non-aromatic monocyclic ring is preferred.

Where the ring A' is a fused ring, other ring that is fused with the monocyclic ring include, for example, a cycloalkane, an arene, and a heterocyclic ring. The definitions, examples and preferred examples of the cycloalkane, arene, heterocyclic ring are the same as those of the other rings in the fused ring for ring A'.

The substituents which may be possessed by the ring A' are similar to those of (i) to (vii) described above, and the preferred ranges are as well. The number of substituents is, for example, 1 to 5, preferably 1 to 3, more preferably 1 or 2.

Preferably, the ring A' is a 7- to 9-membered monocyclic ring or a fused ring of 7- to 9-membered monocyclic and other ring. The reaction of such a ring A' with an azide group is also known as the Strin-promoted azide-alkyne cyclization (SPAAC) reaction (e.g., Org. Biomol. Chem. 2013, 11, 6439; Angew. Chem Int. Ed. 2015, 54, 1190; J. Am. Chem. Soc. 2004, 126, 15046; J. Am. Chem. Soc. 2008, 130, 11486; Chem. Commun. 2010, 46, 97. For example, the rings of formulae (I") to (vii") described in WO2017/191817 may be used as such ring A.

In the reaction, the molar ratio of the functional substance to the azide group-introduced antibody derivative or salt thereof (functional substance/azide group-introduced antibody derivative or salt thereof) is not particularly limited because it varies depending on factors such as the type of the azide group-introduced antibody derivative or salt thereof and functional substance, and the reaction time, and is, for example, 2 or more, preferably 3 or more, and more preferably 5 or more. A sufficient amount (eg, an excess) of a functional substance can be used for the azide group-introduced antibody derivative or a salt thereof in order to sufficiently react the functional substance with the thiol group of the azide group-introduced antibody derivative in a short reaction time.

The determination of the formation of the conjugate or salt thereof, which depends on its specific raw materials and the molecular weight of a product, can be performed by electrophoresis, chromatography (e.g., gel permutation chromatography, ion-exchange chromatography, reversed phase column chromatography, and high-performance liquid chromatography (HPLC)), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of the introduced functional substance can be performed by electrophoresis, chromatography, or mass spectrometry, for example, and preferably mass spectrometry. The conjugate or salt thereof can be purified as appropriate by any method such as chromatography (e.g., chromatography described above and affinity chromatography).

### 6. Uses

The compound or salt thereof of the present invention can highly control the average ratio of bonding of an immunoglobulin unit and an azide group-containing modifying group (azide group-containing modifying group/immunoglobulin unit) within a desired range (1.0 to 3.0). The compound or salt thereof of the invention can also regioselectively modify lysine residues in heavy chains in an immunoglobulin unit. Accordingly, the present invention provides a reagent for derivatizing an antibody, comprising the compound or a salt thereof of the present invention.

The reagent of the present invention may be provided in a form of a composition further comprising other components. Examples of such other compounds include solutions and stabilizers (e.g., antioxidants and preservatives). Among solutions, aqueous solutions are preferred. Examples of aqueous solutions include water (e.g., distilled water, sterilized distilled water, purified water, and a physiological saline solution) and buffers (e.g., an aqueous phosphoric acid solution, a Tris-hydrochloric acid buffer, a carbonic acid-bicarbonic acid buffer, an aqueous boric acid solution, a glycine-sodium hydroxide buffer, and a citric acid buffer); buffers are preferred. The pH of solutions is e.g., 5.0 to 9.0 and preferably 5.5 to 8.5. The reagent of the present invention can be provided in a liquid form or a powder form (e.g., freeze-dried powder).

The intermediate antibody or salt thereof of the present invention and the azide group-introduced antibody derivative or salt thereof of the present invention are useful as intermediates for preparing a conjugate of an antibody and a functional substance or a salt thereof, for example.

The conjugate or salt thereof of the present invention is useful as pharmaceuticals or reagents (e.g., diagnostic reagents and reagents for research), for example. In particular, the conjugate or salt thereof of the present invention regioselectively modified with a functional substance and having an average bonding ratio of an antibody and a functional substance which is in a desired rage (1.0 to 3.0) is useful as pharmaceuticals. It is reported that when the number of bonds and the bond positions of a drug of an antibody drug conjugate (ADC) are changed, pharmacokinetics, a releasing rate of the drug, and effects change. Given these circumstances, next-generation ADCs are required to control the number and positions of a drug to be conjugated. It is believed that when the number and positions are constant, the problems of expected efficacy, variations in conjugate medicines, and lot difference, or what is called regulation, will be solved. Therefore, the conjugate or salt thereof of the present invention can solve such a problem of regulation.

The conjugate or salt thereof of the present invention having a functional substance may be provided in the form of a pharmaceutical composition. The pharmaceutical composition may comprise a pharmaceutically allowable carrier in addition to the conjugate or salt thereof of the present invention. Examples of the pharmaceutically allowable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The conjugate or salt thereof of the present invention may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set as appropriate.

### EXAMPLES

The present invention is explained by the following Examples in more detail, but the present invention is not limited to the following Examples.

### Example 1: Synthesis of IgG1 Modification Reagents

### (1-1) Synthesis of Modification Reagent (1)

### (1-1-1) Synthesis of Linker Intermediate (2)

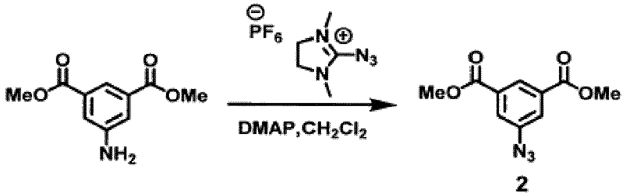

2-Azido-1,3-dimethylimidazolinium hexafluorophosphate (4.0 g, 14.0 mmol) was dissolved in CH₂Cl₂ (23.4 mL), and dimethyl-5-aminoisophthalate (977 mg, 4.67 mmol) and DMAP (1.7 g, 14.0 mmol) were added and the mixture was stirred at 50°C for 1 hour. After confirming the reaction with LC/MS and TLC (hexane/ethyl acetate=1/1), the reaction solution was concentrated. The reactant was eluted with a mixed solution of hexane and ethyl acetate and the fractions were confirmed by TLC (hexane/ethyl acetate=2/1). The fraction containing the product was collected and concentrated in vacuo to remove the organic solvents, followed by vacuum-drying to obtain the linker intermediate (2) (1.08 g, 4.59 mmol).

### (1-1-2) Synthesis of Linker Intermediate (3)

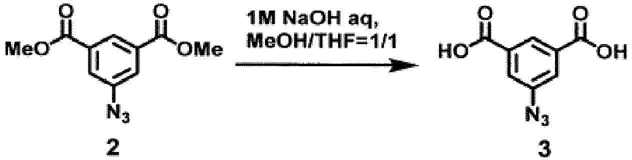

The linker intermediate 2 (1.08 g, 4.59 mmol) synthesized in (1-1-1) was dissolved in methanol (23 mL), and aqueous 1 M NaOH (13.8 mL, 13.8 mmol) and THF (23 mL) were added thereto, and the mixture was stirred at room temperature for 2 hours. After confirming the reaction with LC/MS, the reaction solution was acidified with 2 M HCl aqueous solution. Then, the reactant was extracted with ethyl acetate and 2 M HCl aqueous solution, the organic layer was concentrated, and dried in vacuo to obtain linker intermediate 3 (953.2 mg, 4.60 mmol).

### (1-1-3) Synthesis of Linker (4)

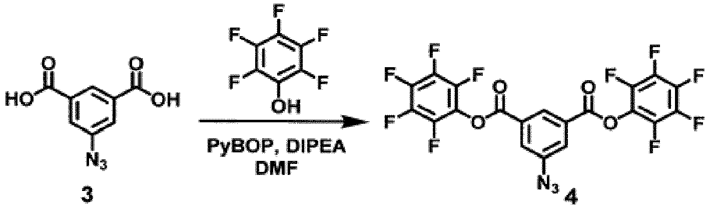

The linker intermediates 3 (200 mg, 0.97 mmol) synthesized in (1-1-2) were dissolved in DMF (4.85 mL), and pentafluorophenol (893 mg, 4.85 mmol), PyBOP (1.0 g, 1.94 mmol), DIPEA (990 µL, 5.82 mmol), and DMAP (11.9 mg, 0.097 mmol) were added and the mixture was stirred for 2 hours at room temperature. After confirming the reaction with LC/MS and TLC (hexane/ethyl acetate=5/1), the reactant was extracted with ethyl acetate and 2 M HCl aqueous solution, and the organic layer was concentrated. The reactant was eluted with a mixed solution of hexane and ethyl acetate and the fractions were confirmed by TLC (hexane/ethyl acetate=5/1). Fractions containing the product were collected and concentrated in vacuo to remove the organic solvents, and then dried in vacuo to obtain the linker (4) (173.4 mg, 0.32 mmol).

### (1-1-4) Synthesis of Peptide Intermediate (5)

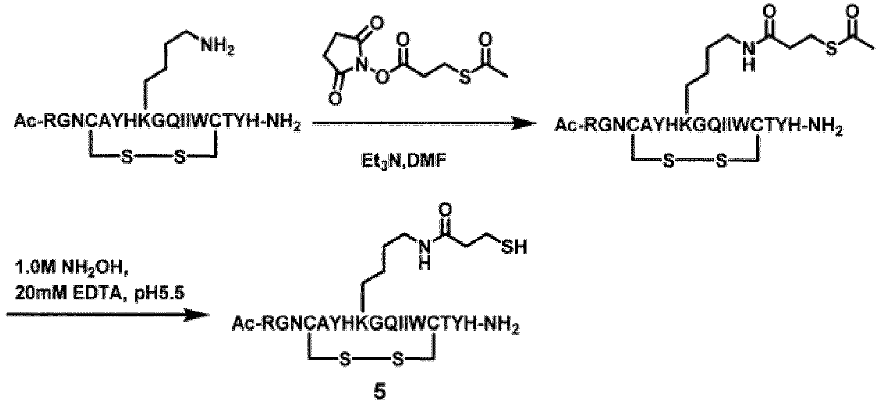

Each of the above amino acid sequences is the amino acid sequence of SEQ ID NO: 2.

The peptide consisting of the amino acid sequence of SEQ ID NO: X1 was synthesized in solid phase according to the methods described in WO 2018/199337 (the same applies hereinafter for synthesizing the peptide). Ac-RGNCAYHKGQIIWCTYH-NH₂ (SEQ ID NO: 2, 50.0 mg, 19.6 µmol, with two cysteines at positions 4 and 14 each forming an intramolecular disulfide bond) and N-Succinimidyl-3(Acetylthio)propionate (48.0 mg, 196 µmol) were dissolved in DMF (1.00 mL), and triethylamine (8.2 µL, 58.8 µmol) was added, and the mixture was stirred at room temperature for 1.5 hours. After confirming the reaction with LC-MS, 1.0 M NH₂OH, 20 mM EDTA (2 mL) was added to the reaction solution, and the mixture was stirred for another 2 hours. After the reaction was confirmed by LC-MS, the reaction was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. The fraction containing the product was collected, and the acetonitrile was removed by concentration in vacuo, followed by freeze-drying to obtain the peptide intermediate (5) (29.1 mg, 13.4 µmol).
MS(ESI) m/z: z=3 727.10 [M+3H]³⁺

### (1-1-5) Synthesis of Modification Reagent (1)

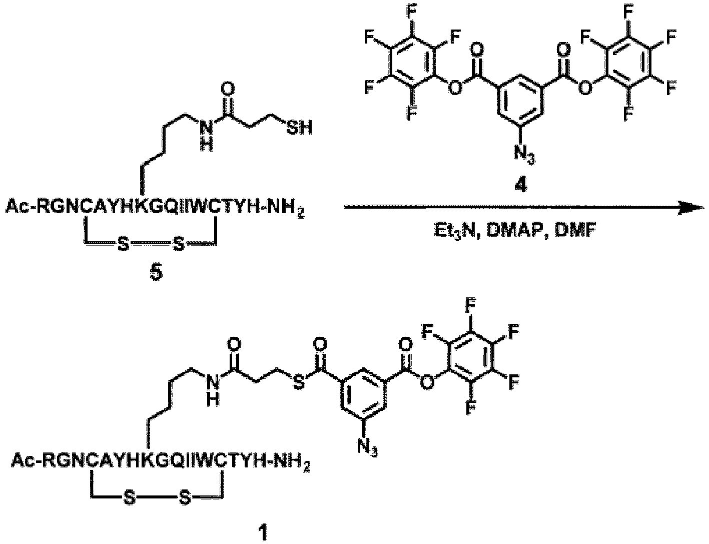

Each of the above amino acid sequences is the amino acid sequence of SEQ ID NO: 2.

The peptide intermediate (5) synthesized in (1-1-4) (29.1 mg, 13.4 µmol) and the linker (4) synthesized in (1-1-3) (72.3 mg, 134 µmol) were dissolved in DMF (1.00 mL), and triethylamine (5.6 µL, 40.2 µmol) and DMAP were added, and the mixture was stirred at room temperature for 1 hour. After the reaction was confirmed by LC-MS, the reaction was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. The fraction containing the product was collected, and the acetonitrile was removed by concentration in vacuo, followed by freeze-drying to obtain the modification reagent (1) (9.0 mg, 3.55 µmol).
MS(ESI) m/z: z=3 845.55 [M+3H]³⁺

### (1-2) Synthesis of Modification Reagent (6)

### (1-2-1) Synthesis of Linker Intermediate (7)

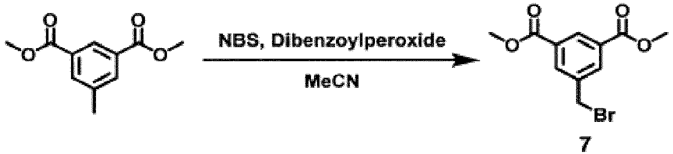

Dimethyl-5-methylisophthalate (830 mg, 4.0 mmol) was dissolved in acetonitrile (100 mL), and NBS (783 mg, 4.4 mmol) and benzoyl peroxide (40 mg, 0.17 mmol) were added and the mixture was stirred at 90°C for 20 hours. After confirming the reaction with LC/MS and TLC (hexane/ethyl acetate=5/1), the reaction solution was concentrated. The mixture was dissolved in hexane/ethyl acetate=1/1 solvent mixture and filtered. The crystals were collected and dried in vacuo to give the linker intermediate (7) (630 mg, 2.19 mmol).

### (1-2-2) Synthesis of Linker Intermediate (8)

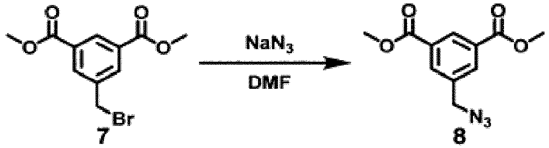

Sodium azide (204 mg, 3.13 mmol) was dissolved in DMF (2.0 mL) and the linker intermediate (7) (300 mg, 1.04 mmol) synthesized in (1-2-1) was added and the mixture was stirred at 60°C for 3 hours. After confirming the reaction with LC/MS and TLC (hexane/ethyl acetate=5/1), the reactant was extracted with ethyl acetate and water, and the organic layer was concentrated. The reactant was eluted with a mixed solution of hexane and ethyl acetate and the fractions were confirmed by TLC (hexane/ethyl acetate=5/1). Fractions containing the product were collected and concentrated in vacuo to remove the organic solvents, followed by vacuum-drying to obtain linker intermediate (8) (268 mg, 1.08 mmol).

### (1-2-3) Synthesis of Linker Intermediate (9)

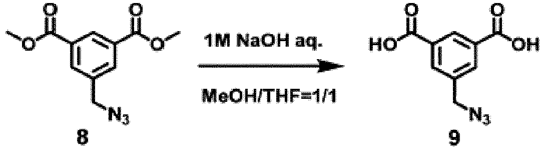

The compound (268 mg, 1.08 mmol) synthesized in (1-2-2) was dissolved in 5.4 mL, and THF (5 mL) and an aqueous 1 M NaOH solution were added thereto, and the mixture was stirred at room temperature for 2 hours. After the reaction was confirmed by MS, the reaction solution was acidified with 2 M HCl aqueous solution, and the reactant was extracted with ethylacetate and 2 M HCl aqueous solution, and the organic layer was concentrated. Vacuum-drying gave the linker intermediate (9) (227 mg, 1.00 mmol).

### (1-2-4) Synthesis of Linker Intermediate (10)

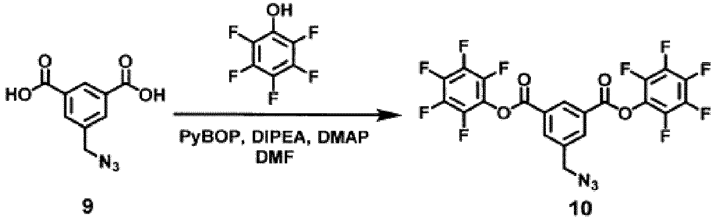

The linker intermediate (9) (227 mg, 1.00 mmol) synthesized in (1-2-3) was dissolved in DMF (5.0 mL), and pentafluorophenol (920 mg, 5.00 mmol), PyBOP (1.3 g, 2.5 mmol), DIPEA (1.02 mL, 6.0 mmol) and DMAP (12.2 mg, 0.1 mmol) were added and the mixture was stirred at room temperature for 20 hours. After confirming the reaction with LC/MS and TLC (hexane/ethyl acetate=5/1), the reactant was extracted with ethyl acetate and an aqueous 1M HCl solution, and the organic layer was concentrated. The reactant was eluted with a mixed solution of hexane and ethyl acetate and the fractions were confirmed by TLC (hexane/ethyl acetate=5/1). Fractions containing the product were collected and concentrated in vacuo to remove the organic solvents, followed by vacuum-drying to obtain linker intermediate (10) (395.7 mg, 0.72 mmol).

### (1-2-5) Synthesis of Modification Reagent (6)

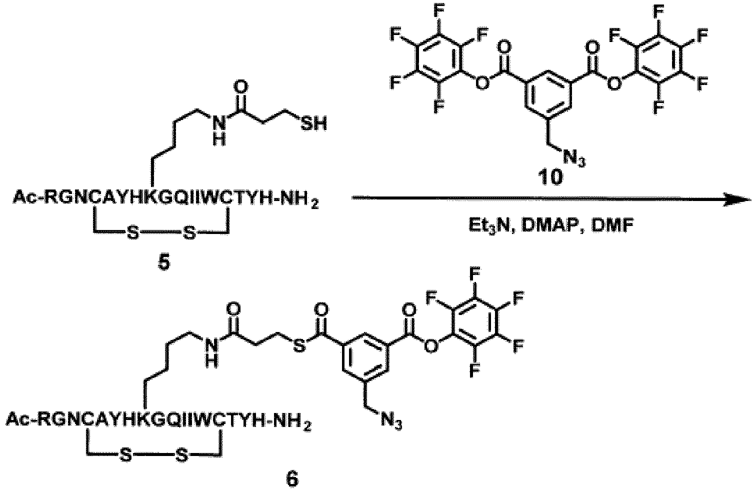

Each of the above amino acid sequences is the amino acid sequence of SEQ ID NO: 2.

The peptide intermediate (5) synthesized in (1-1-4) (30.3 mg, 13.9 µmol) and the linker intermediate (10) synthesized in (1-2-4) (76.9 mg, 139 µmol) were dissolved in DMF (1.00 mL), and triethylamine (5.8 µL, 41.7 µmol) and DMAP were added, and the mixture was stirred at room temperature for 1 hour. After the reaction was confirmed by LC-MS, the reaction was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. Fractions containing the product were collected and concentrated in vacuo to remove acetonitrile, followed by freeze-drying to afford the modification reagent (6) (14.4 mg, 5.65 µmol).
MS(ESI) m/z: z=3 850.20 [M+3H]³⁺

### (1-3) Synthesis of Modification Reagent (11)

### (1-3-1) Synthesis of Linker Intermediate (12)

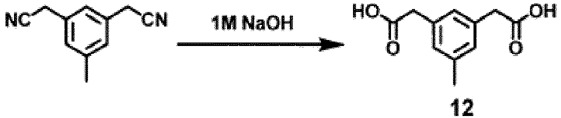

5-Methyl-1,3-benzenediacetonitrile (5.0 g, 29.4 mmol) was dissolved in aqueous 1 M NaOH (100 mL) and DMF was added and the mixture was stirred at 110°C for 20 hours. After confirming the reaction with LC/MS, the reaction solution was concentrated. The concentrate was acidified with aqueous 2 M HCl and the reactant was extracted with aqueous ethylacetate and 2 M HCl and the organic layer was concentrated. Vacuum-drying gave the linker intermediate (12) (6.0 g, 28.8 mmol).

### (1-3-2) Synthesis of Linker Intermediate (13)

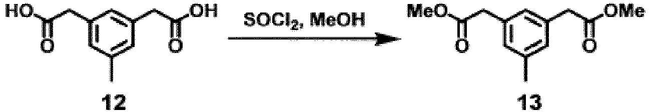

The linker intermediate (12) (6.0 g, 28.8 mmol) synthesized in (1-3-1) was dissolved in methanol, thionyl chloride (21.4 mL, 288 mmol) was added at 0°C, and the mixture was stirred at room temperature for 3 hours. After the reaction was confirmed by LC/MS, the reactant was extracted with ethyl acetate and sodium bicarbonate, and the organic layer was concentrated to obtain the linker intermediate (13).

### (1-3-3) Synthesis of Linker Intermediate (14)

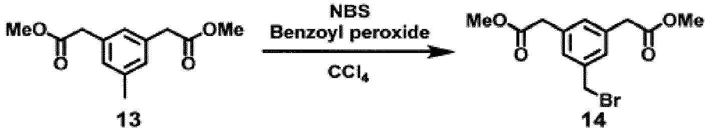

The compound synthesized in (1-3-2) was dissolved in carbon tetrachloride (28.8 mL), and N-bromosuccinimide (5.6 g, 31.6 mmol) and benzoylperoxide (157 mg, 0.49 mmol) were added, and the mixture was stirred at 85°C for 2 hours. N-Bromosuccinimide (5.6 g, 31.6 mmol) was then added and the mixture was stirred at 85°C for 30 minutes. After confirming the reaction with LC/MS and TLC (hexane/ethyl acetate=3/1), the crystals were removed and concentrated in vacuo to remove the organic solvents. The reactant was eluted with a mixed solution of hexane and ethyl acetate and the fractions were confirmed by TLC (hexane/ethyl acetate=3/1). Fractions containing the product were collected and concentrated in vacuo to remove the organic solvents, followed by vacuum-drying to obtain linker intermediate (14) (870 mg, 2.76 mmol).

### (1-3-4) Synthesis of Linker Intermediate (15)

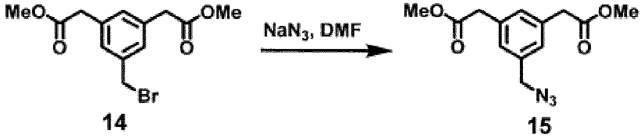

The linker intermediate (14) (870 mg, 2.76 mmol) synthesized in (1-3-3) was dissolved in DMF (5.0 mL) and sodium azide (540 mg, 8.28 mmol) was added and the mixture was stirred at room temperature for 1 hour. After confirming the reaction with LC/MS and TLC (hexane/ethyl acetate=3/1), the reactant was extracted with ethyl acetate and water, the organic layer was concentrated, and vaccum-drying gave a linker intermediate (15).

### (1-3-5) Synthesis of Linker Intermediate (16)

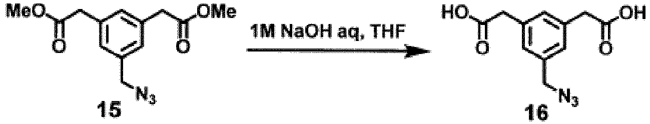

The linker intermediate (16) synthesized in (1-3-4) was dissolved in THF (3.0 mL) and 1 M NaOH aqueous solution (7.0 mL) was added at 0°C and the mixture was stirred at room temperature for 1 hour. After the reaction was confirmed by LC/MS, the reaction solution was acidified with 2 M HCl aqueous solution, and the reactant was extracted with ethylacetate and 2 M HCl aqueous solution, and the organic layer was concentrated. Vacuum-drying gave the linker intermediate (16) (637.8 mg, 2.56 mmol).

### (1-3-6) Synthesis of Linker Intermediate (17)

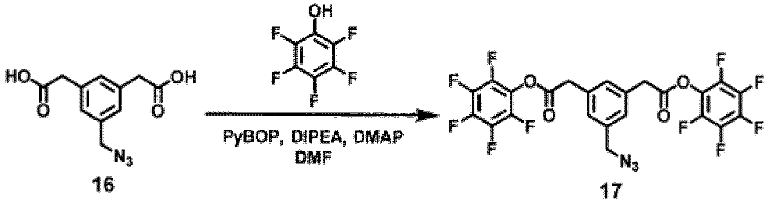

The linker intermediate (16) synthesized with (1-3-5) (237 mg, 0.95 mmol) were dissolved in DMF (4.75 mL), and pentafluorophenol (874 mg, 4.75 mmol), PyBOP (1.24 g, 2.38 mmol), DIPEA (969 µL, 5.70 mmol) and DAMP (11.6 mg, 0.095 mmol) were added and the mixture was stirred for 20 hours at room temperature. After confirming the reaction with LC/MS and TLC (hexane/ethyl acetate=3/1), the reactant was extracted with ethyl acetate and water, and the organic layer was concentrated. The reactant was eluted with a mixed solution of hexane and ethyl acetate and the fractions were confirmed by TLC (hexane/ethyl acetate=3/1). Fractions containing the product were collected and concentrated in vacuo to remove the organic solvents, followed by vacuum-drying to obtain linker intermediate (17) (300 mg, 0.52 mmol).

### (1-3-7) Synthesis of Modification Reagent (11)

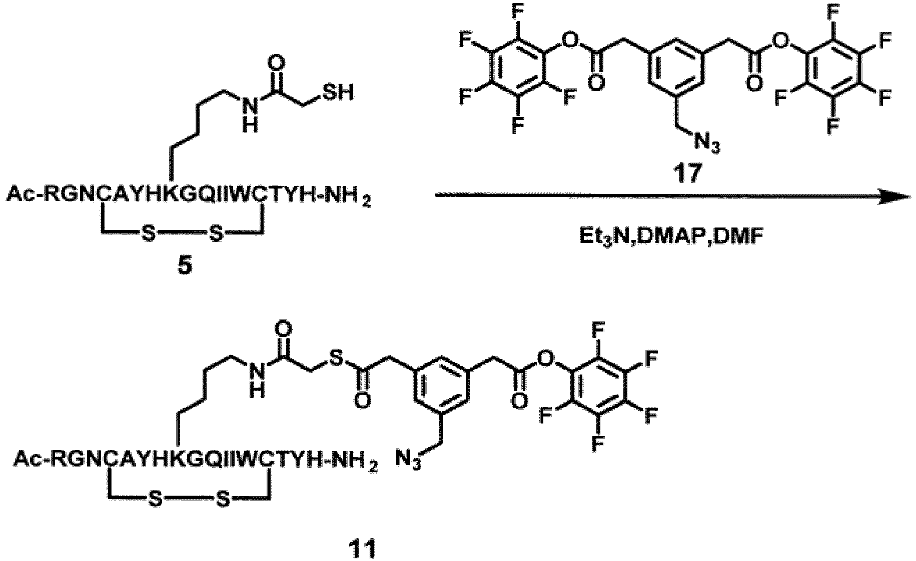

Each of the above amino acid sequences is the amino acid sequence of SEQ ID NO: 2.

The peptide intermediate (5) synthesized in (1-1-4) (15.0 mg, 6.93 µmol, with two cysteines at positions 4 and 14 each forming an intramolecular disulfide bond) and the linker intermediate (17) (40.2 mg, 69.3 µmol) synthesized in (1-3-6) were dissolved in DMF (1.00 mL), and triethylamine (2.89 µL, 20.79 µmol) and DMAP were added, and the mixture was stirred at room temperature for 1 hour. After the reaction was confirmed by LC-MS, the reaction was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. Fractions containing the product were collected and concentrated in vacuo to remove acetonitrile, followed by freeze-drying to afford the modification reagent (11) (1.44 mg, 0.56 umol).
MS(ESI) m/z: z=3 854.30 [M+3H]³⁺

### (1-4) Synthesis of Modification Reagent (18)

### (1-4-1) Synthesis of Linker Intermediate (19)

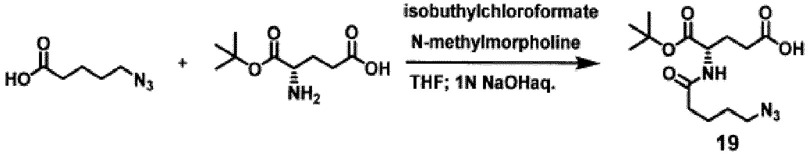

5-Azidopentanoic acid (800 mg, 5.59 mmol) was dissolved in THF (14 mL), and isobutyl chloroformate (808 µL, 6.15 mmol) and N-methylmorpholine (873 µL, 8.39 mmol) were added, and the mixture was stirred at 0°C for 30 minutes, and then hydrazine hydrate (1.36 g, 6.71 mmol) dissolved in an aqueous 1 M NaOH solution (4 mL) was added, and the mixture was stirred at room temperature for 3 hours. After concentrating under reduced pressure, an aqueous 1 M NaOH solution was added to adjust pH in the system to pH 10, and after washing with ethyl acetate, 1 M HCl aqueous solution was added to the aqueous layer to adjust pH in the system to 3.0, the obtained solution was wached by adding ethyl acetate, and sodium sulfate was added to the obtained ethyl acetate solution. Sodium sulfate was removed by filtration and purified by concentration column chromatography (dichloromethane:methanol=10:1) under reduced pressure. The fraction containing the product was collected and concentrated in vacuo to afford the linker intermediate (19).
¹H NMR (400 MHz, Chloroform-d) δ6.29 (d, J=7.7 Hz, 1H), 4.56 (td, J=8.0, 4.9 Hz, 1H), 3.32 (t, J=6.6 Hz, 2H), 2.53-2.38 (m, 3H), 2.36-2.16 (m, 3H), 2.12 (s,2H), 1.96 (dq, J=14.7, 7.6 Hz, 1H), 1.84-1.59 (m, 4H), 1.50 (s, 9H).
MS(ESI) m/z: 329 [M+H]⁺

### (1-4-2) Synthesis of Linker Intermediate (20)

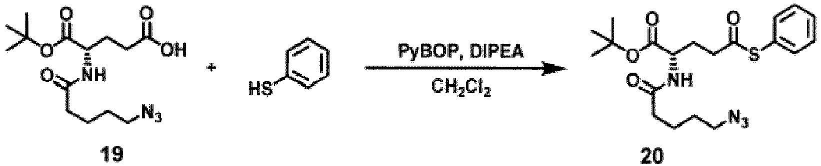

The linker intermediate (19) (2.41 g, 5.59 mmol) was dissolved in dichloromethane (28 mL), and thiophenol (627 µL, 6.15 mmol), benzotriazol-1-yloxy (3.49 g, 6.71 mmol), and DIPEA (1.42 mL, 8.39 mmol) were added, and the mixture was stirred at room temperature for 2 hours. Then, the mixture was concentrated under reduced pressure and purified by column chromatography (hexane:ethyl acetate=4:1). The fraction containing the product was collected and concentrated in vacuo to afford the linker intermediate (20) (2.20 g, 5.23 mmol).
¹H NMR (400 MHz, Chloroform-d) δ7.43 (s, 5H), 6.10 (d, J=7.8 Hz, 1H), 4.55 (td, J=7.7, 4.9 Hz, 1H), 3.31 (t, J=6.7 Hz, 2H), 2.87-2.63 (m, 2H), 2.28 (dd, J=8.7, 5.9 Hz, 2H), 2.16-1.98 (m,1H), 1.83-1.58 (m,4H), 1.50 (s, 9H), 1.37-1.22 (m, 2H), 0.91 (t, J=6.7Hz, 1H).
MS(ESI) m/z: 421 [M+H]⁺

### (1-4-3) Synthesis of Linker Intermediate (21)

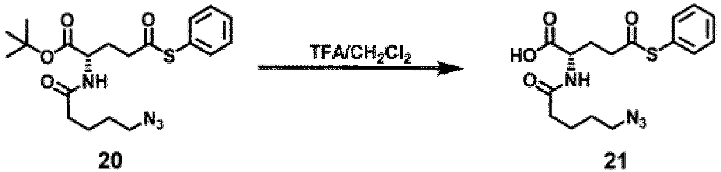

The linker intermediate (20) (2.20 g, 5.23 mmol) was dissolved in dichloromethane (10 mL), and trifluoroacetic acid (10mL) was added, and the mixture was stirred at room temperature for 1 hour, then concentrated in vacuo to remove dichloromethane, and water was added and the mixture was freeze-dried to obtain the linker intermediate (21) (1.98 g, 5.43 mmol).
¹H NMR (400 MHz, Chloroform-d) δ7.44 (s, J=6.3, 4.6, 2.4 Hz, 5H), 6.76 (s, 1H), 4.62 (td, J=7.5, 4.9 Hz, 1H), 3.31 (t, J=6.6 Hz, 2H), 2.88 (qt, J=16.8, 6.8 Hz, 2H), 2.33 (dt, J=12.4, 6.8 Hz, 3H), 2.18 (dq, J=14.4, 7.4 Hz, 1H), 1.74 (dq, J=11.8, 7.5, 6.9 Hz, 2H), 1.63 (ddd, J=17.7, 10.5, 4.8 Hz, 2H).
MS(ESI) m/z: 365 [M+H]⁺

### (1-4-4) Synthesis of Linker Intermediate (22)

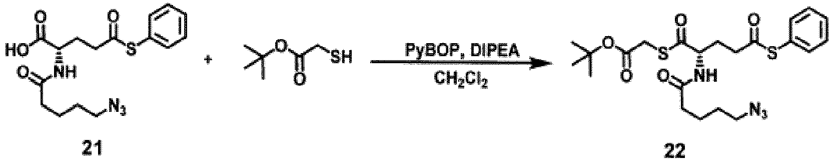

The linker intermediate (21) (100 mg, 0.274 mmol) was dissolved in dichloromethane (3 mL) (40.6 µL, 0.280 mmol), benzotriazol-1-yloxy (150 mg, 0.288 mmol) and DIPEA (70.1 µL, 0.412 mmol) were added, and the mixture was stirred at room temperature for 2 hours. an aqueous 1 M HCl solution was added to adjust pH in the system to pH 3, and the solution was diluted with dichloromethane, washed with water and brine, and then sodium sulfate was added. Sodium sulfate was removed by filtration, and the solution was concentrated under reduced pressure, and the product was purified by column chromatography (hexane : ethyl acetate=4:1). The fraction containing the product was collected and concentrated in vacuo to afford the linker intermediate (22) (84.7 mg, 0.171 mmol).
¹H NMR (400 MHz, Chloroform-d) δ7.50-7.38 (m, 5H), 6.33 (d, J=8.4Hz, 1H), 4.78 (tdd, J=7.8, 4.6, 3.0 Hz, 1H), 3.70-3.54 (m, 2H), 3.32 (dt, J=9.1, 6.7 Hz, 2H), 2.96-2.67 (m, 2H), 2.30 (pd, J=7.1, 4.5 Hz, 2H), 1.85-1.60 (m, 6H), 1.49 (d, J=2.8Hz, 9H).
MS(ESI) m/z: 495 [M+H]⁺

### (1-4-5) Synthesis of Linker Intermediate (23)

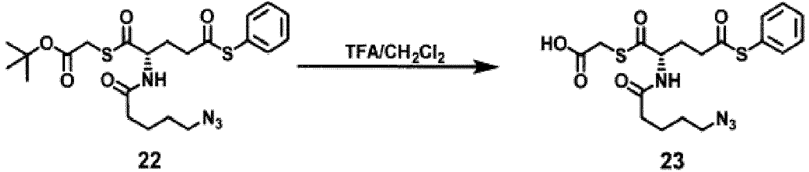

The linker intermediate (22) (84.7 mg, 0.171 mmol) was dissolved in dichloromethane (5 mL), trifluoroacetic acid (5 mL) was added, and the mixture was stirred at room temperature for 1 hour, then concentrated in vacuo to remove dichloromethane, and water was added and the mixture was freeze-dried. Then, the mixture was purified by column chromatography (dichloromethane : methanol=10:1). The fraction containing the product was collected and concentrated in vacuo to afford the linker intermediate (23) (46.8 mg, 0.107 mmol).
¹H NMR (400 MHz, methanol-d4) δ7.44 (dq, J=2.3, 1.5 Hz, 5H), 4.69-4.57 (m, 1H), 3.79-3.67 (m, 2H), 3.40-3.30 (m, 2H), 2.89-2.71 (m, 2H), 2.44-2.23 (m, 4H), 2.08-1.95 (m, 1H), 1.82-1.61 (m, 4H).
MS(ESI) m/z: 439 [M+H]⁺

### (1-4-6) Synthesis of Modification Reagent (18)

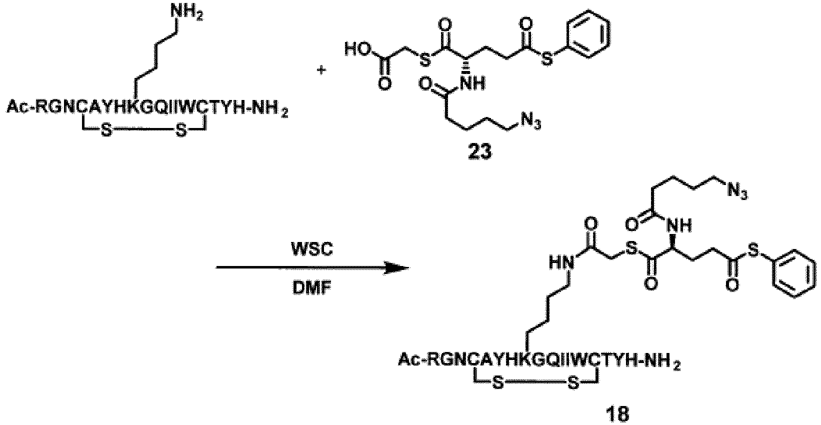

Each of the above amino acid sequences is the amino acid sequence of SEQ ID NO: 2.

Ac-RGNCAYHKGQIIWCTYH-NH₂ (SEQ ID NO: 2, 30.9 mg, 14.9 µmol, with two cysteines at positions 4 and 14 each forming an intramolecular disulfide bond) was dissolved in (468 µL), a linker intermediate (23) (46.8 mg, 0.107 mmol) and WSC·HCl (29.7 mg, 0.155 mmol) were added, and the mixture was stirred at room temperature for 5 hours, and then the reactant was eluted by reverse phase preparative chromatography. The fraction containing the product was collected, and the acetonitrile was removed by concentration in vacuo, followed by freeze-drying to obtain the modification reagent (18) (15.1 mg, 6.02 µmol).

### (1-5) Synthesis of Modification Reagent (24)

### (1-5-1) Synthesis of Linker Intermediate (25)

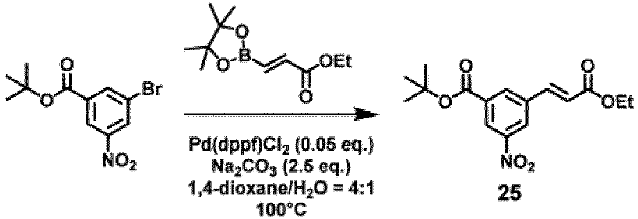

tert-Butyl 3-bromo-5-nitrobenzoate (500 mg, 1.65 mmol) was dissolved in a mixed solution of 1,4-Dioxane/H₂O=4/1 (8.25 mL), and 2-ethoxycarbonylvinylboronic acid pinacol ester (447 mg, 1.98 mmol), Pd(dppf)Cl₂ (67.8 mg, 0.083 mmol) and Na₂CO₃ (438 mg, 4.13 mmol) were added and the mixture was stirred at 100°C for 2 hours. After confirming the reaction with LC/MS and TLC (hexane/ethyl acetate=10/1), the reactant was extracted with ethyl acetate and water, and the organic layer was concentrated. The reactant was eluted with a mixed solution of hexane and ethyl acetate and the fractions were confirmed by TLC (hexane/ethyl acetate=10/1). The fraction containing the product was collected and concentrated in vacuo to remove the organic solvents, followed by vacuum-drying to obtain the linker intermediate (25) (371.7 mg, 1.16 mmol).

1H NMR (400 MHz, Chloroform-d) δ=8.69 (d, J=1.8, 1H), 8.43 (t, J=2.0, 1H), 8.35 (d, J=1.6, 1H), 7.67 (d, J=16.0, 1H), 6.55 (d, J=16.0, 1H), 4.23 (q, J=7.1, 2H), 1.57 (s, 9H), 1.36-1.22 (m, 3H).

### (1-5-2) Synthesis of Linker Intermediate (26)

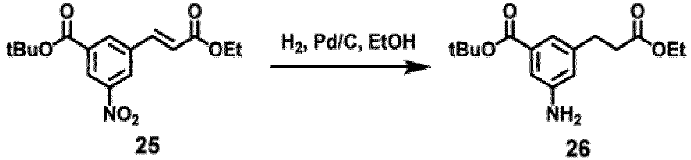

The linker intermediate (25) (371.7 mg, 1.16 mmol) was dissolved in methanol (5.8 mL), and ethyl acetate (5.0mL) and Pd/C (5% Pd, 53% H₂O) (5.0 mol%) were added and the mixture was stirred under hydrogen for 3 hours. After confirming the reaction with TLC (hexane/ethyl acetate=5/1), the reaction was filtered through a membrane filter, the filtrate was concentrated, and then dried in vacuo to obtain a linker intermediate (26) (320.5 mg, 1.09 mmol).

¹H NMR (400 MHz, Chloroform-d) δ=7.24-7.11 (m, 1H), 6.75 (t, J=1.9, 1H), 4.14 (q, J=7.1, 2H), 2.90 (t, J=7.8, 2H), 2.61 (t, J=7.8, 2H), 1.58 (s, 9H), 1.25 (td, J=7.1, 4.5, 3H).

### (1-5-3) Synthesis of Linker Intermediate (27)

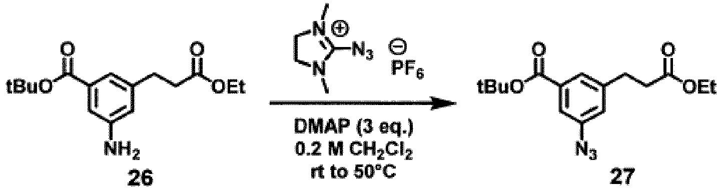

The linker intermediate (26) (320.5 g, 1.09 mmol) was dissolved in CH₂Cl₂ (5.45 mL), and 2-azido-1,3-dimethylimidazolinium hexafluorophosphate (932 mg, 3.27 mmol) and DMAP (399 mg, 3.27 mmol) were added and the mixture was stirred for 1.5 hours. After confirming the reaction with TLC (hexane/ethyl acetate=2/1), the reaction solution was concentrated. The mixture was eluted with a mixed solvent of hexane and ethyl acetate and the product wads confirmed by TLC (hexane/ethyl acetate=2/1). The fraction containing the product was collected and concentrated in vacuo to remove the organic solvents, followed by vacuum-drying to obtain the linker intermediate (27) (278 mg, 1.18 mmol).

¹H NMR (400 MHz, Chloroform-d) δ=7.60 (d, J=1.5, 1H), 7.50 (t, J=1.8, 1H), 7.02 (t, J=2.0, 1H), 4.14 (q, J=7.1, 2H), 2.98 (t, J=7.7, 2H), 2.64 (t, J=7.7, 2H), 1.60 (s, 10H), 1.25 (t, J=7.1, 4H).

### (1-5-4) Synthesis of Linker Intermediate (28)

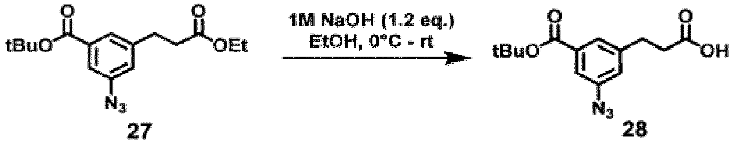

The linker intermediate (27) (278 mg, 1.18 mmol) was dissolved in THF (4.35 mL) and aqueous 1 M NaOH (1.74 mL) and ethanol (4 mL) were added at 0°C and the mixture was stirred at room temperature for 1.5 hours. The reaction was confirmed with LC/MS and TLC (hexane/ethyl acetate=2/1), and the reaction solution was acidified with an aqueous 1M HCl solution (2.61 mL) at 0°C, the reactant was extracted with ethyl acetate and aqueous 1 M HCl, the organic layer was concentrated and vaccum-drying give the linker intermediate (28) (246.6 mg, 0.85 mmol).

¹H NMR (400 MHz, Chloroform-d) δ=7.61 (d, J=1.5, 1H), 7.52 (t, J=1.8, 1H), 7.02 (t, J=1.9, 1H), 3.00 (t, J=7.7, 2H), 2.72 (t, J=7.7, 2H), 1.60 (s, 8H).

### (1-5-5) Synthesis of Linker Intermediate (29)

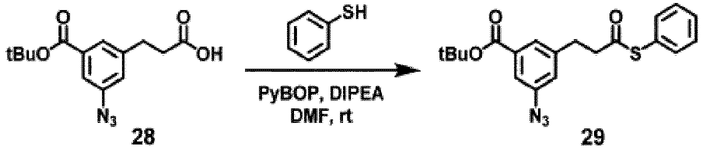

The linker intermediate (28) (246.6 mg, 0.85 mmol) was dissolved in DMF (4.25 mL) and benzenethiol (86. 7 µL, 0.85 mmol), PyBOP (442 mg, 0.85 mmol) and DIPEA (218 µL, 1.28 mmol) were added and the mixture was stirred at room temperature for 1.5 hours. After confirming the reaction with LC/MS and TLC (hexane/ethyl acetate=4/1), the reactant was extracted with ethyl acetate and water, and the organic layer was concentrated. The reactant was eluted with a mixed solution of hexane and ethyl acetate and the fractions were confirmed by TLC (hexane/ethyl acetate=4/1). Fractions containing the product were collected and concentrated in vacuo to remove the organic solvents, followed by vacuum-drying to obtain linker intermediate (29) (250.9 mg, 0.65 mmol).

¹H NMR (400 MHz, Chloroform-d) δ=7.53 (d, J=1.6, 1H), 7.44 (t, J=1.8, 1H), 7.34 (p, J=3.6, 5H), 6.93 (t, J=1.9, 1H), 3.04-2.80 (m, 4H), 1.53 (s, 10H).

### (1-5-6) Synthesis of Linker Intermediate (30)

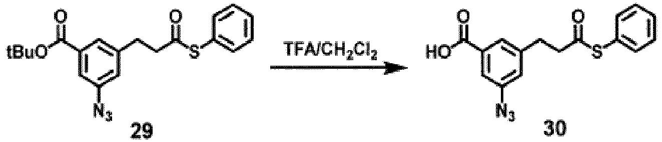

The linker intermediate (29) (250.9 mg, 0.65 mmol) was dissolved in a mixed solvent of CH2Cl2/TFA=1/1 (10 mL) and the mixture was stirred at room temperature for 1 hour. After confirming the reaction with LC/MS and TLC (hexane/ethyl acetate=4/1), the organic solvent was removed by concentration in vacuo, followed by vacuum-drying to obtain a linker intermediate (30) (215.1 mg, 0.66 mmol).

¹H NMR (400 MHz, Chloroform-d) δ=7.74 (s, 1H), 7.64 (d, J=1.8, 1H), 7.49-7.32 (m, 4H), 7.10 (d, J=2.0, 1H), 3.19-2.88 (m, 4H).

### (1-5-7) Synthesis of Linker Intermediate (31)

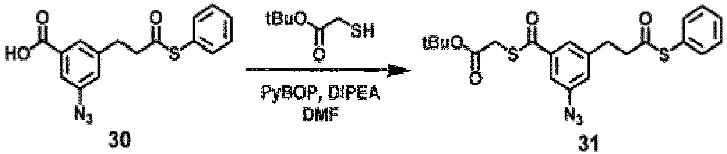

The linker intermediate (30) (215.1 mg, 0.66 mmol) was dissolved in DMF (3.3 mL), and tert-butyl-2-sulfanylacetate (97.8 mg, 0.66 mmol), PyBOP (343 mg, 0.66 mmol) and DIPEA (168 µL, 0.99 mmol) were added and the mixture was stirred at room temperature for 1 hour. After confirming the reaction with LC/MS and TLC (hexane/ethyl acetate=5/1), the reactant was extracted with ethyl acetate and water, and the organic layer was concentrated. The reactant was eluted with a mixed solution of hexane and ethyl acetate and the fractions were confirmed by TLC (hexane/ethyl acetate=5/1). Fractions containing the product were collected and concentrated in vacuo to remove the organic solvents, followed by vacuum-drying to obtain linker intermediate (31) (247.8 mg, 0.54 mmol).

¹H NMR (400 MHz, Chloroform-d) δ=7.60 (d, J=1.6, 1H), 7.49 (t, J=1.9, 1H), 7.42 (p, J=3.7, 4H), 7.08 (d, J=1.9, 1H), 3.82 (d, J=3.1, 2H), 3.03 (dd, J=21.1, 6.9, 4H), 1.50 (s, 9H).

### (1-5-8) Synthesis of Linker Intermediate (32)

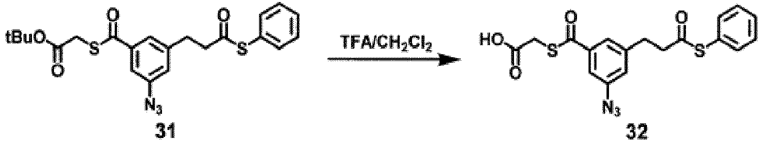

The linker intermediate (31) (247.8 mg, 0.54 mmol) was dissolved in a mixed solvent of CH₂Cl₂/TFA=1/1 (10 mL) and the mixture was stirred at room temperature for 1 hour. After confirming the reaction with LC/MS and TLC (hexane/ethyl acetate=5/1), the organic solvent was removed by concentration in vacuo, followed by vacuum-drying to obtain the linker intermediate (32) (227.3 mg, 0.56 mmol).
¹H NMR (400 MHz, Chloroform-d) δ=7.70 (d, J=1.6, 1H), 7.42 (t, J=1.9, 1H), 7.41 (p, J=3.7, 4H), 7.01 (d, J=1.9, 1H), 3.67 (d, J=3.1, 2H), 3.20 (dd, J=21.1, 6.9, 4H) .
MS(ESI) m/z: z=1 402.29 [M+H]⁺

### (1-5-9) Synthesis of Modification Reagent (24)

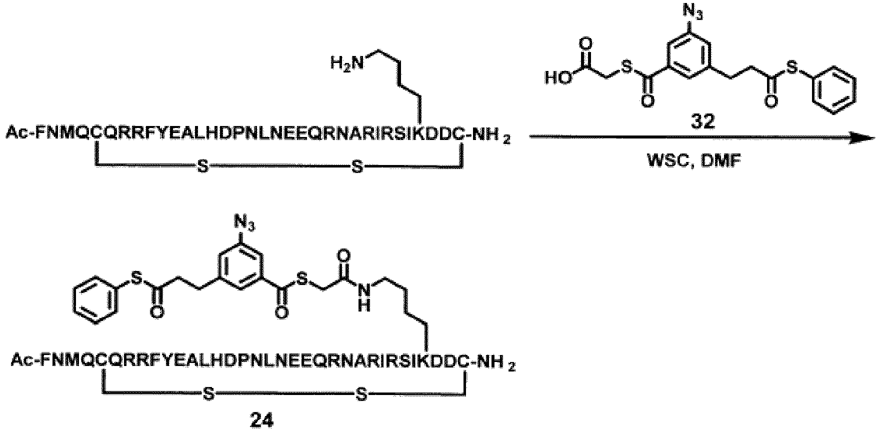

Each of the above amino acid sequences is the amino acid sequence of SEQ ID NO: 4.

Ac-FNMQCQRRFYEALHDPNLNEEQRNARIRSIKDDC-NH₂ (SEQ ID NO: 4, 330.0 mg, 7.06 µmol, with the two cysteines at positions 5 and 34 each forming an intramolecular disulfide bond) and the linker intermediate (32) (29 mg, 70.6 µmol), WSC·HCl (13.5 mg, 70. 6 µmol) were dissolved in DMF (1.00 mL) and the mixture was stirred at room temperature for 1 hour. After the reaction was confirmed by LC-MS, the reaction was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. Fractions containing the product were collected and concentrated in vacuo to remove acetonitrile, followed by freeze-drying to afford the modification reagent (24) (5.5 mg, 1.19 µmol).
MS(ESI) m/z: z=3 928.30 [M+3H]³⁺

### (1-6) Synthesis of Modification Reagent (33)

### (1-6-1) Synthesis of Linker Intermediate (34)

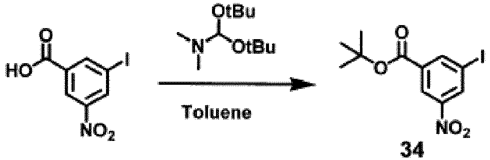

3-Iodo-5-nitrobenzoic acid (1.0 g, 3.41 mmol) was dissolved in toluene (17 mL) and N,N-dimethylformamide di-tert-butyl acetal (5 mL, 20.5 mmol) was added and the mixture was stirred at 80°C for 2 hours. After confirming the reaction with LC/MS and TLC (hexane/ethyl acetate=5/1), the reaction solution was concentrated. Vacuum-drying gave the linker intermediate (34) (1.37 g, 3.92 mmol).

### (1-6-2) Synthesis of Linker Intermediate (35)

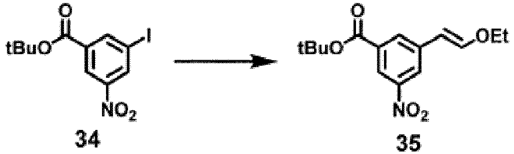

The linker intermediate (34) (1.25 g, 3.58 mmol) was dissolved in a mixed solution of 1,4-dioxane/H₂O=4/1 (18 mL), and (E)-ethoxyethene-2-boronic acid pinacol ester (851 mg, 4.3 mmol), Pd(dppf)Cl₂ (146 mg, 0.179 mmol) and Na₂CO₃ (948 mg, 8.95 mmol) were added, and the mixture was stirred at 100°C for 3 hours. After confirming the reaction with LC/MS and TLC (hexane/ethyl acetate=10/1), the reactant was extracted with ethyl acetate and water, and the organic layer was concentrated. The reactant was eluted with a mixed solution of hexane and ethyl acetate and the fractions were confirmed by TLC (hexane/ethyl acetate=10/1). The fraction containing the product was collected and concentrated in vacuo to remove the organic solvents, followed by vacuum-drying to obtain the linker intermediate (35) (415 mg, 1.41 mmol).

(1-6-3) Synthesis of Linker Intermediate (36)

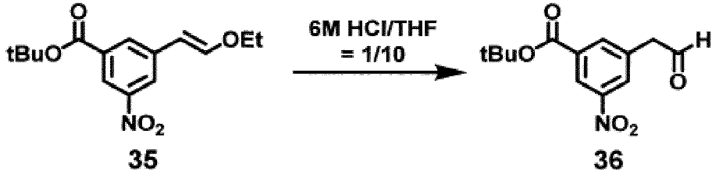

The linker intermediate (35) (614 mg, 2.09 mmol) was dissolved in THF (20 mL) and 1 M HCl aqueous solution (2.0 mL) and 6 M HCl aqueous solution (1 mL) were added and the mixture was stirred at 60°C for 20 hours. After confirming the reaction with TLC (hexane/ethyl acetate=2/1), the reactant was extracted with an aqueous solution of ethyl acetate and 1 M HCl, and the organic layer was concentrated. The reactant was eluted with a mixed solution of hexane and ethyl acetate and the fractions were confirmed by TLC (hexane/ethyl acetate=2/1). The fraction containing the product was collected and concentrated in vacuo to remove the organic solvents, followed by vacuum-drying to obtain the linker intermediate (36) (426.4 mg, 1.61 mmol).

### (1-6-4) Synthesis of Linker Intermediate (37)

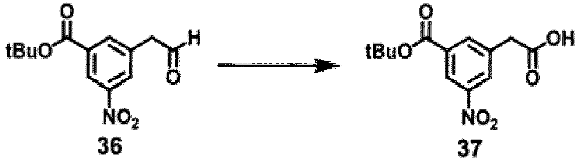

The linker intermediate (36) (426.4 mg, 1.61 mmol) was dissolved in tBuOH (16.1 mL), 2-methyl-2-butene (1.71 mL, 16.1 mmol), 0.85 M NaH₂PO₄ aqueous solution (11.4 mL, 9.66 mmol), and 0.5 M NaClO₂ aqueous solution (16.1 mL, 8.05 mmol) were added, and the mixture was stirred at room temperature for 1.5 hours. After confirming the reaction with LC/MS, aqueous 1.0M NaHSOs solution (16.1 mL, 16.1 mmol) was added and the reaction was stopped. The aqueous 1 M HCl solution was added to make it slightly acidic, the reactant was extracted with ethylacetate, and the organic layer was concentrated. Vacuum-drying gave the linker intermediate (37) (591.5 mg, 2.10 mmol).

### (1-6-5) Synthesis of Linker Intermediate (38)

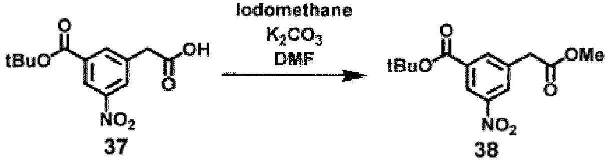

The linker intermediate (37) (591.5 mg, 2.10 mmol) was dissolved in DMF and iodomethane (392 µg, 6.30 mmol) and potassium carbonate (319 mg, 2.31 mmol) were added and the mixture was stirred for 1.5 hours. After confirming the reaction with LC/MS and TLC (hexane/ethyl acetate=3/1), the reactant was extracted with ethyl acetate and water, and the organic layer was concentrated. Vacuum-drying gave the linker intermediate (38) (413.5 mg, 1.40 mmol) .

### (1-6-6) Synthesis of Linker Intermediate (39)

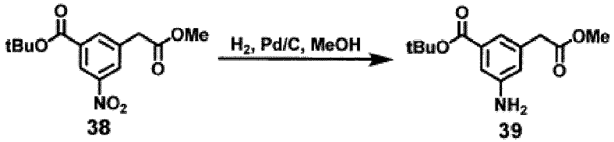

The linker intermediate (38) (413.5 mg, 1.40 mmol) was dissolved in methanol and Pd/C (317 mg, 0.07 mmol) was added and the mixture was stirred under hydrogen for 1 hour. After the reaction was confirmed by LC/MS, Pd/C was removed by a membrane filter, and the solvents was concentrated. Vacuum-drying gave the linker intermediate (39) (348.4 mg, 1.31 mmol).

### (1-6-7) Synthesis of Linker Intermediate (40)

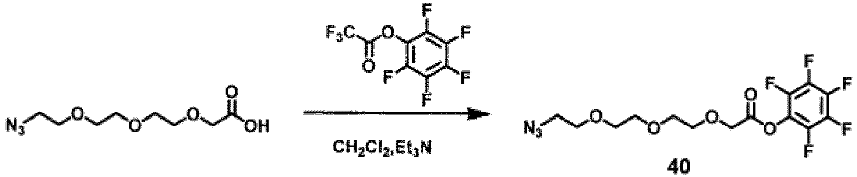

11-Azido-3,6,9-trioxaundecanoic acid (700 mg, 3.0 mmol) was dissolved in CH₂Cl₂ (15.0 mL) and triethylamine (2.09 mL, 15.0 mmol) and pentafluorophenyltrifluoroacetic acid (1.02 mL, 6.0 mmol) were added at 0°C and the mixture was stirred at 0°C for 1 hour. After confirming the reaction with TLC (hexane/ethyl acetate=2/1), the reaction solution was concentrated. The reactant was eluted with a mixed solution of hexane and ethyl acetate and the fractions were confirmed by TLC (hexane/ethyl acetate=2/1). Fractions containing the product were collected and concentrated in vacuo to remove the organic solvents, followed by vacuum-drying to obtain linker intermediate (40) (1.4 g, 3.51 mmol).

### (1-6-8) Synthesis of Linker Intermediate (41)

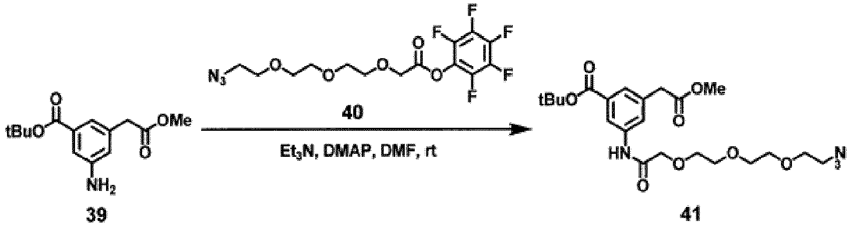

The linker intermediate (39) (348.4 mg, 1.31 mmol) was dissolved in DMF (6.55 mL) and the linker intermediate (40) (523 mg, 1.31 mmol), DIPEA (445 µL, 2.62 mmol) and DMAP (16.0 mg, 0.131 mmol) were added and the mixture was stirred at room temperature for 1 hour. After the reaction was confirmed by LC/MS, the reactant was extracted with water and ethyl acetate, and the organic layer was concentrated. Vacuum-drying gave the linker intermediate (41) (429.4 mg, 0.89 mmol).

### (1-6-9) Synthesis of Linker Intermediate (42)

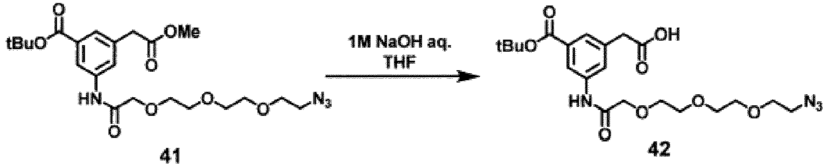

The linker intermediate (41) (429.4 mg, 0.89 mmol) was dissolved in THF (4.45 mL) and aqueous 1 M NaOH solution (2.67 mL, 2.67 mmol) was added and the mixture was stirred at room temperature for 1 hour. After the reaction was confirmed by LC/MS, the reaction solution was acidified with 2 M HCl aqueous solution, the reactant was extracted with ethylacetate, and the organic layer was concentrated. Vacuum-drying gave the linker intermediate (42) (412.5 mg, 0.88 mmol) .

### (1-6-10) Synthesis of Linker Intermediate (43)

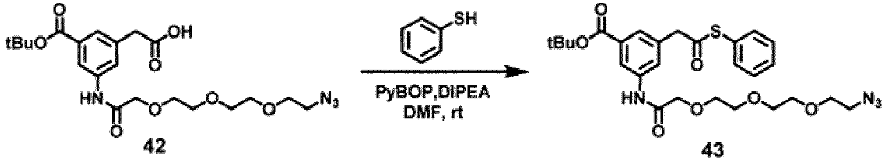

The linker intermediate (42) (412.5 mg, 0.88 mmol) was dissolved in DMF (4.40 mL) and benzenethiol (89. 7 µL, 0.88 mmol), PyBOP (458 mg, 0.88 mmol) and DIPEA (226 µL, 1.33 mmol) were added and the mixture was stirred at room temperature for 1 hour. After confirming the reaction with LC/MS and TLC (ethyl acetate), the reactant was extracted with ethyl acetate and water, and the organic layer was concentrated. The reactant was eluted with a mixed solution of hexane and ethyl acetate, and the fractions were confirmed by TLC (ethyl acetate). Fractions containing the product were collected and concentrated in vacuo to remove the organic solvents, followed by vacuum-drying to obtain linker intermediate (43) (388.3 mg, 0.69 mmol) .

### (1-6-11) Synthesis of Linker Intermediate (44)

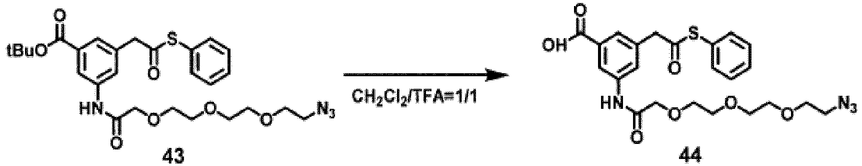

The linker intermediate (43) (388.3 mg, 0.69 mmol) was dissolved in a mixed solvent of CH₂Cl₂/TFA=1/1 and the mixture was stirred at room temperature for 1 hour. After the reaction was confirmed with LC/MS and TLC (ethyl acetate), the organic solvents was removed, followed by vacuum-drying to obtain the linker intermediate (44) (382.7 mg, 0.76 mmol).

### (1-6-12) Synthesis of Linker Intermediate (45)

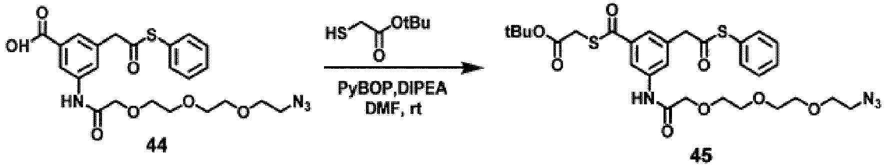

The linker intermediate (44) (382.7 mg, 0.76 mmol) was dissolved in DMF (3.8 mL), and tBu-2-sulfanylacetate (112.6 mL, 0.76 mmol), PyBOP (395 mg, 0.76 mmol) and DIPEA (194 µL, 1.14 mmol) were added and the mixture was stirred at room temperature for 1 hour. After confirming the reaction with LC/MS and TLC (ethyl acetate), the reactant was extracted with ethyl acetate and water, and the organic layer was concentrated. The reactant was eluted with a mixed solution of hexane and ethyl acetate, and the fractions were confirmed by TLC (ethyl acetate). Fractions containing the product were collected and concentrated in vacuo to remove the organic solvents, followed by vacuum-drying to obtain linker intermediate (45) (350 mg, 0.55 mmol).

### (1-6-13) Synthesis of Linker Intermediate (46)

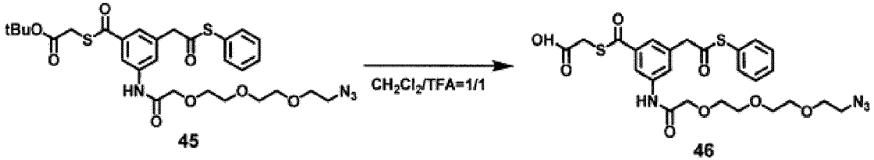

The linker intermediate (45) (350 mg, 0.55 mmol) was dissolved in a mixed solvent of CH₂Cl₂/TFA=1/1 and the mixture was stirred at room temperature for 1 hour. After the reaction was confirmed by LC/MS, the organic solvent was removed, and the reactant was eluted with a mixed solution of dichloromethane and methanol. The respective fractions were confirmed by LC/MS. The fraction containing the product was collected and concentrated in vacuo to remove the organic solvents, followed by vacuum-drying to obtain linker intermediate (46) (130.8 mg, 0.23 mmol).
MS(ESI) m/z: z=1 577.83 [M+H]⁺

### (1-6-14) Synthesis of Modification Reagent (33)

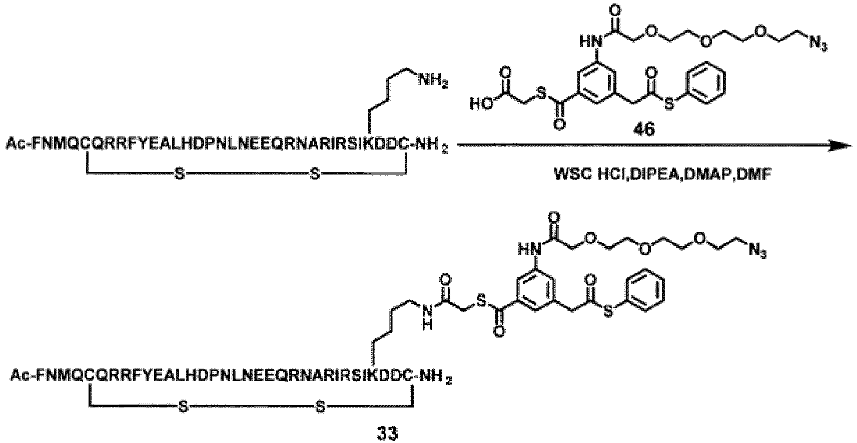

Each of the above amino acid sequences is the amino acid sequence of SEQ ID NO: 4.

Ac-FNMQCQRRFYEALHDPNLNEEQRNARIRSIKDDC-NH₂ (SEQ ID NO: 4, 30.0 mg, 7.06 µmol, with two cysteines at positions 4 and 14 each forming an intramolecular disulfide bond) and the linker intermediate (46) (40.7 mg, 70.6 µmol) were dissolved in DMF (1.00 mL) and WSC·HCl (13.5 mg, 70. 6 µmol) and DIPEA (3. 6 µL, 21.2 µmol) were added, and the mixture was stirred at room temperature for 2 hours. After the reaction was confirmed by LC-MS, the reaction was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. Fractions containing the product were collected and concentrated in vacuo to remove acetonitrile, followed by freeze-drying to afford the modification reagent (33) (4.1 mg, 0.85 µmol).
MS(ESI) m/z: z=3 802.50 [M+3H]³⁺

### (1-7) Synthesis of Modification Reagent (47)

### (1-7-1) Synthesis of Linker Intermediate (48)

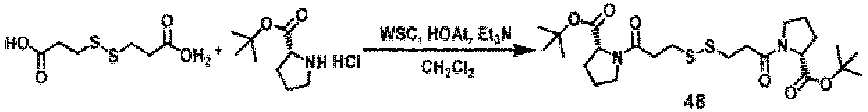

3,3'-dithiodipropionic acid (608 mg, 2.89 mmol) was dissolved in dichloromethane (15 mL), and D-proline tert-butylhydrochloride (1.32 g, 6.36 mmol), WSC·HCl (1.39 mg, 2.72 mmol), 1-hydroxy-7-azabenzotriazole (78.7 mg, 0.578 mmol) and triatylamine (2.00 mL, 14.5 mmol) were added, and the mixture was stirred at room temperature overnight. After concentration under reduced pressure, the solution was diluted by adding ethyl acetate, the mixture was washed with water and brine, and then sodium sulfate was added. Sodium sulfate was removed by filtration, the solution was concentrated under reduced pressure, and the reactant was purified by column chromatography (hexane:ethyl acetate=1:1). The fraction containing the product was collected and concentrated in vacuo to give the linker intermediate (48) (1.06 g, 2.04 mmol).
¹H NMR (400MHz, Chloroform-d) δ4.40 (dd, J=8.6, 3.5 Hz, 2H), 3.64-3.49 (m, J=8.5, 7.9, 4H), 3.08-2.86 (m, 4H), 2.77 (td, J=8.1, 7.6, 3.3 Hz, 4H), 2.23-1.87 (m, 8H), 1.68 (s, 2H), 1.47 (s, 16H), 1.40-1.24 (m, 2H), 0.90 (t, J=6.6Hz, 2H).
MS(ESI) m/z: 517 [M+H]⁺

### (1-7-2) Synthesis of Linker Intermediate (49)

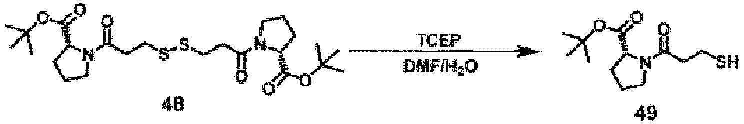

The linker intermediate (48) (1.06 g,2.04 mmol) was dissolved in DMF (5 mL), tris(2-carboxyethyl)phosphine (760 mg,2.65 mmol) dissolved in water (5 mL) was added, and the mixture was stirred at room temperature overnight, and the reaction was diluted with ethyl acetate, washed with water and brine, and then sodium sulfate was added. Sodium sulfate was removed by filtration, concentrated under reduced pressure, and purified by column chromatography (hexane:ethyl acetate=1:1). The fraction containing the product was collected and concentrated in vacuo to afford the linker intermediate (49) (859 mg,3.70 mmol).
¹H NMR (400MHz, Chloroform-d) δ4.42 (dd, J=8.6, 3.5 Hz, 1H), 3.70-3.56 (m, 2H), 3.52 (dt, J=9.6, 6.9 Hz, 1H), 2.84 (pd, J=11.1, 9.7, 6.0 Hz, 3H), 2.75-2.43 (m, 3H), 2.31-1.86 (m, 5H), 1.86-1.64 (m, 3H), 1.48 (s,9H).
MS(ESI) m/z: 260 [M+H]⁺

### (1-7-3) Synthesis of Linker Intermediate (50)

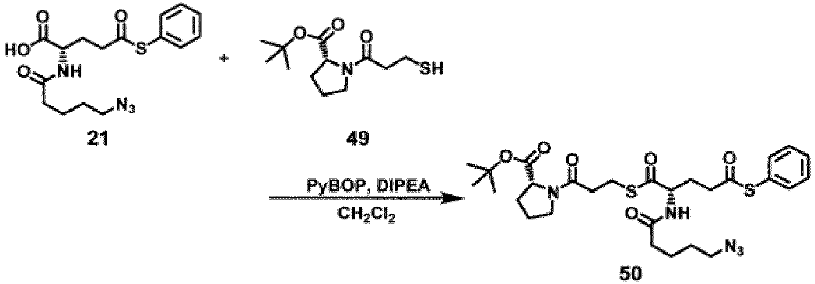

The linker intermediate (21) (52.4 mg, 0.144 mmol) was dissolved in dichloromethane (1 mL), the linker intermediate (49) (41.9 mg, 0.158 mmol), benzotriazol-1-yloxy (89.9 mg, 0.173 mmol) and DIPEA (73.5 µL, 0.432 mmol) were added, and the mixture was stirred at room temperature for 1 hour. After concentration under reduced pressure, the mixture was purified by column chromatography (hexane:ethyl acetate=1:1). The fraction containing the product was collected and concentrated in vacuo to afford the linker intermediate (50) (38.7 mg, 0.0639 mmol).
¹H NMR (400 MHz, Chloroform-d) δ7.49-7.37 (m, 5H), 6.26 (q, J=11.3, 9.5 Hz, 1H), 4.72 (tt, J=8.1, 3.9 Hz, 1H), 4.39 (dd, J=8.6, 3.3 Hz, 1H), 3.67-3.40 (m, 2H), 3.31 (t, J=6.6 Hz, 2H), 3.20 (dt, J=14.4, 7.1 Hz, 2H), 2.92-2.40 (m, 4H), 2.30 (t, J=6.9, 6.2 Hz, 3H), 1.80-1.60 (m, 4H), 1.48 (d, J=2.1 Hz, 9H).
MS(ESI) m/z: 606 [M+H]⁺

### (1-7-4) Synthesis of Linker Intermediate (51)

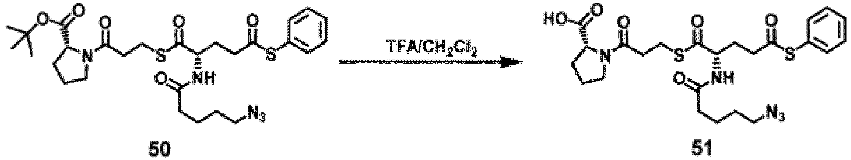

The linker intermediate (50) (38.7 mg, 0.0639 mmol) was dissolved in dichloromethane (5 mL), and trifluoroacetic acid (5 mL) was added, and the mixture was stirred at room temperature for 1 hour, then concentrated in vacuo to remove dichloromethane, and water was added, followed by freeze-drying to obtain the linker intermediate (51) (39.8 mg, 0.0639 mmol).

¹H NMR (400 MHz, Chloroform-d) δ7.85 (s, 3H), 7.43 (q, J=5.8, 4.9 Hz, 5H), 6.82 (s, 1H), 4.80-4.48 (m, 2H), 3.78-3.40 (m, 2H), 3.31 (t, J=6.5 Hz, 2H), 3.19 (t, J=6.9 Hz, 2H), 2.94-2.51 (m, 4H), 2.41-2.22 (m, 4H), 2.22-1.87 (m, 4H), 1.68 (dp, J=28.1, 7.3 Hz, 4H).

### (1-7-5) Synthesis of Linker Intermediate (52)

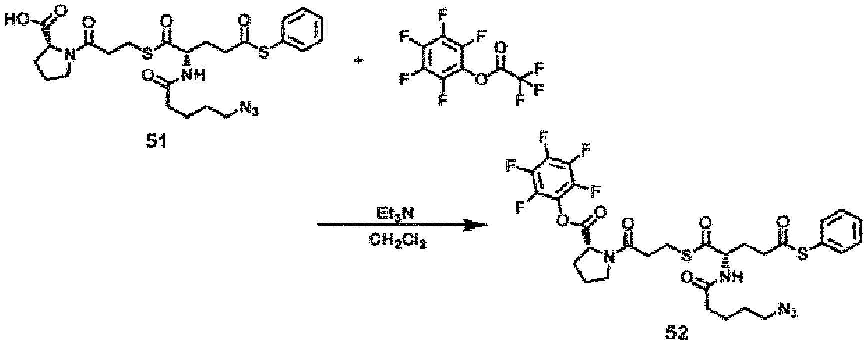

The linker intermediate (51) (37.7 mg, 0.0686 mmol) was dissolved in dichloromethane (1 mL), pentafluorophenyl trifluoroacetic acid (23.4 mg, 0.137 mmol), triethylamine (28.6 microL, 0.206 mmol) was added, and the mixture was stirred at room temperature for 1hour. After concentration under reduced pressure, the mixture was purified by column chromatography (hexane:ethyl acetate=1:1). The fraction containing the product was collected and concentrated in vacuo to afford the linker intermediate (52) (37.4 mg, 0.0523 mmol).
MS(ESI) m/z: 716 [M+H]⁺

### (1-7-6) Synthesis of Modification Reagent (47)

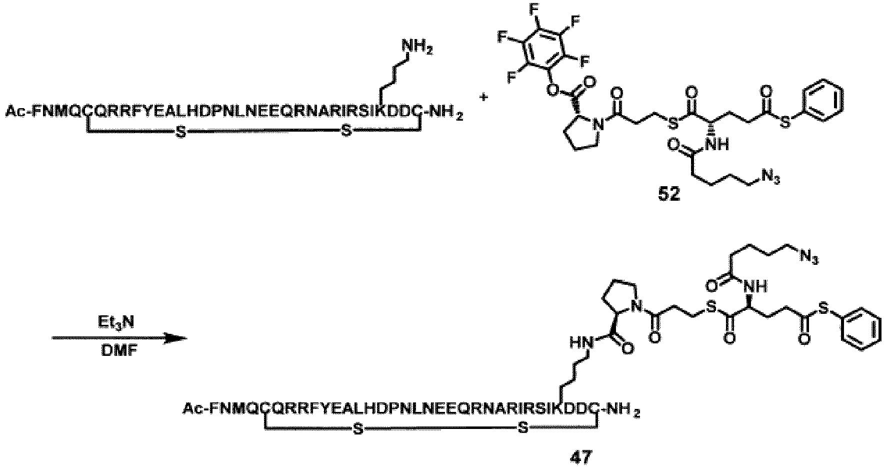

Each of the above amino acid sequences is the amino acid sequence of SEQ ID NO: 4.

Ac-FNMQCQRRFYEALHDPNLNEEQRNARIRSIKDDC-NH₂ peptide (SEQ ID NO: 4, 22.2 mg, 5.23 µmol, with two cysteines at positions 5 and 35 each forming an intramolecular disulfide bond) was dissolved in DMF (374 µL), a linker intermediate (52) (37.4 mg, 52.3 µmol) and triethylamine (2.2 µL, 0.0157 mmol) were added, and the mixture was stirred at room temperature for 3 hours, and then purified by reverse-phase preparative chromatography. The fraction containing the product was collected, and the acetonitrile was removed by concentration in vacuo, followed by freeze-drying to obtain the above-described modification reagent (47) (7.76 mg, 1.62 µmmol).
MS(ESI) m/z: z=3 1595 [M+3H]³⁺, z=4 1196 [M+4H]⁴⁺, z=5 957 [M+5H]⁵⁺, z=6 798 [M+6H]⁶⁺

### (1-8) Synthesis of Modification Reagent (53)

### (1-8-1) Synthesis of Linker Intermediate (54)

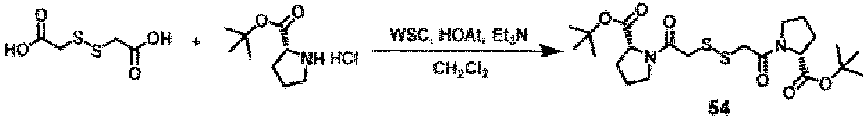

Dithioglycolic acid (613 mg, 3.36 mmol) was dissolved in dichloromethane (17 mL), and D-proline tert-butylhydrochloride (1.53 g, 7.40 mmol), WSC·HCl (1.61 mg, 8.40 mmol), 1-hydroxy-7-azabenzotriazole (91.4 mg, 0.672 mmol), and triatylamine (2.33 mL, 16.8 mmol) were added, and the mixture was stirred at room temperature overnight. After concentration under reduced pressure, ethyl acetate was added and diluted, the solution was washed with water and brine, and then sodium sulfate was added. Sodium sulfate was removed by filtration, the solution was concentrated under reduced pressure, and the reactant was purified by column chromatography (hexane:ethyl acetate=1:1). The fraction containing the product was collected and concentrated in vacuo to give the linker intermediate (54) (1.09 g, 2.23 mmol).
¹H NMR (400 MHz, Chloroform-d) δ4.39 (dt, J=8.3, 3.6 Hz, 2H), 3.79-3.60 (m, 8H), 2.35-2.14 (m, 2H), 2.14-1.85 (m, 6H), 1.47 (s, 18H).
MS(ESI) m/z: 489 [M+H]⁺

### (1-8-2) Synthesis of Linker Intermediate (55)

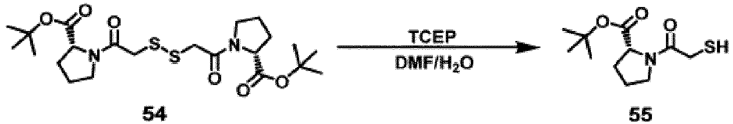

The linker intermediate (54) (1.09 g, 2.23 mmol) was dissolved in DMF (5 mL), and tris(2-carboxyethyl)phosphine (831 mg, 2.90 mmol) dissolved in water (5 mL) was added, and the mixture was stirred at room temperature overnight, and the reaction solution was diluted with ethyl acetate, washed with water and brine, and then sodium sulfate was added. Sodium sulfate was removed by filtration, the solution was concentrated under reduced pressure, and the reactant was purified by column chromatography (hexane:ethyl acetate=1:1). The fraction containing the product was collected and concentrated in vacuo to afford the linker intermediate (55) (867 mg, 3.53 mmol) .

¹H NMR (400 MHz, Chloroform-d) δ4.39 (ddd, J=14.0, 8.4, 3.1 Hz, 1H), 3.77-3.53 (m, 2H), 3.39-3.24 (m, 2H), 2.33-2.06 (m, 2H), 2.06 (m, 2H), 1.49 (d, J=4.5 Hz, 9H).
MS(ESI) m/z: 246 [M+H]⁺

### (1-8-3) Synthesis of Linker Intermediate (56)

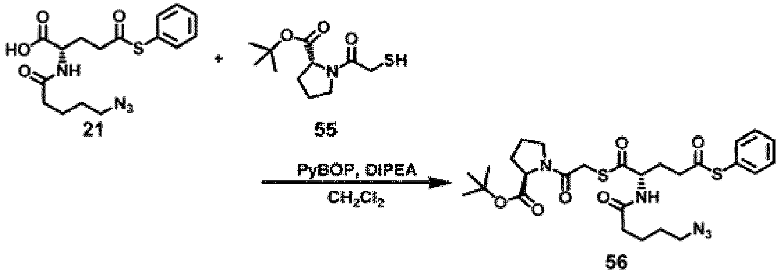

The linker intermediate (21) (264 g, 0.723 mmol) was dissolved in dichloromethane (4 mL), and the linker intermediate (55) (195 mg, 0.795 mmol), benzotriazol-1-yloxy (452 mg, 0.868 mmol) and DIPEA (370 µL, 2.17 mmol) were added, and the mixture was stirred at room temperature for 1 hour. After concentration under reduced pressure, the mixture was purified by column chromatography (hexane:ethyl acetate=1:1). The fraction containing the product was collected and concentrated in vacuo to afford the linker intermediate (56) (180 mg, 0.303 mmol).

¹H NMR (400 MHz, Chloroform-d) δ7.44 (p, J=3.5 Hz, 5H), 6.52 (m, 1H), 4.75 (td, J=8.5, 4.6 Hz, 1H), 4.42 (ddd, J=19.2, 8.4, 3.3 Hz, 1H), 3.80-3.49 (m, 4H), 3.39-3.26 (m, 2H), 2.94-2.67 (m, 2H), 2.39-2.27 (m, 2H), 2.27-1.87 (m, 3H), 1.87-1.57 (m, 8H), 1.47 (d, J=3.2 Hz, 9H).
MS(ESI) m/z: 592 [M+H]⁺

### (1-8-4) Synthesis of Linker Intermediate (57)

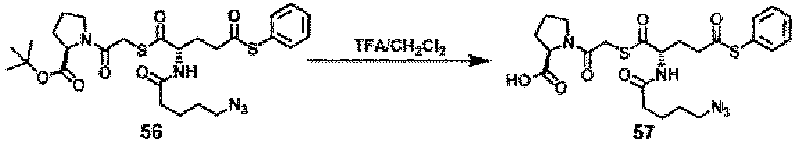

The linker intermediate (56) (180 mg, 0.303 mmol) was dissolved in dichloromethane (5 mL), trifluoroacetic acid (5mL) was added, and the mixture was stirred at room temperature for 1 hour, then concentrated in vacuo to remove dichloromethane, and water was added, followd by freeze-drying to obtain the linker intermediate (57) (172.6 mg,0.322 mmol).

¹H NMR (400 MHz, Chloroform-d) δ7.43 (q, J=5.7, 4.7 Hz, 5H), 4.71 (td, J=8.7, 4.7 Hz, 1H), 4.53 (t, J=5.7 Hz, 1H), 3.97-3.47 (m, 4H), 3.32 (dt, J=12.9, 6.5 Hz, 2H), 2.82 (qq, J=15.4, 7.4, 6.1 Hz, 2H), 2.44-2.00 (m, 8H), 1.87-1.59 (m, 4H) .
MS(ESI) m/z: 536 [M+H]⁺

### (1-8-5) Synthesis of Linker Intermediate (58)

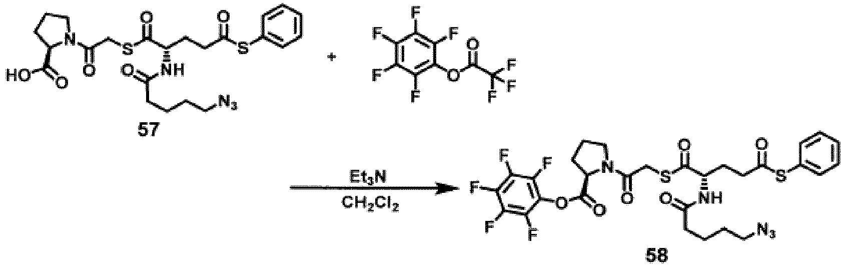

The linker intermediate (57) (172.6 mg, 0.322 mmol) was dissolved in dichloromethane (1.6 mL), and pentafluorophenyl trifluoroacetic acid (110 mL, 0.644 mmol) and triethylamine (134 µL, 0.966 mmol) was added, and the mixture was stirred at room 3hours. After concentration under reduced pressure, the mixture was purified by column chromatography (hexane:ethyl acetate=1:1). The fraction containing the product was collected and concentrated in vacuo to afford the linker intermediate (58) (158.6 mg, 0.226 mmol).
¹H NMR (400 MHz, Chloroform-d) δ7.47-7.36 (m, 5H), 6.59-6.40 (m, 1H), 4.92-4.78 (m, 1H), 4.73 (tt, J=7.6, 3.5 Hz, 1H), 4.01-3.62 (m, 4H), 3.31 (qd, J=6.6, 2.8 Hz, 2H), 2.99-2.68 (m, 2H), 2.50-2.09 (m, 8H), 1.84-1.56 (m, 4H) .
MS(ESI) m/z: 702 [M+H]⁺

### (1-8-6) Synthesis of Modification Reagent (53)

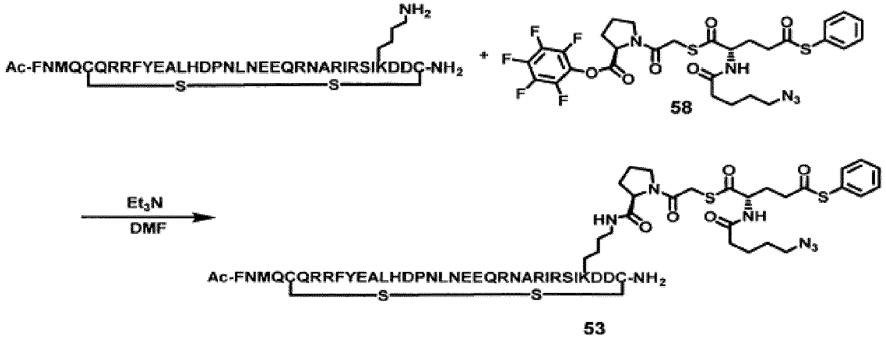

Each of the above amino acid sequences is the amino acid sequence of SEQ ID NO: 4.

The peptide of Ac-FNMQCQRRFYEALHDPNLNEEQRNARIRSIKDDC-NH₂ (SEQ ID NO: 4, 96.1 mg, 22.6 µmol, with two cysteines at positions 5 and 35 each forming an intramolecular disulfide bond) was dissolved in DMF (1.59 mL), and the linker intermediate (58) (159 mg, 226 µmol) and triethylamine (9.4 µL, 67.8 µmol) were added, and the mixture was stirred at room temperature for 3 hours, and then the reactant was purified by reverse-phase preparative chromatography. Fractions containing the product were collected, and the acetonitrile was removed by concentration in vacuo, followed by freeze-drying to obtain the modification reagent (53) (49.4 mg, 10.4 µmmol).
MS(ESI) m/z: z=3 1590 [M+3H]³⁺, z=4 1193 [M+4H]⁴⁺, z=5 954 [M+5H]⁵⁺, z=6 795 [M+6H]⁶⁺

### (1-9) Synthesis of the modification reagent (59)

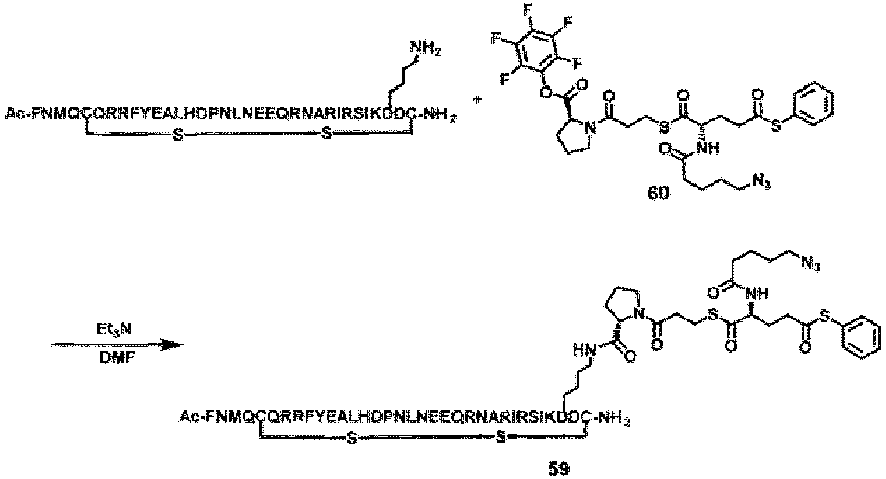

Each of the above amino acid sequences is the amino acid sequence of SEQ ID NO: 4.

The peptide of Ac-FNMQCQRRFYEALHDPNLNEEQRNARIRSIKDDC-NH₂ (SEQ ID NO: 4, 24.5 mg, 5.77 µmol, with two cysteines at positions 5 and 35 each forming an intramolecular disulfide bond) was dissolved in DMF (500 µL), a linker intermediate (41.3 mg, 57.7 µmol) derived from L-proline tert-butyl hydrochloride (60) by procedures similar to those of the linker intermediate (52) in Example (1-7-5) and triethylamine (2.40 µL, 17.3 mmol) were added, and the mixture was stirred at room temperature for 3 hours, and then the reactant was purified by reverse phase preparative chromatography. The fraction containing the product was collected, and the acetonitrile was removed by concentration in vacuo, followed by freeze-drying to obtain a modification reagent (59) (13.4 mg, 2.80 pmmol).
MS(ESI) m/z: z=4 1196 [M+4H]⁴⁺, z=5 957 [M+5H]⁵⁺, z=6 798 [M+6H]⁶⁺

### (1-10) Synthesis of Modification Reagent (61)

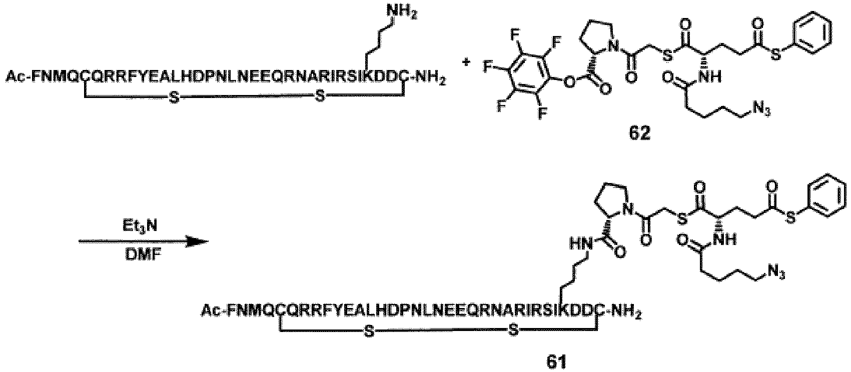

Each of the above amino acid sequences is the amino acid sequence of SEQ ID NO: 4.

The peptide of Ac-FNMQCQRRFYEALHDPNLNEEQRNARIRSIKDDC-NH₂ (SEQ ID NO: 4, 14.0 mg, 3.31 µmol, with two cysteines at positions 5 and 35 each forming an intramolecular disulfide bond) was dissolved in DMF (300 mL), and a linker intermediate (62) (23.2 mg, 33.1 µmol) derived from L-proline tert-butyl hydrochloride by procedures similar to those of the linker intermediate (58) in Example (1-8-5) and triethylamine (1.40 µL, 9.93 µmol) were added, followed by stirring at room temperature for 3 hours, and then the reactant was purified by reverse phase preparative chromatography. The fraction containing the product was collected, and the acetonitrile was removed by concentration in vacuo, followed by freeze-drying to obtain the above-described modification reagent (61) (6.6 mg, 1.38 pmmol).
MS(ESI) m/z: z=3 1590 [M+3H]³⁺, z=4 1193 [M+4H]⁴⁺, z=6 795 [M+6H]⁶⁺

### (1-11) Synthesis of Modification Reagent (63)

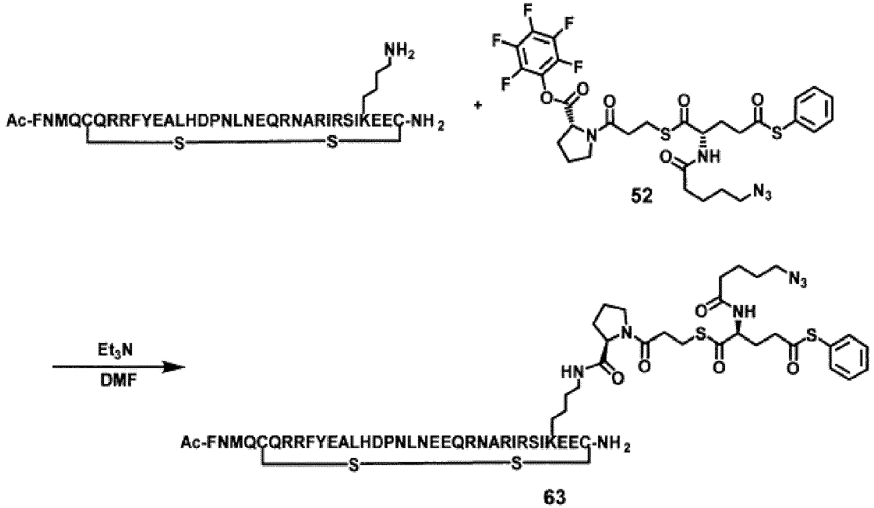

Each of the above amino acid sequences is the amino acid sequence of SEQ ID NO: 5.

The peptide of Ac-FNMQCQRRFYEALHDPNLNEEQRNARIRSIKEEC-NH₂ (SEQ ID NO: 5, 100 mg, 23.4 µmol, with two cysteines at positions 5 and 35 each forming an intramolecular disulfide bond) was dissolved in DMF (1002 µL), and the linker intermediate (52) (50.3 mg, 70.2 µmol) and triethylamine (9.8 µL, 70.2 µmol) were added, and the mixture was stirred at room temperature for 30 minutes, and then the reactant was purified by reverse-phase preparative chromatography. The fraction containing the product was collected, and the acetonitrile was removed by concentration in vacuo, followed by freeze-drying to obtain the modification reagent (63) (63.7 mg, 13.2 pmmol).
MS(ESI) m/z: z=3 1603 [M+3H]³⁺, z=4 1203 [M+4H]⁴⁺, z=5 963[M+5H]⁵⁺

### (1-12) Synthesis of Modification Reagent (64)

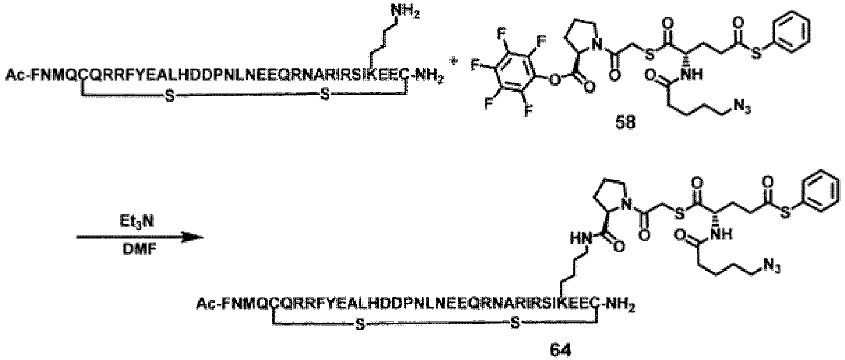

Each of the above amino acid sequences is the amino acid sequence of SEQ ID NO: 5.

The peptide of Ac-FNMQCQRRFYEALHDPNLNEEQRNARIRSIKEEC-NH₂ (SEQ ID NO: 5, 101 mg, 23.6 µmol, with two cysteines at positions 5 and 35 each forming an intramolecular disulfide bond) was dissolved in DMF (1.01 mL), and the linker intermediate (58) (50.0 mg, 71.3 µmol) and triethylamine (9.9 µL, 71.3 µmol) were added, and the mixture was stirred at room temperature for 1.5 hours, and then the reactant was purified by reverse-phase preparative chromatography. The fraction containing the product was collected, and the acetonitrile was removed by concentration in vacuo, followed by freeze-drying to obtain the modification reagent (64) (61.2 mg, 12.8 pmmol).
MS(ESI) m/z: z=3 1599 [M+3H]³⁺, z=4 1200 [M+4H]⁴⁺, z=5 960 [M+5H]⁵⁺, z=6 800 [M+6H]⁶⁺

### (1-13) Synthesis of Modification Reagent (65)

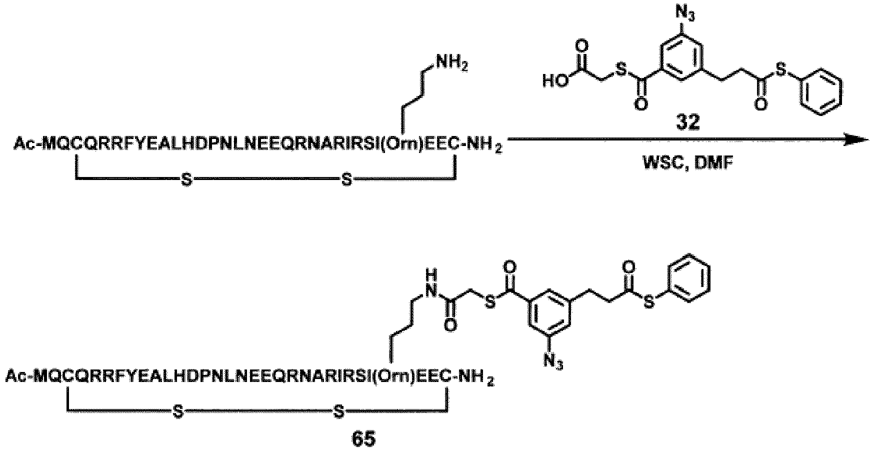

Each of the above amino acid sequences is the amino acid sequence of SEQ ID NO: 6.

Ac-MQCQRRFYEALHDPNLNEEQRNARIRSI(Orn)EEC-NH₂ (SEQ ID NO: 6, 30.0 mg, 7.08 µmol, with the two cysteines at positions 3 and 32 each forming an intramolecular disulfide bond) and the linker intermediate (32) (28.4 mg, 70.8 µmol) were dissolved in DMF (1.00 mL) and WSC·HCl (13.6 mg, 70.8 µmol) were added and the mixture was stirred at room temperature for 2 hours. After the reaction was confirmed by LC-MS, the reaction solution was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. Fractions containing the product were collected and concentrated in vacuo to remove acetonitrile, followed by freeze-drying to afford the above peptide (5.9 mg, 1.34 µmol).
MS(ESI) m/z: z=4 1098.20 [M+3H]³⁺

### (1-14) Synthesis of Modification Reagent (66)

### (1-14-1) Synthesis of Linker Intermediate (67)

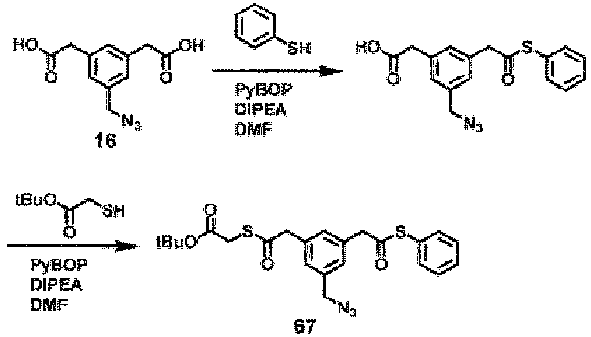

The linker intermediate (16) (400 mg, 1.60 mmol) synthesized in Example (1-3-5) was dissolved in DMF (8.0 mL), and benzenethiol (163 µL, 1.60 mmol), PyBOP (833 mg, 1.60 mmol) and DIPEA (408 µL, 2.40 mmol) were added, and the mixture was stirred at room temperature for 1 hour. After confirming the reaction with LC/MS, a tBu-2-Sulfanylacetate (237 mg, 1.60 mmol), PyBOP (833 mg, 1.60 mmol) and DIPEA (408 µL, 2.40 mmol) were added and the mixture was stirred at room temperature for 1 hour. After confirming the reaction with LC/MS and TLC (hexane/ethyl acetate=3/1), the reactant was extracted with ethyl acetate and water, and the organic layer was concentrated. The reactant was eluted with a mixed solution of hexane and ethyl acetate and the fractions were confirmed by TLC (hexane/ethyl acetate=3/1). The fractions containing the product were collected and concentrated in vacuo to remove the organic solvent to afford the linker intermediate (67).

### (1-14-2) Synthesis of Linker Intermediate (68)

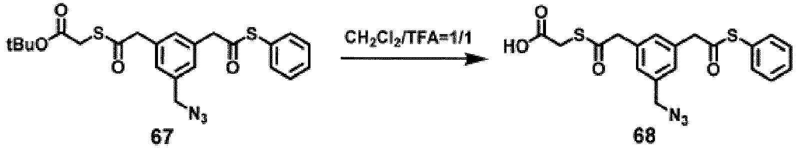

The linker intermediate (67) was dissolved in a mixed solvent of CH₂Cl₂/TFA=1/1 (10.0 mL) and the mixture was stirred at room temperature for 1 hour. After the reaction was confirmed with LC/MS and TLC (dichloromethane/methanol=10/1), the organic solvent was removed. The reactant was eluted with a mixed solution of dichloromethane and methanol, and the fractions were confirmed by LC/MS. The fractions containing the product were collected and concentrated in vacuo to remove the organic solvents, followed by vacuum-drying to obtain the linker intermediate (68) (162.9 mg, 0.39 mmol).
¹H NMR (400 MHz, Chloroform-d) δ=7.39 (s, 5H), 7.23 (s, 2H), 7.19 (s, 1H), 4.36 (s, 2H), 3.92 (d, J=8.5, 4H), 3.72 (s, 2H).
MS(ESI) m/z: z=1 402.29 [M+H]⁺

### (1-14-3) Synthesis of Modification Reagent (66)

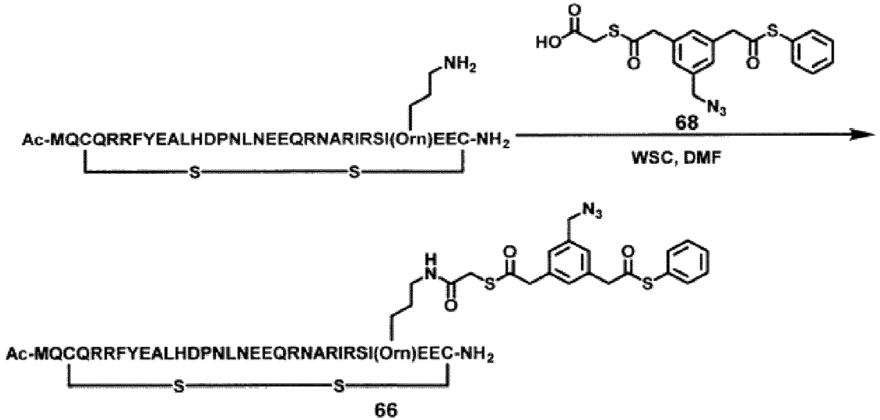

Each of the above amino acid sequences is the amino acid sequence of SEQ ID NO: 6.

Ac-MQCQRRFYEALHDPNLNEEQRNARIRSI(Orn)EEC-NH₂ (SEQ ID No. 6, 25.0 mg, 6.2 µl, with two cysteines at positions 3 and 32 each forming an intramolecular disulfide bond) and the Linker intermediates (68) (25.0 mg, 62.0 ul) were dissolved in DMF (1.00 mL), and WSC·HCl (11.9 mg, 62.0 µmol) was added and the mixture was stirred at room temperature for 2 hours. After the reaction was confirmed by LC-MS, the reaction solution was dissolved in a 0.05% aqueous trifluoroacetic acid solution and subjected to reverse-phase high-performance liquid chromatography using octadodecyl group-chemically bonded silica gel as a filler, and the reactant was eluted with a mixed solution of water and acetonitrile containing 0.05% trifluoroacetic acid, and the respective fractions were confirmed by LC-MS. Fractions containing the product were collected and concentrated in vacuo to remove acetonitrile, followed by freeze-drying to afford the modification reagent (66) (8.60 mg, 1.95 µmol).
MS(ESI) m/z: z=4 1101.70 [M+3H]³⁺

### [Example 2: Modification of anti-HER2 antibody trastuzumab using modification reagents having an affinity substance and analysis thereof]

### (2-1) Modification of Anti-HER2 Antibody Trastuzumab Using Modification Reagents having Affinity substances

### (2-1-1) Modification reaction with modification reagents (1, 6, 11)

A modification reaction of the anti-HER2 antibody trastuzumab was performed using the modification reagent (1) synthesized in Example (1-1-4). The modification reagent (1) was dissolved in DMF so as to be 30 mM. 500 µg of the anti-HER2 IgG antibody trastuzumab (Chugai Pharmaceutical Co., Ltd.) was dissolved in ammonium acetate buffer (pH 5.5), 6.88 µL of DMF (5.0 mg/mL) and 1.12 µL of 30 mM peptide reagent (10 equivalents to the antibody) were added, and the mixture was stirred at 37°C for 1 hour to obtain trastuzumab-peptide complex (69).

Antibody modification reaction was performed using the modification reagent (6) in a similar manner to obtain the trastuzumab-peptide complex (70).

Antibody modification reaction was performed using the modification reagent (11) in a similar manner to obtain the trastuzumab-peptide complex (71).

### (2-1-2) Modification reaction using the modification reagent (18)

A modification reaction of the anti-HER2 antibody trastuzumab was performed using the modification reagent (18) synthesized in Example (1-4-6). The modification reagent (18) was dissolved in DMF so as to be 10 mM. 500 µg of the anti-HER2 IgG antibody trastuzumab (Chugai Pharmaceutical Co., Ltd.) was dissolved in ammonium acetate buffer (pH 5.5), and 88 µL of DMF6. (5.0 mg/mL) and 3.3 µL of 10 mM peptide reagent (10 equivalents to the antibody) were added, and the mixture was stirred at room temperature for 1 hour to obtain trastuzumab-peptide complex (72).

### (2-1-3) Modification reactions with modification reagents (24, 33, 65, 66)

The modification reagent (24) synthesized in Example (1-5-9) was dissolved in dimethyl sulfoxide so as to be 20 mM. 500 µg of the anti-HER2 IgG antibody trastuzumab (Chugai Pharmaceutical Co., Ltd.) was dissolved in 100 µL of 0.1 M HEPES buffer (pH 8.0) (5.0 mg/mL) or 200 µL (2.5 mg/mL), and 1.69 µL of 20 mM peptide reagent (10 equivalents to the antibody) was added and the mixture was stirred at room temperature for 1 hour to obtain trastuzumab-peptide complex (73).

Antibody modification reaction was performed using the modification reagent (33) in a similar manner to obtain the trastuzumab-peptide complex (74).

Antibody modification reaction was performed using the modification reagent (65) in a similar manner to obtain the trastuzumab-peptide complex (75).

Antibody modification reaction was performed using the modification reagent (66) in a similar manner to obtain the trastuzumab-peptide complex (76).

### (2-1-4) Modification reactions with modification reagents (47, 53, 59, 61, 63, 64)

The modification reagent (47) synthesized in Example (1-7-6) was dissolved in DMF so as to be 20 mM. 500 µg of the anti-HER2 IgG antibody trastuzumab (Chugai Pharmaceutical Co., Ltd.) was dissolved in 100 µL of 50 mM HEPES buffer (pH 8.2) (5.0 mg/mL), and 1.69 µL of 20 mM peptide reagent (10 equivalents to the antibody) was added and the mixture was stirred at room temperature for 1 hour to obtain trastuzumab-peptide complex (77).

Antibody modification reaction was performed using the modification reagent (53) in a similar manner to obtain the trastuzumab-peptide complex (78).

Antibody modification reaction was performed using the modification reagent (59) in a similar manner to obtain the trastuzumab-peptide complex (79).

Antibody modification reaction was performed using the modification reagent (61) in a similar manner to obtain the trastuzumab-peptide complex (80).

Antibody modification reaction was performed using the modification reagent (63) in a similar manner to obtain the trastuzumab-peptide complex (81).

Antibody modification reaction was performed using the modification reagent (64) in a similar manner to obtain the trastuzumab-peptide complex (82).

### (2-2) Analysis by ESI-TOFMS

ESI-TOFMS analyses of trastuzumab-peptide complexes (69) were performed according to a published report (WO2019/240287A1). The raw material trastuzumab peak was observed at 148222. The reaction product had a peak at 152915 into which two modification reagents (1) were introduced. Subsequently, the peptide/antibody bonding ratio was confirmed by DAR calculator (Agilent Software, Inc.), and the results are shown in Table 1. The average peptide/antibody bonding ratio calculated from DAR peak and % Area in Table 1 was 1.9. Therefore, the production of an antibody intermediate (average peptide/antibody bonding ratio 1.9) represented by the following structural formula was confirmed.

**Table 1**

| DAR Peak | Observed mass(Da) | Peak Area | %Area |
|---|---|---|---|
| 1 | 150568.2 | 1.17E+005 | 12.3 |
| 2 | 152916.7 | 8.34E+005 | 87.7 |

Similarly, ESI-TOFMS analyses of the trastuzumab-peptide complex (70) were performed, and the reaction product was peaked at 152945 into which two modification reagents (6) were introduced. Subsequently, the peptide/antibody bonding ratio was confirmed by DAR calculator (Agilent Software, Inc.), and the results are shown in Table 2. The average peptide/antibody bonding ratio calculated from DAR peak and % Area in Table 2 was 1.7. Therefore, the production of an antibody intermediate (average peptide/antibody bonding ratio 1.7) represented by the following structural formula was confirmed.

**Table 2**

| DAR Peak | Observed mass(Da) | Peak Area | %Area |
|---|---|---|---|
| 0 | 148219.5 | 1.01E+004 | 1.1 |
| 1 | 150581.7 | 2.52E+005 | 27.3 |
| 2 | 152944.0 | 6.62E+005 | 71.6 |

Similarly, ESI-TOFMS analyses of the trastuzumab-peptide complex (71) were performed, and the product was peaked at 152973 into which two modification reagents (11) were introduced. Subsequently, the peptide/antibody bonding ratio was confirmed by DAR calculator (Agilent Software, Inc.), and the results are shown in Table 3. The average peptide/antibody bonding ratio calculated from DAR peak and % Area in Table 3 was 1.8. Therefore, the production of an antibody intermediate (average peptide/antibody bonding ratio 1.8) represented by the following structural formula was confirmed.

**Table 3**

| DAR Peak | Observed mass(Da) | Peak Area | %Area |
|---|---|---|---|
| 1 | 150597.2 | 2.12E+005 | 20.8 |
| 2 | 152972.5 | 8.05E+005 | 79.2 |

Similarly, ESI-TOFMS analyses of the trastuzumab-peptide complex (72) were performed, and the product was peaked at 153023 into which two modification reagents (18) were introduced. Subsequently, the peptide/antibody bonding ratio was confirmed by DAR calculator (Agilent Software, Inc.), and the results are shown in Table 4. The average peptide/antibody bonding ratio calculated from DAR peak and % Area in Table 4 was 1.8. Therefore, the production of an antibody intermediate (average peptide/antibody bonding ratio 1.8) represented by the following structural formula was confirmed.

**Table 4**

| DAR Peak | Observed mass(Da) | Peak Area | %Area |
|---|---|---|---|
| 1 | 150875.3 | 1.22E+005 | 20.6 |
| 2 | 153021.75 | 4.70E+005 | 79.4 |

Similarly, ESI-TOFMS analyses of the trastuzumab-peptide complex (73) were performed, and the product was peaked at 157267 into which two modification reagents (24) were introduced. Subsequently, the peptide/antibody bonding ratio was confirmed by DAR calculator (Agilent Software, Inc.), and the results are shown in Table 5. The average peptide/antibody bonding ratio calculated from DAR peak and % Area in Table 5 was 1.7. Therefore, the production of an antibody intermediate (average peptide/antibody bonding ratio 1.7) represented by the following structural formula was confirmed.

**Table 5**

| DAR Peak | Observed mass(Da) | Peak Area | %Area |
|---|---|---|---|
| 1 | 152743.1 | 2.19E+005 | 25.5 |
| 2 | 157267.4 | 6.40E+005 | 74.5 |

Similarly, ESI-TOFMS analyses of the trastuzumab-peptide complex (74) were performed, and the product was peaked at 157619 into which two modification reagents (33) were introduced. Subsequently, the peptide/antibody bonding ratio was confirmed by DAR calculator (Agilent Software, Inc.), and the results are shown in Table 6. The average peptide/antibody bonding ratio calculated from DAR peak and % Area in Table 6 was 1.5. Therefore, the production of an antibody intermediate (average peptide/antibody bonding ratio 1.5) represented by the following structural formula was confirmed.

**Table 6**

| DAR Peak | Observed mass(Da) | Peak Area | %Area |
|---|---|---|---|
| 0 | 148222.7 | 1.40E+004 | 5.6 |
| 1 | 152921.0 | 1.18E+005 | 47.0 |
| 2 | 157618.7 | 1.19E+005 | 47.5 |
| 3 | 157618.7 | 1.50E+004 | 5.7 |

Similarly, ESI-TOFMS analyses of the trastuzumab-peptide complex (75) were performed, and the product was peaked at 161051 into which two modification reagents (65) were introduced. Subsequently, the peptide/antibody bonding ratio was confirmed by DAR calculator (Agilent Software, Inc.), and the results are shown in Table 7. The average peptide/antibody bonding ratio calculated from DAR peak and % Area in Table 7 was 2.0. Therefore, the production of an antibody intermediate (average peptide/antibody bonding ratio 2.0) represented by the following structural formula was confirmed.

**Table 7**

| DAR Peak | Observed mass(Da) | Peak Area | %Area |
|---|---|---|---|
| 1 | 152497.4 | 2.43E+004 | 3.6 |
| 2 | 156772.2 | 6.43E+005 | 96.4 |
| 3 | 161046.8 | 1.03E+005 | 1.5 |

Similarly, ESI-TOFMS analyses of the trastuzumab-peptide complex (76) were performed, and the product was peaked at 156802 into which two modification reagents (66) were introduced. Subsequently, the peptide/antibody bonding ratio was confirmed by DAR calculator (Agilent Software, Inc.), and the results are shown in Table 8. The average peptide/antibody bonding ratio calculated from DAR peak and % Area in Table 8 was 1.5. Therefore, the production of an antibody intermediate (average peptide/antibody bonding ratio 1.5) represented by the following structural formula was confirmed.

**Table 8**

| DAR Peak | Observed mass(Da) | Peak Area | %Area |
|---|---|---|---|
| 1 | 148218.8 | 1.56E+004 | 3.5 |
| 2 | 152509.0 | 1.72E+005 | 38.8 |
| 3 | 156799.6 | 2.55E+005 | 57.7 |

Similarly, ESI-TOFMS analyses of the trastuzumab-peptide complex (77) were performed, and the product was peaked at 157567 into which two modification reagents (47) were introduced. Subsequently, the peptide/antibody bonding ratio was confirmed by DAR calculator (Agilent Software, Inc.), and the results are shown in Table 9. The average peptide/antibody bonding ratio calculated from DAR peak and % Area in Table 9 was 2.0. Therefore, the production of an antibody intermediate (average peptide/antibody bonding ratio 2.0) represented by the following structural formula was confirmed.

**Table 9**

| DAR Peak | Observed mass(Da) | Peak Area | %Area |
|---|---|---|---|
| 0 | 148496.65 | 2.33E+003 | 0.3 |
| 1 | 153047.95 | 1.41E+004 | 1.9 |
| 2 | 157563.95 | 6.70E+005 | 90.5 |
| 3 | 162405.95 | 5.42E+004 | 7.3 |

Similarly, ESI-TOFMS analyses of the trastuzumab-peptide complex (78) were performed, and the product was peaked at 157540 into which two modification reagents (53) were introduced. Subsequently, the peptide/antibody bonding ratio was confirmed by DAR calculator (Agilent Software, Inc.), and the results are shown in Table 10. The average peptide/antibody bonding ratio calculated from DAR peak and % Area in Table 10 was 2.0. Therefore, the production of an antibody intermediate (average peptide/antibody bonding ratio 2.0) represented by the following structural formula was confirmed.

**Table 10**

| DAR Peak | Observed mass(Da) | Peak Area | %Area |
|---|---|---|---|
| 0 | 153043.55 | 6.37E+004 | 5.0 |
| 1 | 157538.5 | 1.16E+006 | 91.3 |
| 2 | 162201.65 | 4.68E+004 | 3.7 |

Similarly, ESI-TOFMS analyses of the trastuzumab-peptide complex (79) were performed, and the product was peaked at 157563 into which two modification reagents (59) were introduced. Subsequently, the peptide/antibody bonding ratio was confirmed by DAR calculator (Agilent Software, Inc.), and the results are shown in Table 11. The average peptide/antibody bonding ratio calculated from DAR peak and % Area in Table 11 was 1.6. Therefore, the production of an antibody intermediate (average peptide/antibody bonding ratio 1.6) represented by the following structural formula was confirmed.

**Table 11**

| DAR Peak | Observed mass(Da) | Peak Area | %Area |
|---|---|---|---|
| 0 | 148223.05 | 1.17E+004 | 2.1 |
| 1 | 152891.95 | 1.98E+005 | 35.3 |
| 2 | 157559.55 | 3.43E+005 | 61.2 |
| 3 | 162228.2 | 7.86E+003 | 1.4 |

Similarly, ESI-TOFMS analyses of the trastuzumab-peptide complex (80) were performed, and the product was peaked at 157543 into which two modification reagents (61) were introduced. Subsequently, the peptide/antibody bonding ratio was confirmed by DAR calculator (Agilent Software, Inc.), and the results are shown in Table 12. The average peptide/antibody bonding ratio calculated from DAR peak and % Area in Table 12 was 1.6. Therefore, the production of an antibody intermediate (average peptide/antibody bonding ratio 1.6) represented by the following structural formula was confirmed.

**Table 12**

| DAR Peak | Observed mass(Da) | Peak Area | %Area |
|---|---|---|---|
| 1 | 148384.45 | 1.14E+002 | 1.6 |
| 2 | 152880.25 | 2.56E+003 | 35.2 |
| 3 | 157537.05 | 4.60E+003 | 63.2 |

Similarly, ESI-TOFMS analyses of the trastuzumab-peptide complex (81) were performed, and the product was peaked at 152784 into which two modification reagents (63) were introduced. Subsequently, the peptide/antibody bonding ratio was confirmed by DAR calculator (Agilent Software, Inc.), and the results are shown in Table 13. The average peptide/antibody bonding ratio calculated from DAR peak and % Area in Table 13 was 2.0. Therefore, the production of an antibody intermediate (average peptide/antibody bonding ratio 2.0) represented by the following structural formula was confirmed.

**Table 13**

| DAR Peak | Observed mass(Da) | Peak Area | %Area |
|---|---|---|---|
| 1 | 153180.75 | 1.26E+004 | 1.4 |
| 2 | 157781.6 | 8.32E+005 | 91.7 |
| 3 | 162319 | 6.27E+004 | 6.9 |

Similarly, ESI-TOFMS analyses of trastuzumab-peptide complex (82) were performed, and the product was peaked at 157596 into which two modification reagents (64) were introduced. Subsequently, the peptide/antibody bonding ratio was confirmed by DAR calculator (Agilent Software, Inc.), and the results are shown in Table 14. The average peptide/antibody bonding ratio calculated from DAR peak and % Area in Table 14 was 2.0. Therefore, the production of an antibody intermediate (average peptide/antibody bonding ratio 2.0) represented by the following structural formula was confirmed.

**Table 14**

| DAR Peak | Observed mass(Da) | Peak Area | %Area |
|---|---|---|---|
| 1 | 153160.25 | 2.21E+003 | 0.2 |
| 2 | 157594.6 | 9.62E+005 | 96.7 |
| 3 | 162439.6 | 3.05E+004 | 3.0 |

### (2-3) Confirmation of heavy chain selectivity by ESI-TOFMS under reducing condition

ESI-TOFMS analyses of trastuzumab-peptide complexes (69) under reducing condition were performed according to a published report (WO2019/240287A1). It was observed that the raw material trastuzumab had heavy chain peaks at 50594 and 50755 and light chain peaks at 23439, and the reaction product was identified as 52917 and 53079 where a linker(s) was introduced into the heavy chain, and 23439 which was the same as the raw material in the light chain.

Similarly, ESI-TOFMS analyses of the trastuzumab-peptide complex (70) were performed, and the reaction product was identified as 52956 and 53118 where a linker(s) was introduced into the heavy chain, and 23439 which was the same as the raw material in the light chain.

Similarly, ESI-TOFMS analyses of the trastuzumab-peptide complex (71) were performed, and the reaction product was identified as 52971 and 53133 where a linker(s) was introduced into the heavy chain, and 23439 which was the same as the raw material in the light chain.

Similarly, ESI-TOFMS analyses of the trastuzumab-peptide complex (72) were performed, and the reaction product was identified as 52996 and 53157 where a linker(s) was introduced into the heavy chain, and 23439 which was the same as the raw material in the light chain.

Similarly, ESI-TOFMS analyses of the trastuzumab-peptide complex (73) were performed, and the reaction product was identified as 55091 and 55253 where a linker(s) was introduced into the heavy chain, and 23439 which was the same as the raw material in the light chain.

Similarly, ESI-TOFMS analyses of the trastuzumab-peptide complex (75) were performed, and the reaction product was identified as 54849 and 55010 where a linker(s) was introduced into the heavy chain, and 23439 which was the same as the raw material in the light chain.

Similarly, ESI-TOFMS analyses of the trastuzumab-peptide complex (76) were performed, and the reaction product was identified as 54888 and 55050 where a linker(s) was introduced into the heavy chain, and 23439 which was the same as the raw material in the light chain.

Similarly, ESI-TOFMS analyses of the trastuzumab-peptide complex (77) were performed, and the reaction product was identified as 55268 and 55430 where a linker(s) was introduced into the heavy chain, and 23439 which was the same as the raw material in the light chain.

Similarly, ESI-TOFMS analyses of the trastuzumab-peptide complex (78) were performed and the reaction product was identified as 55254 and 55416 where a linker(s) was introduced into the heavy chain, and 23439 which was the same as the raw material in the light chain.

Similarly, ESI-TOFMS analyses of the trastuzumab-peptide complex (81) were performed, and the reaction product was identified as 55296 and 55458 where a linker(s) was introduced into the heavy chain, and 23439 which was the same as the raw material in the light chain.

Similarly, ESI-TOFMS analyses of the trastuzumab-peptide complex (82) were performed, and the reaction product was identified as 55281 and 55443 where a linker(s) was introduced into the heavy chain, and 23439 which was the same as the raw material in the light chain.

[Example 3: Linker cleavage of trastuzumab-affinity substance complex and analysis by ESI-TOFMS]

### (3-1) Linker cleavage reaction of the trastuzumab-affinity substance complex

Trastuzumab-peptide complex (69) was used to perform linker cleavage using hydroxyamine hydrochloride according to a published report (WO2019/240287A1) to obtain azide-introduced antibody (83).

In a similar manner, the linker cleavage reaction of the trastuzumab-peptide complex (70) was performed using hydroxyamine hydrochloride to obtain the azide-introduced antibody (84).

In a similar manner, the linker cleavage reaction of the trastuzumab-peptide complex (71) was performed using hydroxyamine hydrochloride to obtain the azide-introduced antibody (85).

In a similar manner, the linker cleavage reaction of the trastuzumab-peptide complex (72) was performed using hydroxyamine hydrochloride to obtain the azide-introduced antibody (86).

In a similar manner, the linker cleavage reaction of the trastuzumab-peptide complex (73) was performed using hydroxyamine hydrochloride to obtain the azide-introduced antibody (87).

In a similar manner, the linker cleavage reaction of the trastuzumab-peptide complex (74) was performed using hydroxyamine hydrochloride to obtain the azide-introduced antibody (88).

In a similar manner, the linker cleavage reaction of the trastuzumab-peptide complex (75) was performed using hydroxyamine hydrochloride to obtain the azide-introduced antibody (87).

In a similar manner, the linker cleavage reaction of the trastuzumab-peptide complex (76) was performed using hydroxyamine hydrochloride to obtain the azide-introduced antibody (89).

In a similar manner, the linker cleavage reaction of the trastuzumab-peptide complex (77) was performed using hydroxyamine hydrochloride to obtain the azide-introduced antibody (90).

In a similar manner, the linker cleavage reaction of the trastuzumab-peptide complex (78) was performed using hydroxyamine hydrochloride to obtain the azide-introduced antibody (90).

In a similar manner, the linker cleavage reaction of the trastuzumab-peptide complex (79) was performed using hydroxyamine hydrochloride to obtain the azide-introduced antibody (90).

In a similar manner, the linker cleavage reaction of the trastuzumab-peptide complex (80) was performed using hydroxyamine hydrochloride to obtain the azide-introduced antibody (90).

In a similar manner, the linker cleavage reaction of the trastuzumab-peptide complex (81) was performed using hydroxyamine hydrochloride to obtain the azide-introduced antibody (90).

In a similar manner, the linker cleavage reaction of the trastuzumab-peptide complex (82) was performed using hydroxyamine hydrochloride to obtain the azide-introduced antibody (90).

In a similar manner, the linker cleavage reaction of the trastuzumab-peptide complex (72) was performed using methoxyamine hydrochloride to obtain the azide-introduced antibody (91).

In a similar manner, the linker cleavage reaction of the trastuzumab-peptide complex (77) was performed using methoxyamine hydrochloride to obtain the azide-introduced antibody (92).

In a similar manner, the linker cleavage reaction of the trastuzumab-peptide complex (78) was performed using methoxyamine hydrochloride to obtain the azide-introduced antibody (92).

In a similar manner, the linker cleavage reaction of the trastuzumab-peptide complex (79) was performed using methoxyamine hydrochloride to obtain the azide-introduced antibody (92).

In a similar manner, the linker cleavage reaction of the trastuzumab-peptide complex (80) was performed using methoxyamine hydrochloride to obtain the azide-introduced antibody (92).

In a similar manner, the linker cleavage reaction of the trastuzumab-peptide complex (81) was performed using methoxyamine hydrochloride to obtain the azide-introduced antibody (92).

In a similar manner, the linker cleavage reaction of the trastuzumab-peptide complex (82) was performed using methoxyamine hydrochloride to obtain the azide-introduced antibody (92).

### (3-2) Analysis by ESI-TOFMS

ESI-TOFMS analyses of the azide-transfected antibody (84) were performed according to a published report (WO2019/240287A1) and a peak was confirmed at 148657 where the linker was cleaved.

Similarly, ESI-TOFMS of the azide-introduced antibody (85) was analyzed and a peak was confirmed at 148712 where the linker was cleaved.

Similarly, ESI-TOFMS of the azide-transfected antibody (86) was analyzed and a peak was confirmed at 148755 at which the linker cleavage proceeded.

Similarly, ESI-TOFMS of the azide-transfected antibody (87) was analyzed and a peak was confirmed at 148679 at which the linker cleavage proceeded.

Similarly, ESI-TOFMS of the azide-transfected antibody (89) was analyzed and a peak was confirmed at 148706 at which the linker cleavage proceeded.

Similarly, ESI-TOFMS of the azide-transfected antibody (90) was analyzed and a peak was confirmed at 148775 at which the linker cleavage proceeded.

Similarly, ESI-TOFMS of the azide-introduced antibody (91) was analyzed and a peak was confirmed at 148779 at which the linker cleavage proceeded.

Similarly, ESI-TOFMS of the azide-introduced antibody (92) was analyzed and a peak was confirmed at 148790 at which the linker cleavage proceeded.

### [Example 4: Conjugation of trastuzumab introduced with azide regioselectively and any compound, and ESI-TOFMS analysis of the product]

### (4-1) Conjugation of azide-introduced antibody with fluorescent substance

In accordance with published reports (WO2019/240287A1 and WO2019/240288A1), carboxyrhodamine 110-PEG4-DBCO (Broadpharm) was added to the azide-introduced antibody (83) and ADC mimic (93) was obtained.

A ADC mimic (94) was obtained from the azide-introduced antibody (84) in a similar manner.

An ADC mimic (95) was obtained from the azide-introduced antibody (85) in a similar manner.

An ADC mimic (96) was obtained from the azide-introduced antibody (86) in a similar manner.

An ADC mimic (97) was obtained from the azide-introduced antibody (87) in a similar manner.

An ADC mimic (98) was obtained from the azide-introduced antibody (88) in a similar manner.

An ADC mimic (99) was obtained from the azide-introduced antibody (89) in a similar manner.

An ADC mimic (100) was obtained from the azide-introduced antibody (90) in a similar manner.

An ADC mimic (101) was obtained from the azide-introduced antibody (91) in a similar manner.

An ADC mimic (102) was obtained from the azide-introduced antibody (92) in a similar manner.

An ADC (103) was obtained by reacting the azide-introduced antibody (83) with DBCO-MMAE (Abzena) in a similar manner.

An ADC (104) was obtained by reacting the azide-introduced antibody (86) with DBCO-MMAE (Abzena) in a similar manner.

An ADC (105) was obtained by reacting the azide-introduced antibody (91) with DBCO-MMAE (Abzena) in a similar manner.

From the above, it was confirmed that the compound or salt thereof of the present invention is useful for producing an antibody-drug conjugate showing excellent DAR.

### (Reference Example 1) Synthesis of Modification Reagent (106) and Derivatization to ADC mimic (107)

The modification reagent (106) was synthesized as follows and reacted with the anti-HER2 antibody trastuzumab according to Example 2 followed by cleavage of the linker according to Example 3 and reaction with carboxyrhodamine 110-PEG4-DBCO (Broadpharm) was performed according to Example 4 to give an ADC mimic (107). 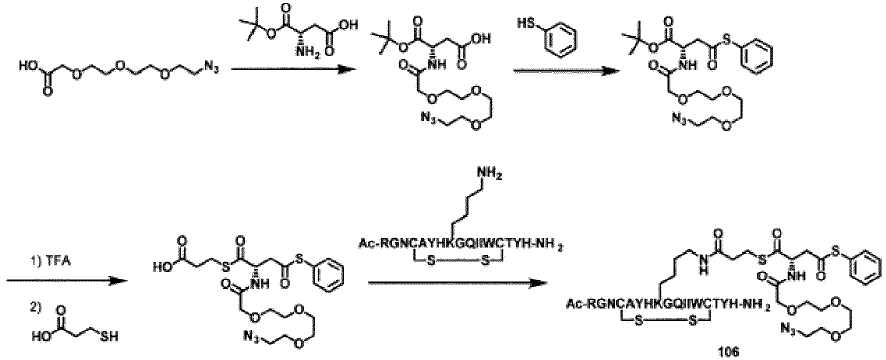 Each of the above amino acid sequences is the amino acid sequence of SEQ ID NO: 2.

### (Reference Example 2) Synthesis of Modification reagent (108) and Derivatization to ADC mimic (109)

The modification reagent (108) was synthesized as follows and reacted with the anti-HER2 antibody trastuzumab according to Example 2 followed by cleavage of the linker according to Example 3 and reaction with carboxyrhodamine 110-PEG4-DBCO (Broadpharm) was performed according to Example 4 to give an ADC mimic (109). 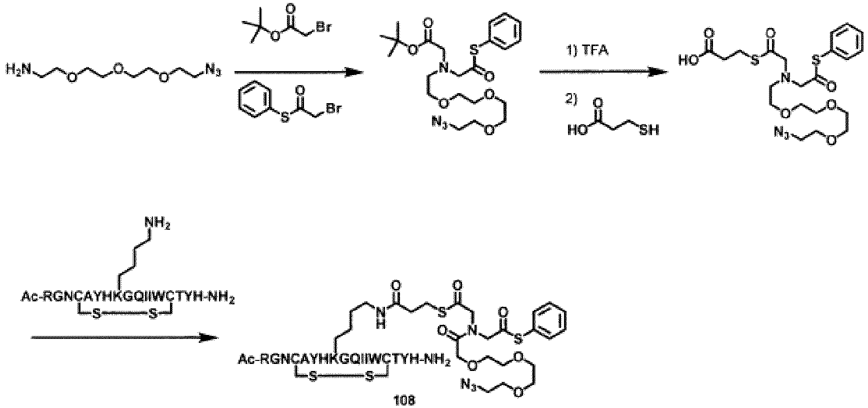 Each of the above amino acid sequences is the amino acid sequence of SEQ ID NO: 2.

### (Reference Example 3) Modification reaction using azide antibody (111) and analysis thereof

An ADC mimic (112) was obtained by reacting the following azide-antibody (111) described in WO2019/240287A1 with carboxyrhodamine 110-PEG4-DBCO (Broadpharm) according to Example 4. In addition, an ADC (113) was obtained by reacting with DBCO-MMAE (Abzena) in a similar manner.

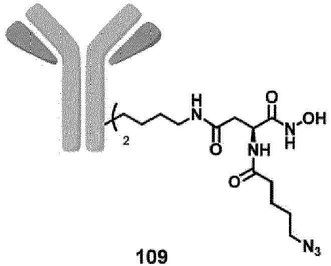

### [Example 5: Assessment of ADC mimic by stability test using rat plasma]

Blood stability of various ADC mimics was assessed by analyzing the amounts of fluorescent molecules that were reduced from ADC mimic when ADC mimic was incubated in rat blood as described below.

### (5-1) Study of Stability in Plasma

Sterile filtration was performed on 700 µL of rat plasma (Charles River) after ADC mimic was added to it so as to be 0.1 mg/mL. The solution was dispensed into six Eppen tubes in 50 µL portions. Three of the six samples were stored in an incubator set at 37°C for 4 days. The remaining three were also stored in a -80°C freezer for 4 days. 100 µL of acetonitrile was added to each sample, and the mixture was vortexed and centrifuged to obtain a precipitate. The resulting supernatant was collected and analyzed by HPLC.

### (5-2) Analysis of Amount of Reduced Fluorescent Molecules Using HPLC Spectrometry

The determination was made using a liquid chromatography/fluorescence detection method to determine the amount of fluorescence molecules that had eliminated from ADC mimic. Three samples stored in the freezer in Example 5-1 corresponded to Day=0, three samples stored at 37°C in Example 5-1 corresponded to Day=4, and the difference in the fluorescent intensities of Day=4 and Day=0 was analyzed.

The results were evaluated as follows. By comparing the fluorescent intensities of Example compounds using the ADC mimic synthesized in Example 16(1) as a control, the rate of increase was calculated using the following equation. A lower rate of increase indicates higher stability in plasma.

Increase in fluorescence intensity of fluorescent molecules eliminated from Example ADC mimic = [(Day=4 fluorescence intensity) - (Day=0 fluorescence intensity)].

Increase in fluorescence intensity of fluorescent molecules eliminated from Control ADC mimic = [(Day=4 fluorescence intensity) - (Day=0 fluorescence intensity)].

Carboxyrhodamine 110-PEG4-DBCO was separately used to calculate the colleration between area-area of fluorescent intensity and concentration for HPLC. An increase in the fluorescent intensity of the respective ADC mimic was converted into a concentration by using the calculation equation. When the concentration of Day 0 was taken as 100%, the percentage of the concentration of the above increase was calculated as the elimination rate.

**Table 15. Results of stability test in plasma using ADC mimic (1)**

| ADC mimic number | Conjugation site | Structure of azide precursor | Elimination rate of fluorescence molecule at Day=4 |
|---|---|---|---|
| 111 | Lys246/248 | 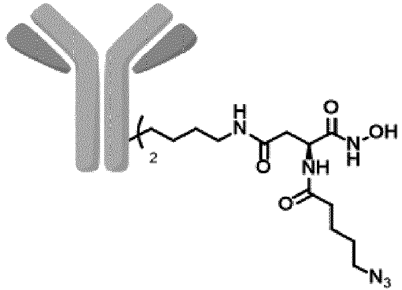 | 21% |
| 93 | Lys246/248 | 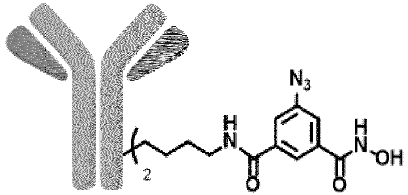 | <3% |
| 94 | Lys246/248 | 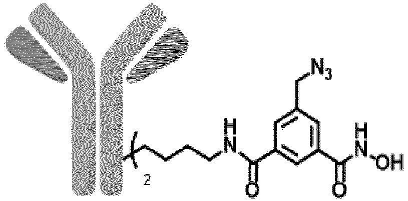 | <3% |
| 95 | Lys246/248 | 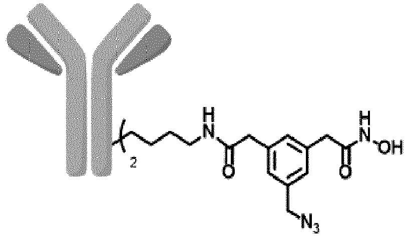 | <3% |
| 96 | Lys246/248 | 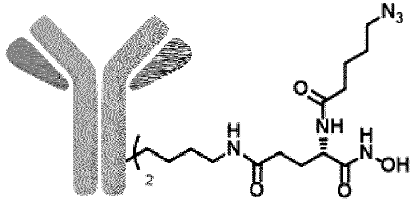 | <3% |

**Table 16. Results of stability test in plasma using ADC mimic (2)**

| ADC mimic number | Conjugation site | Structure of azide precursor | Elimination rate of fluorescence molecule at Day=4 |
|---|---|---|---|
| 101 | Lys246/248 | 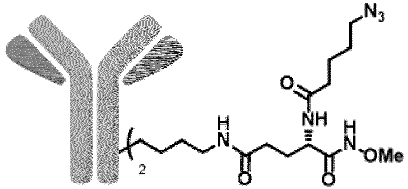 | <3% |
| 97 | Lys288/290 | 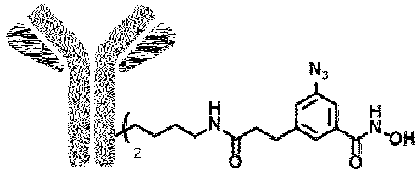 | <3% |
| 98 | Lys288/290 | 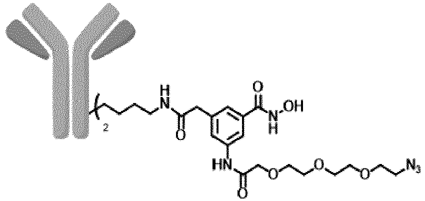 | <3% |
| 99 | Lys288/290 | 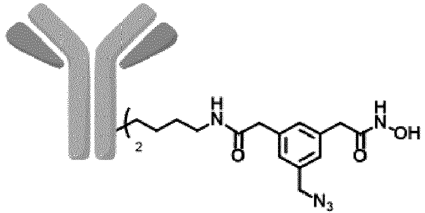 | <3% |
| 100 | Lys288/290 | 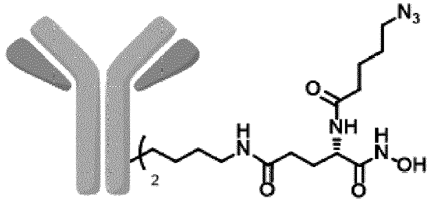 | <3% |

**Table 17. Results of stability test in plasma using ADC mimic (3)**

| ADC mimic number | Conjugation site | Structure of azide precursor | Elimination rate of fluorescenc e molecule at Day=4 |
|---|---|---|---|
| 102 | Lys288/290 | 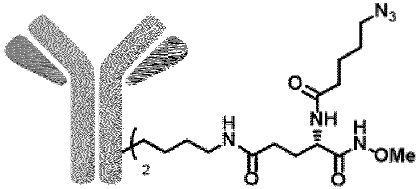 | <3% |
| 107 (Conparative Example 1) | Lys246/248 | 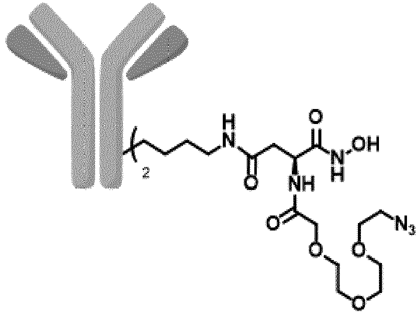 | 20% |
| 109 (Conparative Example 2) | Lys246/248 | 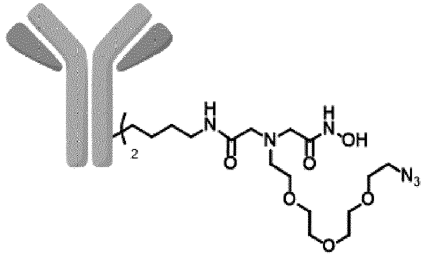 | 16% |

Comparative Example 1 (107) and Comparative Example 2 (109) do not correspond to M (a trivalent group in which a carbon atom in C=O adjacent to M and a carbon atom in C=W are linked by a main chain portion having 3 to 5 carbon atoms). This is because Comparative Examples 1 and 2 are for linking with a main chain portion having 2 carbon atoms (Comparative Example 1), and a main chain portion having 2 carbon atoms and 1 nitrogen atom (Comparative Example 2), instead of having 3 to 5 carbon atoms.

Consequently, ADC mimic synthesized in Example 3 is stable. ADC mimic synthesized in Comparative Examples 1 and 2 were unstable (Tables 15 to 17).

### [Example 6: Assessment of ADC by a stability-test using rat plasma]

Blood stabilization of various ADC was performed according to Example 5.

### (6-1) Study of Stability in Plasma

The ADC synthesized in Example 4-1 and Comparative Example 2 were incubated with rat plasma in a similar manner as in Example 5-1, and three samples stored in a freezer and three samples stored at 37°C were prepared for each ADC.

### (6-2) Analysis of amount of eliminated payloads using HPLC spectrometry

Determinations were made using liquid chromatography mass spectrometry (including tandem mass spectrometry) to determine the amount of eliminated payload. Three samples stored in the freezer in Example 6-1 coreresponded to Day=0, three samples stored at 37 °C in Example 6-1 corresponded to Day=4, and MS intensities of the detected payload of Day=4 and Day=0 was calculated by the extracted ion chromatogram, respectively, and the difference was analyzed.

The ADCs (103) to (105) synthesized in Example 4 were more stable than ADC (112) synthesized from the azide-antibody (109) described in a previous report (WO2019/240287A1), and was similar to the comparative examination of ADC mimic of Example 5.

### [Example 6: Confirmation of regioselectivity of azide-introduced antibody]

The azide-introduced trastuzumab obtained in (4-1) was subjected to peptide mapping in the following steps.

### (6-1) Peptide Mapping of Azide-Introduced Trastuzumab (86)

Peptide-mapping of the azide-introduced trastuzumab (86) was performed according to a published report (WO2019/240288A). Analysis using LC-MS/MS revealed a peptide consisting of 33 amino acid residues including azide-modification to lysine residues by tryptic digestion of trastuzumab, which is the peptide fragment of THTCPPCPAPELLGGPSVFLFPPKPKDTLMISR. Analysis by BioPharma Finder also showed that the modification to the lysine residue at position 246 or 248 in EU numbering occurred highly selectively.

The azide-introduced trastuzumab (86) obtained in (4-1) above was found to be regioselectively conjugating to Lys246 and Lys248 of the heavy chain of the antibody according to EU numbering.

### (6-2) Peptide Mapping of Trastuzumab Azide Introducer (91)

Peptide mapping of the azide-introduced trastuzumab (91) obtained in (4-1) was performed in a similar manner as in (6-1). Analysis using LC-MS/MS revealed a peptide consisting of 33 amino acid residues including azide-modification to lysine residues by tryptic digestion of trastuzumab, which is the peptide fragment of THTCPPCPAPELLGGPSVFLFPPKPKDTLMISR. Analysis by BioPharma Finder also showed that the modification to the lysine residue at position 246 or 248 in EU numbering occurred highly selectively.

### (6-3) Peptide Mapping of Trastuzumab Azide Introducer (90)

Peptide-mapping of the azide-introduced trastuzumab (90) was performed according to a published report (WO2019/240288A). Analysis using LC-MS/MS revealed a peptide consisting of 18 amino acid residues including azide-modification to lysine residues by tryptic digestion of trastuzumab, which is the peptide fragment of FNWYVDGVEVHNAKTKPR. Analysis by BioPharma Finder also showed that the modification to the lysine residue at position 288 or 290 in EU numbering occurred highly selectively.

The azide-introduced trastuzumab (90) obtained in (4-1) described above showed that conjugation proceeded regioselectively at Lys288 and Lys290 of the heavy chain of the antibody according to EU numbering.

### (6-4) Peptide Mapping of Trastuzumab Azide Introducer (92)

Peptide mapping of the azide-introduced trastuzumab (92) obtained in (4-1) was performed in a similar manner as in (6-1). Analysis using LC-MS/MS revealed a peptide consisting of 18 amino acid residues including azido-modification to lysine residues by tryptic digestion of trastuzumab, which is the peptide fragment of FNWYVDGVEVHNAKTKPR. Analysis by BioPharma Finder also showed that the modification to the lysine residue at position 288 or 290 in EU numbering occurred highly selectively.

The azide-introduced trastuzumab (92) obtained in (4-1) described above was found to be regioselectively conjugated to Lys288 and Lys290 of the heavy chain of the antibody according to EU numbering.

### EXAMPLE 7: Regioselective Modification of Differential Target Regions of IgG1 Fc by IgG1 Fc Affinity Peptide Reagents, and Synthesis of Antibody-Drug Conjugates

### (7-1) Preparation of Azide group-Introduced Antibody Derivatives by Regioselective Modification of Anti-HER2 Antibody Trastuzumab Subsequent to Cleavage of Thioester Group

The azide-introduced antibody (91) synthesized in Example (3-1) was replaced with 50 mM HEPES buffer (pH 8.2). To this solution, 10 equivalents of the modification reagent (64) synthesized in Example (1-14) relative to the antibody was added, and the mixture was stirred at room temperature for 3 hours. The reaction solution was replaced with 20 mM ammonium acetate buffer. For the obtained antibody, Mass was measured by ESI-TOFMS. As a result, the peak was confirmed at 158,319 in which two binding peptides were introduced to the azide-introduced antibody synthesized in (3-1).

From the above, it was confirmed that the antibody obtained in Example (7-1) contained modifications of the lysine residue at position 246 or 248 and the lysine residue at position 288 or 290 of the two heavy chains of the antibody according to EU numbering.

### (7-2) Preparation of Azide Group-Introduced Antibody Derivatives by Regioselective Modification of Anti-HER2 Antibody Trastuzumab Subsequent to Cleavage of Thioester Group

The linker cleavage reaction of the antibody-peptide complex obtained in Example (7-1) was performed by the method of Example (3-1) using methoxyamine hydrochloride to obtain an azide-introduced antibody. ESI-TOFMS analyses of the obtained azide antibody were performed. As a result, the linker cleavage proceeded, and the peak was confirmed at 149,350 in which four azide groups were introduced to the antibody.

From the above, it was confirmed that the antibody obtained in this Example (7-2) contained four azido groups (azido group/antibody bonding ratio=4) introduced into the side chain of the lysine residue at position 246 or 248 and the lysine residue at position 288 or 290 of two heavy chains of the antibody according to EU numbering.

### (7-3) Synthesis of DAR=4 ADC

In a similar manner as in Example (4-1), the azide-introduced antibody obtained in Example (7-2) was reacted with DBCO-MMAE (Abzena) to obtain an ADC.

Therefore, it was confirmed that the antibody obtained in Example (7-2) contained four drugs (DAR=4) introduced into the side chain of the lysine residue at position 246 or 248 and the lysine residue at position 288 or 290 of two heavy chains of the antibody according to EU numbering.

[Sequence Listing]

## Claims

1. A compound or salt thereof, the compound being represented by the following formula (I): 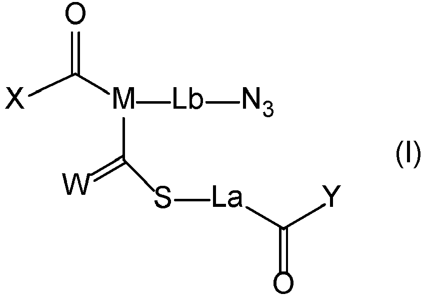 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

2. The compound or salt thereof accordng to claim 1, wherein the leaving group is selected from:
(a) R-S wherein R indicates a hydrogen atom, a monovalent hydrocarbon group which may have a substituent, or a monovalent heterocyclic group which may have a substituent, and S indicates a sulfur atom;
(b) R-O wherein R indicates a hydrogen atom, a monovalent hydrocarbon group which may have a substituent, or a monovalent heterocyclic group which may have a substituent, and O indicates an oxygen atom; or
(c) R_{A}-(R_{B})-N wherein R_{A} and R_{B} each independently represent a hydrogen atom, a monovalent hydrocarbon group which may have a substituent, or a monovalent heterocyclic group which may have a substituent, and N indicates a nitrogen atom; or
(d) a halogen atom.

3. The compound or salt according to claim 1 or 2, wherein the immunoglobulin unit is a human immunoglobulin unit.

4. The compound or salt thereof according to any one of claims 1-3, wherein the immunoglobulin unit is a human IgG.

5. The compound or salt thereof according to any one of claims 1-4, wherein the main chain portion consisting of 3 to 5 carbon atoms in M is a portion comprising a linear alkylene, or a ring-constituting carbon atom, or a combination thereof.

6. The compound or salt thereof according to any one of claims 1-5, wherein the carbonyl group adjacent to La forms an amide bond with an amino group in a side chain of a lysine residue in the affinity peptide.

7. The compound or salt thereof according to any one of claims 1-6, wherein the affinity peptide comprises the amino acid sequence of the following (A):
(A) (X0-3)a-C-Xaa1-Xaa2-Xaa3-Xaa4-Xaa5-Xaa6-I-I-W-C-(X0-3)b (SEQ ID NO: 1). wherein
(X₀₋₃)ₐ is none, or one to three consecutive arbitrary amino acid residues (other than lysine residue and cysteine residue) which are the same or different,
(X₀₋₃)_{b} is none, or one to three consecutive arbitrary amino acid residues (other than lysine residue and cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa 4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue,
Xaa6 is a glutamine residue, a glutamic acid residue, an asparagine residue, an aspartic acid residue, a proline residue, a glycine residue, an arginine residue, a phenylalanine residue, or a histidine residue.

8. The compound or salt thereof according to claim 7, wherein the affinity peptide comprising the amino acid sequence of (A) comprises the amino acid sequence of the following (1):
(1) RGNCAYHKGQIIWCTYH (SEQ ID NO: 2).

9. The compound or salt thereof according to any one of claims 1-6, wherein the affinity peptide comprises the amino acid sequence of the following (B):
(B) PNLNEEQRNARIRSI (SEQ ID NO: 3).

10. The compound or salt according to claim 9, wherein the affinity peptide comprising the amino acid sequence of (B) comprises an amino acid sequence selected from the group consisting of the following (1)-(3):
(1) FNMQCQRRFYEALHDPNLNEEQRNARIRSIKDDC (SEQ ID NO: 4);
(2) FNMQCQRRFYEALHDPNLNEEQRNARIRSIKEEC (SEQ ID NO: 5); or
(3) MQCQRRFYEALHDPNLNEEQRNARIRSI(Orn)EEC (SEQ ID NO: 6).

11. The compound or salt thereof according to any one of claims 7-10, wherein the N-terminal and C-terminal amino acid residues in the affinity peptide may be protected, and
the two thiol groups in the side chain of the two cysteine residues (C) in the affinity peptide may be linked by a disulfide bond or via a linker.

12. A reagent for derivatizing an antibody, the reagent comprising a compound or salt thereof, the compound being represented by the following formula (I): 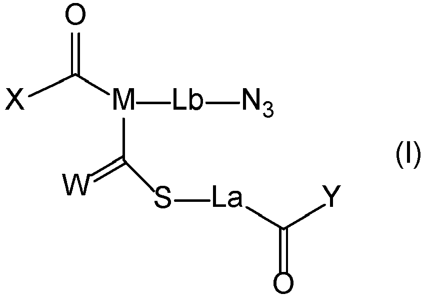 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

13. A compound or salt thereof, the compound being represented by the following formula (I'): 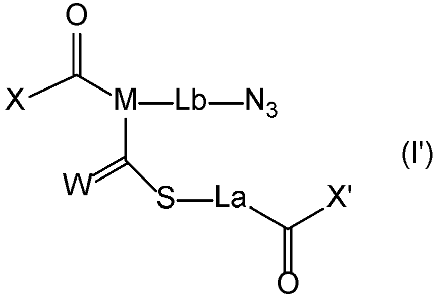 wherein
X indicates a leaving group,
X' indicates a leaving group having a leaving ability which is higher than that of the leaving group X,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

14. A compound or salt thereof according to claim 13, wherein a leaving group having a leaving ability which is higher than that of the leaving group X is a pentafluorophenyloxy group, a tetrafluorophenyloxy group, a paranitrophenyloxy group, or an N-succinimidyloxy group.

15. A compound or salt thereof, the compound being represented by the following formula (I"): 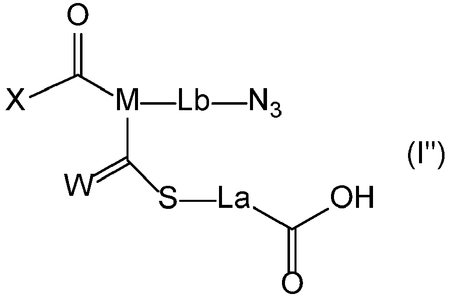 wherein
X indicates a leaving group,
OH indicates a hydroxy group,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group.

16. The antibody intermediate or salt thereof, the antibody intermediate comprising a structural unit represented by the following formula (II): 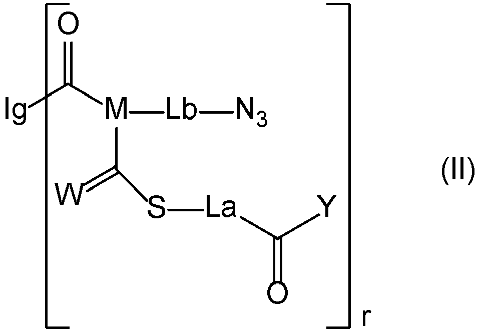 wherein
Ig indicates an immunoglobulin unit comprising two heavy chains and two light chains, and forms an amide bond with a carbonyl group adjacent to Ig via an amino group in a side chain of lysine residues in the two heavy chains,
Y indicates an affinity peptide having a binding region in a CH2 domain in the immunoglobulin unit,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group,
Lb indicates a bond or a divalent group, and
the average ratio r of the amide bond per the two heavy chains is from 1.0 to 3.0.

17. The antibody intermediate or salt thereof according to claim 16, wherein the antibody is a human antibody.

18. The antibody intermediate or salt thereof according to claim 16 or 17, wherein the antibody is human IgG.

19. The antibody intermediate or salt thereof according to any one of claims 16-18, wherein the lysine residue is present at one or more positions selected from the group consisting of the position 246/248, the position 288/290, and the position 317 in a heavy chain of a human IgG according to EU numbering.

20. The antibody intermediate or salt thereof according to any one of claims 16-19, wherein the average ratio r is 1.5 to 2.5.

21. The antibody intermediate or salt thereof according to claim 19 or 20, wherein the lysine residue is present at two positions selected from the group consisting of the position 246/248, the position 288/290, and the position 317 in a heavy chain of a human IgG according to EU numbering.

22. The antibody intermediate or salt thereof according to claim 21, wherein the two positions are the position 246/248 and the position 288/290.

23. An azide group-introduced antibody derivative or salt thereof, the azide group-introduced antibody derivative comprising a structural unit represented by the following formula (III): 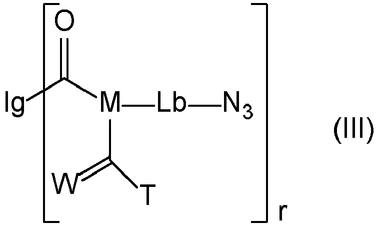 wherein
Ig indicates an immunoglobulin unit comprising two heavy chains and two light chains, and forms an amide bond with a carbonyl group adjacent to Ig via an amino group in a side chain of lysine residues in the two heavy chains,
M indicates a trivalent group linking the carbon atom in C=O adjacent to M and the carbon atom in C=W via a main chain portion consisting of 3 to 5 carbon atoms,
O indicates an oxygen atom,
T indicates a monovalent group,
W indicates an oxygen atom or a sulfur atom,
N₃ indicates an azide group,
Lb indicates a bond or a divalent group, and
the average ratio r of the amide bond per the two heavy chains is from 1.0 to 3.0.

24. The azide group-introduced antibody derivative or salt according to claim 23, wherein the monovalent group indicated by T is a hydroxyamino group which may be substituted.

25. The azide group-introduced antibody derivative or salt according to claim 23 or 24, wherein the antibody is a human antibody.

26. The azide group-introduced antibody derivative or salt thereof according to any one of claims 23-25, wherein the antibody is human IgG.

27. The azide group-introduced antibody derivative or salt thereof according to any one of claims 23-26, wherein the main chain portion consisting of 3 to 5 carbon atoms in M is a portion comprising a linear alkylene, or a ring-constituting carbon atom, or a combination thereof.

28. The azide group-introduced antibody derivative or salt thereof according to any one of claims 23-27, wherein the lysine residue is present at one or more positions selected from the group consisting of the position 246/248, the position 288/290, and the position 317 in a heavy chain of a human IgG according to EU numbering.

29. The azide group-introduced antibody derivative or salt thereof according to any one of claims 23-28, wherein the lysine residue has regioselectivity for lysine residues at the 246/248 position of the human IgG heavy chain according to EU numbering.

30. The azide group-introduced antibody derivative or salt thereof according to any one of claims 23-28, wherein the lysine residue has regioselectivity for the lysine residue at the position 288/290 in a heavy chain of a human IgG according to EU numbering.

31. The azide group-introduced antibody derivative or salt thereof according to any one of claims 23-30, wherein the average ratio r is 1.5-2.5.

32. The azide group-introduced antibody derivative or salt thereof according to any one of claims 28-31, wherein the lysine residue is present at two positions selected from the group consisting of the position 246/248, the position 288/290, and the position 317 in a heavy chain of a human IgG according to EU numbering.

33. The azide group-introduced antibody derivative or salt thereof according to claim 32, wherein the two positions are the position 246/248 and the position 288/290.

34. A conjugate of an antibody and a functional substance or a salt thereof, the conjugate comprising a structural unit represented by the following formula (IV): 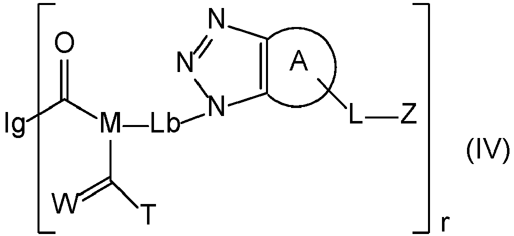 wherein
Ig indicates an immunoglobulin unit comprising two heavy chains and two light chains, and forms an amide bond with a carbonyl group adjacent to Ig via an amino group in a side chain of lysine residues in the two heavy chains,
M indicates a trivalent group linking the carbon atom in C=O adjacent to M and the carbon atom in C=W via a main chain portion consisting of 3 to 5 carbon atoms,
O indicates an oxygen atom,
T indicates a monovalent group,
W indicates an oxygen atom or a sulfur atom,
N indicates a nitrogen atom,
Lb indicates a bond or a divalent group,
Z indicates a functional substance,
L indicates a bond or a divalent group,
the ring A indicates a ring fused with the triazole ring, and
the average ratio r of the amide bond per the two heavy chains is from 1.0 to 3.0.

35. The conjugate or salt thereof according to claim 34, wherein the antibody is a human antibody.

36. The conjugate or salt thereof according to claim 34 or 35, wherein the antibody is a human IgG.

37. The conjugate or salt thereof according to any one of claims 34-36, wherein the main chain portion consisting of 3 to 5 carbon atoms in M is a portion comprising a linear alkylene, or a ring-constituting carbon atom, or a combination thereof.

38. The conjugate or salt thereof according to any one of claims 34-37, wherein the lysine residue is present at one or more positions selected from the group consisting of the position 246/248, the position 288/290, and the position 317 in a heavy chain of a human IgG according to EU numbering.

39. The conjugate or salt thereof according to any one of claims 34-37, wherein the lysine residue has regioselectivity for the lysine residue at the position 246/248 in a heavy chain of a human IgG according to EU numbering.

40. The conjugate or salt thereof according to any one of claims 34-37, wherein the lysine residue has regioselectivity for the lysine residue at the position 288/290 in a heavy chain of a human IgG according to EU numbering.

41. The conjugate or salt thereof according to any one of claims 34-40, wherein the average ratio r is 1.5 to 2.5.

42. The conjugate or salt thereof according to any one of claims 38-41, wherein the lysine residue is present at two positions selected from the group consisting of the position 246/248, the position 288/290, and the position 317 in a heavy chain of a human IgG according to EU numbering.

43. The conjugate or salt thereof according to claim 42, wherein the two positions are the position 246/248 and the position 288/290.

44. A method for producing an antibody intermediate or salt thereof, the method comprising reacting a compound or salt thereof represented by the following formula (I): 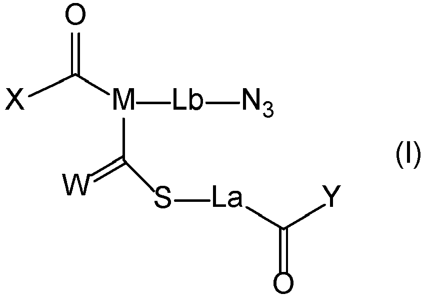 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group,
with an antibody comprising the immunoglobulin unit,
to give an antibody intermediate or salt thereof comprising a structural unit represented by the following formula (II): 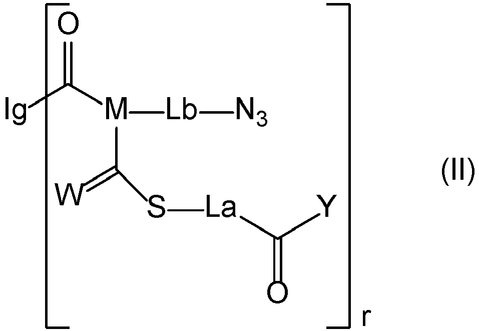
wherein
Ig indicates the immunoglobulin unit,
Y, M, O, S, W, N₃, La, and Lb are the same as those of the above formula (I), and
the average ratio r of the amide bond per the two heavy chains is from 1.0 to 3.0.

45. A method for producing an azide group-introduced antibody derivative or salt thereof, the method comprising:
(1) reacting a compound or salt thereof represented by the following formula (I): 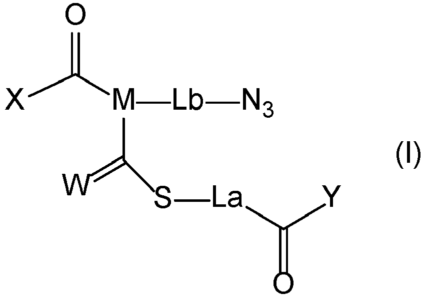 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group,
with an antibody comprising the immunoglobulin unit,
to give an antibody intermediate or salt thereof comprising a structural unit represented by the following formula (II): 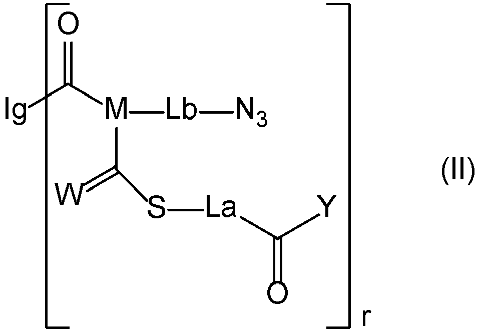
wherein
Ig indicates the immunoglobulin unit,
Y, M, O, S, W, N₃, La, and Lb are the same as those of the above formula (I), and
the average ratio r of the amide bond per the two heavy chains is from 1.0 to 3.0; and
(2) subjecting the antibody intermediate or salt thereof to a thioester cleavage reaction to give an azide group-introduced antibody derivative or salt thereof comprising a structural unit represented by the following formula (III): 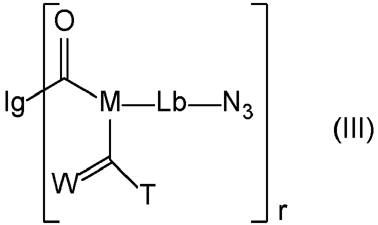 wherein
T indicates a monovalent group, and
Ig, M, O, W, N₃, Lb, and r are the same as those of the above formula (II).

46. A method for producing a conjugate of an antibody and a functional substance or a salt thereof, the method comprising:
(1) reacting a compound or salt thereof represented by the following formula (I): 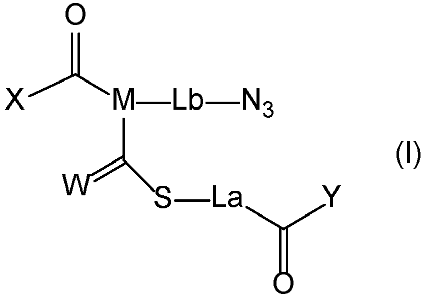 wherein
X indicates a leaving group,
Y indicates an affinity peptide having a binding region in a CH2 domain in an immunoglobulin unit comprising two heavy chains and two light chains,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group, and
Lb indicates a bond or a divalent group,
with an antibody comprising the immunoglobulin unit,
to give an antibody intermediate or salt thereof comprising a structural unit represented by the following formula (II): 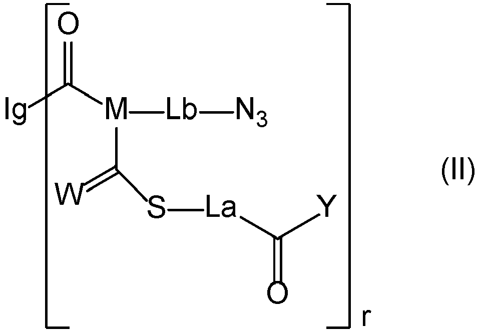
wherein
Ig indicates the immunoglobulin unit,
Y, M, O, S, W, N₃, La, and Lb are the same as those of the above formula (I), and
the average ratio r of the amide bond per the two heavy chains is from 1.0 to 3.0;
(2) subjecting the antibody intermediate or salt thereof to a thioester cleavage reaction to give an azide group-introduced antibody derivative or salt thereof comprising a structural unit represented by the following formula (III): 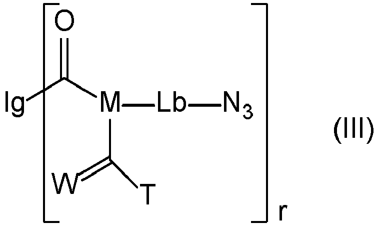 wherein
T indicates a monovalent group, and
Ig, M, O, W, N₃, Lb, and r are the same as those of the above formula (II); and
(3) reacting the azide group-introduced antibody derivative or salt thereof with a target substance represented by the following formula (V): 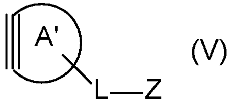 wherein
the ring A' indicates a ring having a triple bond between carbon atoms,
Z indicates a functional substance, and
L indicates a bond or a divalent group,
to give the conjugate of an antibody and a functional substance or the salt thereof which comprises a structural unit represented by the following formula (IV): 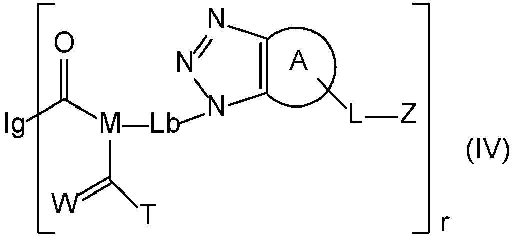
wherein
the ring A indicates a ring fused with the triazole ring,
N indicates a nitrogen atom,
Ig, M, O, T, W, Lb, and r are the same as those of the above formula (III), and
Z and L are the same as those of the above formula (V) .

47. A method for producing an azide group-introduced antibody derivative or salt thereof, the method comprising subjecting the antibody intermediate or salt thereof comprising a structural unit represented by the following formula (II): 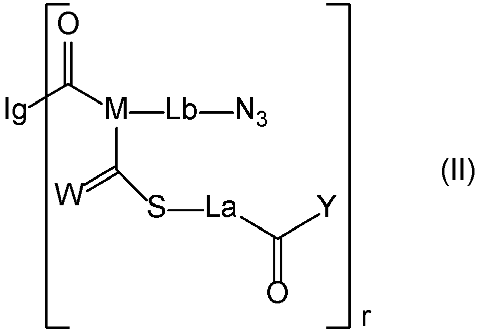 wherein
Ig indicates an immunoglobulin unit comprising two heavy chains and two light chains, and forms an amide bond with a carbonyl group adjacent to Ig via an amino group in a side chain of lysine residues in the two heavy chains,
Y indicates an affinity peptide having a binding region in a CH2 domain in the immunoglobulin unit,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group,
Lb indicates a bond or a divalent group, and
the average ratio r of the amide bond per the two heavy chains is from 1.0 to 3.0,
to a thioester cleavage reaction to give an azide group-introduced antibody derivative or salt thereof comprising a structural unit represented by the following formula (III): 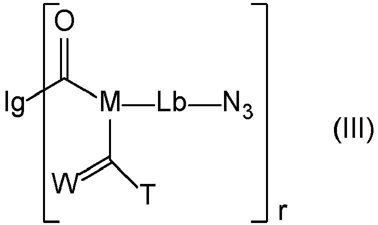
wherein
T indicates a monovalent group, and
Ig, M, O, W, N₃, Lb, and r are the same as those of the above formula (II).

48. A method for producing a conjugate of an antibody and a functional substance or a salt thereof, comprising:
(1) subjecting the antibody intermediate or salt thereof comprising a structural unit represented by formula (II): 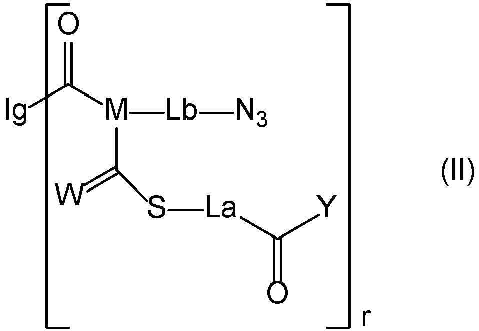 wherein
Ig indicates an immunoglobulin unit comprising two heavy chains and two light chains, and forms an amide bond with a carbonyl group adjacent to Ig via an amino group in a side chain of lysine residues in the two heavy chains,
Y indicates an affinity peptide having a binding region in a CH2 domain in the immunoglobulin unit,
M indicates a trivalent group linking the carbon atom in C=O adjacent to M and the carbon atom in C=W via a main chain portion consisting of 3 to 5 carbon atoms,
O indicates an oxygen atom,
S indicates a sulfur atom,
W indicates an oxygen atom or a sulfur atom,
N₃ indicates an azide group,
La indicates a bond or a divalent group,
Lb indicates a bond or a divalent group, and
the average ratio r of the amide bond per the two heavy chains is from 1.0 to 3.0,
to a thioester cleavage reaction to give an azide group-introduced antibody derivative or salt thereof comprising a structural unit represented by the following formula (III): 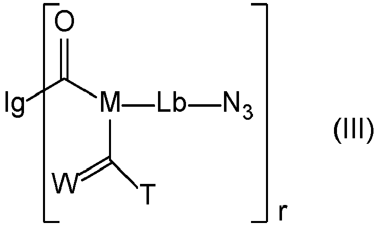
wherein
T indicates a monovalent group, and
Ig, M, O, W, N₃, Lb, and r are the same as those of the above formula (II); and
(2) reacting the azide group-introduced antibody derivative or salt thereof with a target substance represented by formula (V): 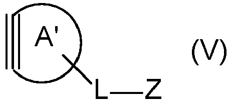 wherein
the ring A' indicates a ring having a triple bond between carbon atoms,
Z indicates a functional substance, and
L indicates a bond or a divalent group,
to give the conjugate of an antibody and a functional substance or the salt thereof comprising a structural unit represented by formula (IV): 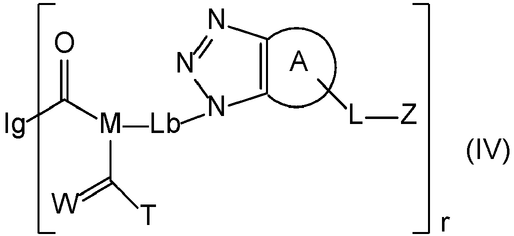
wherein
the ring A indicates a ring fused with the triazole ring,
N indicates a nitrogen atom,
Ig, M, O, T, W, Lb, and r are the same as those of the above formula (III), and
Z and L are the same as those of the above formula (V) .

49. A method for producing a conjugate of an antibody and a functional substance or a salt thereof, the method comprising reacting the azide group-introduced antibody derivative or salt thereof comprises a structural unit represented by the following formula (III): 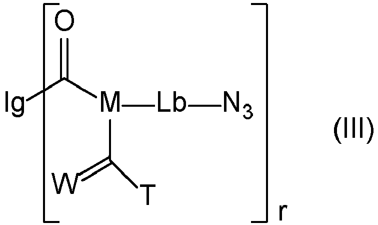 wherein
Ig indicates an immunoglobulin unit comprising two heavy chains and two light chains, and forms an amide bond with a carbonyl group adjacent to Ig via an amino group in a side chain of lysine residues in the two heavy chains,
M indicates a trivalent group linking the carbon atom in C=O adjacent to M and the carbon atom in C=W via a main chain portion consisting of 3 to 5 carbon atoms,
O indicates an oxygen atom,
T indicates a monovalent group,
W indicates an oxygen atom or a sulfur atom,
N₃ indicates an azide group,
Lb indicates a bond or a divalent group, and
the average ratio r of the amide bond per the two heavy chains is from 1.0 to 3.0,
with a target substance represented by the following formula (V): 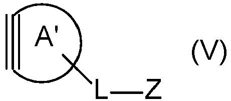
wherein
the ring A' indicates a ring having a triple bond between carbon atoms,
Z indicates a functional substance, and
L indicates a bond or a divalent group,
to give the conjugate of an antibody and a functional substance or the salt thereof comprising a structural unit represented by the following formula (IV): 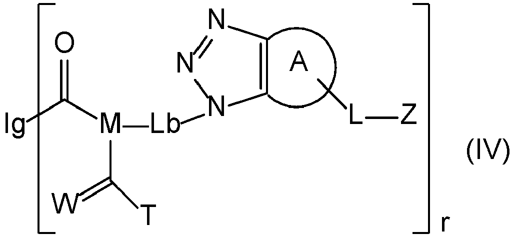
wherein
the ring A indicates a ring fused to a triazole ring,
N indicates a nitrogen atom,
Ig, M, O, T, W, Lb, and r are the same as those of the above formula (III), and
Z and L are the same as those of the above formula (V) .
